# EUROPÄISCHE PATENTSCHRIFT

(11) **EP 2 032 556 B1**
(45) Veröffentlichungstag und Bekanntmachung des Hinweises auf die Patenterteilung: **22.07.2015**
(21) Anmeldenummer: 07764582.8
(22) Anmeldetag: 06.06.2007
(51) Int. Cl.: C07D 401/14, C07D 405/14, C07D 413/14, A01N 43/713, A01N 43/56, C07D 231/14, A01N 43/54, A01N 43/653, A01N 43/80, C07D 403/06

(54) **ANTHRANILSÄUREDIAMID-DERIVATE MIT HETEROAROMATISCHEN UND HETEROCYCLISCHEN SUBSTITUENTEN**
ANTHRANILIC ACID DIAMIDE DERIVATIVE WITH HETERO-AROMATIC AND HETERO-CYCLIC SUBSTITUENTS
DÉRIVÉ DE DIAMIDE D'ACIDE ANTHRANILIQUE AVEC SUBSTITUANTS HÉTÉROAROMATIQUES ET HÉTÉROCYCLIQUES

(30) Priorität: 13.06.2006 DE 102006027336; 12.07.2006 DE 102006032168
(43) Veröffentlichungstag der Anmeldung: 11.03.2009
(73) Patentinhaber: Bayer Intellectual Property GmbH, 40789 Monheim am Rhein (DE)
(72) Erfinder: ALIG, Bernd, 53639 Königswinter (DE); FISCHER, Rüdiger, 50259 Pulheim (DE); FUNKE, Christian, 42799 Leichlingen (DE); GESING, Ernst, Rudolf, F., 40699 Erkrath-Hochdahl (DE); HENSE, Achim, 51379 Leverkusen (DE); MALSAM, Olga, 51503 Rösrath (DE); DREWES, Mark Wilhelm, 40764 Langenfeld (DE); GÖRGENS, Ulrich, 40882 Ratingen (DE); MURATA, Tetsuya, Tochigi 323-0807 (JP); WADA, Katsuaki, Oyama-shi, Tochigi 323-0806 (JP); ARNOLD, Christian, 40764 Langenfeld (DE); SANWALD, Erich, 24159 Kiel (DE)
(74) Vertreter: BIP Patents
(86) Internationale Anmeldenummer: PCT/EP2007/005016
(87) Internationale Veröffentlichungsnummer: WO 2007/144100

(56) Entgegenhaltungen:
- WO-A-2004/046129
- WO-A-2005/018557

## Beschreibung

Die vorliegende Erfindung betrifft neue Insektizide, mehrere Verfahren zu ihrer Herstellung und ihre Verwendung als Wirkstoffe, insbesondere ihre Verwendung als Schädlingshekämpfungsmittel.

Es ist bereits bekannt, dass bestimmte Anthranilamide (z.B. WO 01/70671, WO 03/015519, WO 03/016284, WO 03/015518, WO 03/024222, WO 03/016282, WO 03/016283, WO 03/062226, WO 03/027099, WO 04/027042, WO 04/033468, WO 2004/046129, WO 2004/067528, WO 2005/118552, WO 2005/077934, WO 2005/085234, WO 2006/023783, WO 2006/000336, WO 2006/040113, WO 2006/111341, WO 2007/006670, WO 2007/024833, WO2007/020877) insektizide Eigenschaften besitzen.

Die Wirksamkeit dieser Stoffe ist gut, lässt aber in manchen Fällen zu wünschen übrig.

Es wurden nun neue Anthranilamide der Formel (I) 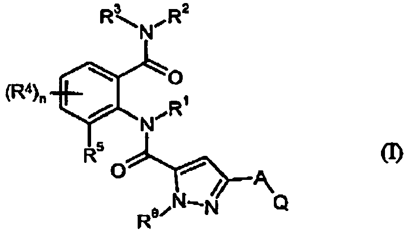 gefunden, in welcher
die Verbindungen der allgemeinen Formel (I) außerdem N-Oxide und Salze umfassen.

Schließlich wurde gefunden, dass die erfindungsgemäßen Verbindungen der Formel (I) sehr gute insektizide Eigenschaften besitzen und sich sowohl im Pflanzenschutz als auch im Materialschutz zur Bekämpfung unerwünschter Schädlinge, wie Insekten, verwenden lassen.

Die erfindungsgemaßen Verbindungen können gegebenenfalls als Mischungen verschiedener möglicher isomerer Formen, insbesondere von Stereoisomeren, wie z. B. E- und Z-, threo- und erythro-, sowie optischen Isomeren, gegebenenfalls aber auch von Tautomeren vorliegen. Es werden sowohl die E- als auch die Z-Isomeren, wie auch die threo- und erythro-, sowie die optischen Isomeren, beliebige Mischungen dieser Isomeren, sowie die möglichen tautomeren Formen beansprucht.

Die Anthranilamide sind durch die Fonnel (I) allgemein definiert. Bevorzugte Restedefinitionen der vorstehenden und nachfolgend genannten Formeln sind im Folgenden angegeben. Diese Definitionen gelten für die Endprodukte der Formel (I) wie für alle Zwischenprodukte gleichermaßen.
- R¹: steht für Wasserstoff, Methyl, Cyclopropyl, Cyanomethyl, Methoxymethyl, Methylthiomethyl, Methylsulfinylmethyl oder Methylsulfonylmethyl.
- R¹: steht besonders bevorzugt für Wasserstoff.
- R²: steht für Wasserstoff oder C₁-C₆-Alkyl.
- R²: steht für Wasserstoff oder Methyl.
- R²: steht besonders bevorzugt für Wasserstoff.
- R⁵: steht für Wasserstoff oder für jeweils gegebenenfalls einfach oder mehrfach, gleich oder verschieden substituiertes C₁-C₆-Alkyl, C₁-C₆-Alkoxy, wobei die Substituenten unabhängig voneinander ausgewählt sein können aus Halogen, Cyano, Nitro, Hydroxy, C₁-C₆-Alkyl, C₃-C₆-Cycloalkyl, C₁-C₄-Alkoxy, C₁-C₄-Haloalkoxy, C₁-C₄-Alkylthio, C₁-C₄-Alkylsulfinyl, C₁-C₄-Alkylsulfonyl, C₁-C₄-Alkylsulfimino, C₁-C₄-Alkylsulfimino-C₁-C₄-alkyl, C₁-C₄-Alkylsulfimino-C₂-C₅-alkylcarbonyl, C₁-C₄-Alkylsulfoximino, C₁-C₄-Alkylsulfoximino-C₁-C₄-alkyl, C₁-C₄-Alkylsulfoximino-C₂-C₅-alkylcarbonyl, C₂-C₆-Alkoxycarbonyl, C₁-C₆-Alkylcarbonyl oder C₃-C₆-Trialkylsilyl,
- R³: steht weiterhin für gegebenenfalls einfach oder mehrfach, gleich oder verschieden substituiertes C₃-C₆-Cycloalkyl wobei die Substituenten unabhängig voneinander ausgewählt sein können aus Halogen, Cyano, Nitro, Hydroxy, C₁-C₆-Alkyl, C₃-C₆-Cycloalkyl, C₁-C₄-Alkoxy, C₁-C₄-Haloalkoxy, C₁-C₄-Alkylthio, C₁-C₄-Alkylsulfinyl, C₁-C₄-Alkylsulfonyl, C₁-C₄-Alkylsulfimino, C₁-C₄-Alkylsulfimino-C₁-C₄-alkyl C₁-C₄-Alkylsulfimino-C₂-C₅-alkylcarbonyl C₁-C₄-Alkylsulfoximino, C₁-C₄-Alkylsulfoximino-C₁-C₄-alkyl, C₁-C₄-Alkylsulfoximino-C₂-C₅-alkylcarbonyl, C₂-C₆-Alkoxycarbonyl, C₂-C₆-Alkylcarbonyl oder C₃-C₆-Trialkylsilyl,
- R³: steht besonders bevorzugt für C₁-C₄-Alkyl (Methyl, Ethyl, n-Propyl, iso-Propyl, n-Butyl, iso-Butyl, sec-Butyl oder tert-Butyl) oder Cyano-C₁-C₃-Alkyl (Cyanomethyl, 1-Cyanoethyl, 2-Cyanoethyl, 1-Cyano-n-Propyl, 2-Cyano-n-Propyl, 3-Cyano-n-Propyl, 1-Cyano-iso-Propyl, 2-Cyano-iso-Propyl).
- R³: steht insbesondere bevorzugt für Methyl, iso-Propyl oder Cyanomethyl.
- R⁴: steht für Wasserstoff, C₁-C₄-Alkyl, C₁-C₂-Haloalkyl, Halogen, Cyano oder C₁-C₂-Haloalkoxy.
Besonders bevorzugt stehen ausserdem zwei benachbarte Reste R⁴ für -(CH₂)₄-, -(CH=CH-)₂-, -O(CH₂)₂O-, -O(CF₂)₂O-, -(CH=CH-CH=N)- oder -(CH=CH-N=CH)-.
- R⁴: steht besonders bevorzugt für Wasserstoff, Methyl, Trifluormethyl, Cyano, Fluor, Chlor, Brom, lod oder Trifluormethoxy. Ganz besonders bevorzugt stehen ausserdem zwei benachbarte Reste R⁴ für-(CH₂)₄-, oder -(CH=CH-)₂-.
- R⁴: steht insbesondere bevorzugt für Chlor oder Brom,
- R¹: steht weiterhin insbesondere bevorzugt für Iod oder Cyano. Insbesondere bevorzugt stehen ausserdem zwei benachbarte Reste R⁴ für -(CH=CH-)₂-.
- R⁵: steht für C₁-C₄-Alkyl, C₃-C₆-Cycloalkyl, C₁-C₄-Haloalkyl, C₁-C₆-Halocycloalkyl, C₂-C₆-Alkenyl, C₂-C₄-Haloalkenyl, C₂-C₄Alkinyl, C₂-C₄-Haloalkinyl, C₁-C₄-Alkoxy, C₁-C₄-Haloalkoxy, Fluor, Chlor, Brom, Iod, Cyano, Nitro oder C₃-C₆-Trialkylsityl.
- R⁵: steht besonders bevorzugt für Methyl, Fluor, Chlor, Brom oder Iod.
- R⁵: steht insbesondere bevorzugt für Methyl oder Chlor.
- R⁶: steht bevorzugt für oder
- R⁶: steht weiterhin bevorzugt für cycloalkoxy,
- R⁶: steht besonders bevorzugt für Methyl oder 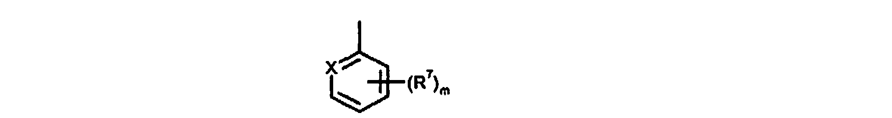
- R⁷: steht unabhängig voneinander für Wasserstoff Halogen oder C₁-C₄-Haloalkyl,
- R⁷: steht besonders bevorzugt für Fluor, Chlor oder Brom,
- R⁷: steht insbesondere bevorzugt für Chlor.
- m: steht bevorzugt 3,
- m: steht für 1 oder 2,
- m: steht besonders bevorzugt für 1,
- X: steht bevorzugt für N, CCl, CBr oder Cl,
- X: steht für N, CH, CF, CCI oder CBr,
- X: steht besonders bevorzugt für N, CCI oder CH.
- A: steht besonders bevorzugt für -CH₂-, -CH(CH₃), C(CH₃)₂ oder CH₂CH₂,
- A: steht weiterhin besonders bevorzugt für -CH(CN)-,
- A: steht ganz besonders bevorzugt für CH₂ oder CH(CH₃),
- A: steht insbesondere bevorzugt für CH₂,
- Q: steht für einen gegebenenfalls cinfach oder mehrfach substituierten 5-oder 6-gliedrigen aromatischen heterocyclischen Ring der Reihe Q-36 bis Q-40 oder ein aromatisches 9-gliedriges annelliertes heterobicyclisches Ringsystem Q-54 bis Q-56, wobei die Substituenten unabhängig voneinander ausgewählt sein können aus C₁-C₃-Alkyl, C₁-C₃-Haloalkyl, C₁-C₂-Alkoxy, Halogen, Cyano, Hydroxy, Nitro oder C₁-C₂-Haloalkoxy.
- Q: steht weiterhin für einen gegebenenfalls einfach oder mehrfach, substituierten 5- oder 6-gliedrigen aromatischen heterocyclischen Ring der Reihe Q-36 bis Q-40 und Q-58 bis Q-59, einen aromatisches 9-gliedriges annelliertes heterobicyclisches Ringsystem Q-54 bis Q-56 sowie für ein 5-gliedrigen heterocyclischen Ring Q-60 bis Q-61, wobei die Substituenten unabhängig voneinander ausgewählt sein können aus C₁-C₃-Alkyl, C₁-C₃-Haloalkyl, C₁-C₂-Alkoxy, Halogen, Cyano, Hydroxy, Nitro oder C₁-C₂-Haloalkoxy, oder wobei die Substituenten unabhängig voneinander ausgewählt sein können aus Phenyl oder einem 5- oder 6-gliedrigen heteroaromatischen Ring, wobei Phenyl oder der Ring gegebenenfalls einfach oder mehrfach, gleich oder verschieden mit C₁-C₆-Alkyl, C₂-C₆-Alkenyl, C₂-C₆-Alkinyl, C₁-C₆-Cycloalkyl, C₁-C₆-Haloalkyl, C₂-C₆-Haloalkenyl, C₂-C₆-Haloalkinyl, C₃-C₆-Halocycloalkyl, Halogen, CN, NO₂, C₁-C₄-Alkoxy, C₁-C₄-Haloalkoxy substituiert sein können,
- Q: steht besonders bevorzugt für einen gegebenenfalls einfach oder mehrfach substituierten aromatischen heterocyclischen Ring der Reihe Q-37, Q-38, Q-39, Q-40, Q-58 und Q-59, sowie für ein 5-gliedrigen heterocyclischen Ring Q-60, wobei die Substituenten unabhängig voneinander ausgewählt sein können aus C₁-C₃-Alkyl, C₁-C₃-Haloalkyl, C₁-C₂-Alkoxy, Halogen, Cyano, Hydroxy, Nitro oder C₁-C₂-Haloalkoxy,
oder wobei die Substituenten unabhängig voneinander ausgewählt sein können aus Phenyl oder einem 5- oder 6-gliedrigen heteroaromatischen Ring, wobei Phenyl oder der Ring gegebenenfalls einfach oder mehrfach, gleich oder verschieden mit C₁-C₆-Alkyl, C₂-C₆-Alkenyl, C₂-C₆-Alkinyl, C₃-C₆-Cycloalkyl, C₁-C₆-Haloalkyl, C₂-C₆-Haloalkenyl, C₂-C₆-Haloalkinyl, C₃-C₆-Halocycloalkyl, Halogen, CN, NO₂, C₁-C₄-Alkoxy, C₁-C₄-Haloalkoxy substituiert sein können,
- Q: steht weiterhin besonders bevorzugt für einen gegebenenfalls einfach oder mehrfach substituierten aromatischen heterocyclischen Ring der Reihe Q-37, Q-38, Q-39, Q-40, Q-58 und Q-59, sowie für ein 5-gliedrigen heterocyclischen Ring Q-60, wobei die Substituenten unabhängig voneinander ausgewählt sein können aus C₁-C₃-Alkyl, C₁-C₃-Haloalkyl, Halogen, Cyano, Nitro oder C₁-C₂-Haloalkoxy,
oder wobei die Substituenten unabhängig voneinander ausgewählt sein können aus Phenyl oder einem 5- oder 6-gliedrigen heteroaromatischen Ring, wobei Phenyl oder der Ring gegebenenfalls einfach oder mehrfach, gleich oder verschieden mit C₁-C₆-Alkyl, C₂-C₆-Alkenyl, C₂-C₆-Alkinyl, C₃-C₆-Cycloalkyl, C₁-C₆-Haloalkyl, C₂-C₆-Haloalkenyl, C₂-C₆-Haloalkinyl, C₃-C₆-Halocycloalkyl, Halogen, CN, NO₂, C₁-C₄-Alkoxy, C₁-C₄-Haloalkoxy substituiert sein können,
- Q: steht insbesondere bevorzugt für einen gegebenenfalls einfach, zweifach oder dreifach an Kohlenstoffatomen substituierten aromatischen hetero-cyclischen Ring Q-37, Q-40, Q-58 und Q-59, sowie für ein 5-gliedrigen heterocyclischen Ring Q-60, wobei die Substituenten unabhängig voneinander ausgewählt sein können aus Chlor, Fluor, Iod, Brom, Cyano, Trifluormethyl und Pentafluorethyl,
oder wobei die Substituenten unabhängig voneinander ausgewählt sein können aus Phenyl, wobei der Phenyl-Ring gegebenenfalls einfach oder mehrfach, gleich oder verschieden mit C₁-C₆-Alkyl, C₂-C₆-Alkenyl, C₂-C₆-Alkinyl, C₃-C₆-Cycloalkyl, C₁-C₆-Haloalkyl, C₂-C₆-Haloalkenyl, C₂-C₆-Haloalkinyl, C₃-C₆-Halocycloalkyl, Halogen, CN, NO₂, C₁-C₄-Haloalkoxy substituiert sein können,
- Q: steht weiterhin insbesondere bevorzugt für einen gegebenenfalls einfach oder mehrfach substituierten aromatischen heterocyclischen Ring der Reihe Q-37, Q-40, Q-58 und Q-59, sowie für ein 5-gliedrigen heterocyclischen Ring Q-60, wobei die Substituenten unabhängig voneinander ausgewählt sein können aus Chlor, Fluor, Iod, Cyano, Trifluormethyl und Pentafluorethyl,
oder wobei die Substituenten unabhängig voneinander ausgewählt sein können aus Phenyl, wobei der Phenyl-Ring gegebenenfalls einfach oder mehrfach, gleich oder verschieden mit Chlor, Fluor, Iod, Brom, Cyano, Trifluormethyl und Pentafluorethyl.
substituiert sein kann,

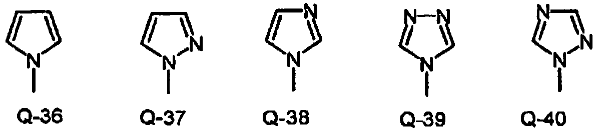

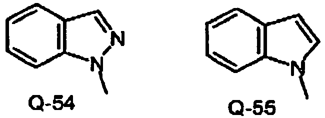

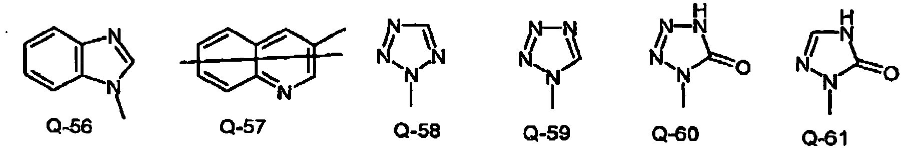

Hervorgehoben sind Verbindungen der Formel (I-1), 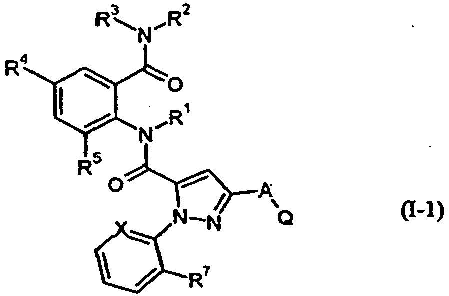 in welcher R¹, R², R³, R⁴, R⁵, R⁷, A, Q und X die oben angegebenen allgemeinen, bevorzugten, besonders bevorzugten, ganz besonders bevorzugten und insbesondere bevorzugten Bedeutungen haben.

Durch Halogen substituierte Reste, z.B. Haloalkyl, sind einfach oder mehrfach bis zur maximal möglichen Substituentenzahl halogeniert. Bei mehrfacher Halogenierung können die Halogenatome gleich oder verschieden sein. Halogen steht dabei für Fluor, Chlor, Brom oder lod, insbesondere für Fluor, Chlor oder Brom.

Bevorzugt, besonders bevorzugt, ganz besonders bevorzugt und insbesondere bevorzugt sind Verbindungen, welche jeweils die unter bevorzugt, besonders bevorzugt, ganz besonders bevorzugt und insbesondere bevorzugt genannten Substituenten tragen.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können einfach oder mehrfach substituiert sein, wobei bei Mehrfachsubstitutionen die Substituenten gleich oder verschieden sein können.

Die oben aufgeführten allgemeinen oder in Vorzugsbereichen aufgeführten Restedefinitionen bzw. Erläuterungen können jedoch auch untereinander, also zwischen den jeweiligen Bereichen und Vorzugsbereichen beliebig kombiniert werden. Sie gelten für die Endprodukte sowie für die Vor- und Zwischenprodukte entsprechend.

Weiterhin wurde gefunden, dass man Anthranilamide der Formel (I) nach einem der folgenden Verfahren erhält.

Anthranilamide der Formel (I) 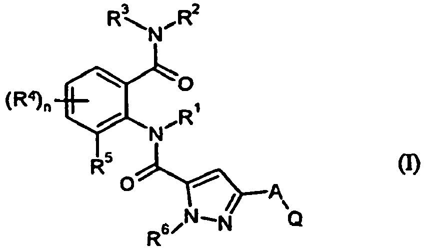 in welcher A, R¹, R², R³, R⁴. R⁵, R⁶, Q und n die oben angegebenen Bedeutungen haben, werden erhalten, indem man
(A) Aniline der Formel (II) 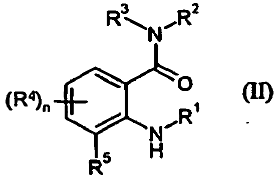 in welcher A, R¹, R², R³, R⁴, R⁵ und n die oben angegebenen Bedeutungen haben,
   mit Carbonsäurechloriden der Formel (III) 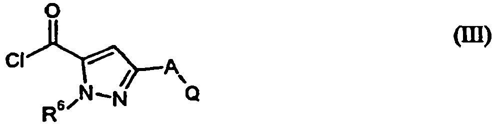 in welcher R⁶, A und Q die oben angegebenen Bedeutungen haben, in Gegenwart eines Säurebindemittels umsetzt,
(B) Aniline der Formel (II) 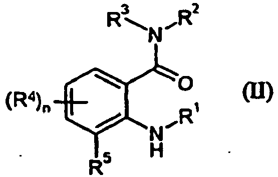 in welcher A, R¹, R², R³, R⁴, R⁵ und n die oben angegebenen Bedeutungen haben,
   mit einer Carbonsäure der Formel (IV) 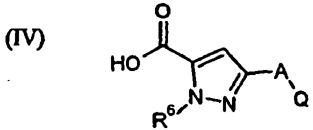 in welcher R⁶, A und Q die oben angegebenen Bedeutungen hat,
   in Gegenwart eines Kondensationsmittels umsetzt oder indem man
(C) zur Synthese von Anthranilamiden der Formel (I), in welcher R¹ für Wasserstoff steht, Benzoxazinone der Formel (V) 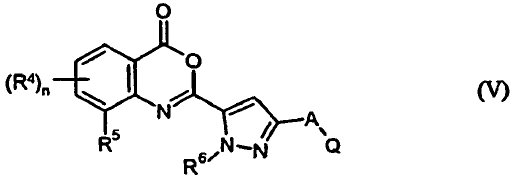 in welcher R⁴, R⁵, R⁶, A, Q und n die oben angegebenen Bedeutungen haben,
   mit einem Amin der Formel (XV) 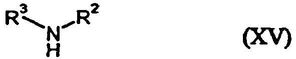 in welcher R² und R³ die oben angegebenen Bedeutungen haben,
   in Gegenwart eines Verdünnungsmittels umsetzt.

### Erläuterung der Verfahren und Zwischenprodukte

### Verfahren (A)

Verwendet man beispielsweise 2-Amino-5-chlor-3,*N*-dimethyl-benzamid und 5-(3,5-Bis-trifluormethyl-pyrazol-1-ylmethyl)-2-(3-chlor-pyridin-2-yl)-2*H*-pyrazol-3-carbonsäurechlorid als Ausgangsstoffe, so kann der Verlauf des erfindungsgemäßen Verfahrens (A) durch das folgende Formelschema veranschaulicht werden.

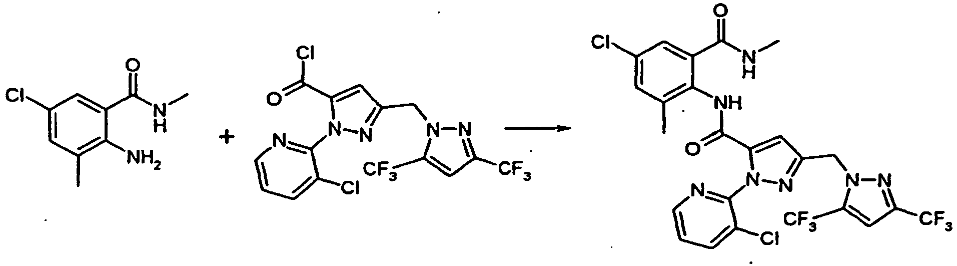

Die bei der Durchführung des erfindungsgemäßen Verfahrens (A) als Ausgangsstoffe benötigten Aminobenzamide sind durch die Formel (II) allgemein definiert. In dieser Formel (II) stehen A, R¹, R², R³, R⁴, R⁵ und n bevorzugt, besonders bevorzugt, ganz besonders bevorzugt bzw. insbesondere bevorzugt für diejenigen Bedeutungen, die bereits im Zusammenhang mit der Beschreibung der erfindungsgemäßen Stoffe der Formel (I) als bevorzugt, besonders bevorzugt usw. für diese Reste genannt wurden.

Das erfindungsgemäße Verfahren (A) wird in Gegenwart eines Säurebindemittels durchgeführt. Hierzu eignen sich alle für solche Kupplungsreaktionen üblichen anorganischen oder organischen Basen. Vorzugsweise verwendbar sind Erdalkalimetall- oder Alkalimetallhydride, -hydroxide, -amide, -alkoholate, -acetate, -carbonate oder -hydrogencarbonate, wie beispielsweise Natriumhydrid, Natriumamid, Lithiumdiisopropylamid, Natrium-methylat, Natrium-ethylat, Kalium-tert.-butylat, Natriumhydroxid, Kaliumhydroxid, Natriumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, Diisopropylethylamin, N,N-Dimethylanilin, N,N-Dimethyl-benzylamin, Pyridin, N-Methylpiperidin, N-Methylmorpholin, N,N-Dimethylamino-pyridin, Diazabicyclooctan (DABCO), Diazabicyclononen (DBN) oder Diazabicycloundecen (DBU). Ebenso lassen sich gegebenenfalls polymer-gestützte Säurebindemittel, wie z.B. polymer-gebundenes Diisopropylamin und polymer-gebundenes Dimethylaminopyridin einsetzen.

Das erfindungsgemäße Verfahren (A) kann gegebenenfalls in Gegenwart eines für solche Reaktionen üblichen inerten organischen Verdünnungsmittel durchgeführt werden. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-amylether, Dioxan, Tetrahydrofuran, 1,2- Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; deren Gemische mit Wasser oder reines Wasser. Besonders bevorzugt verwendbar sind Toluol, Tetrahydrofuran und N,N-Dimethylformamid.

Die Reaktionstemperaturen können bei der Durchführung des erfindungsgemäßen Verfahrens (A) in einem größeren Bereich variiert werden. Im Allgemeinen arbeitet man bei Temperaturen von 0°C bis 150°C, vorzugsweise bei Temperaturen von 20°C bis 100°C.

Das erfindungsgemäße Verfahren wird im Allgemeinen unter Normaldruck durchgeführt. Es ist jedoch auch möglich, das erfindungsgemäße Verfahren unter erhöhtem oder vermindertem Druck - im allgemeinen zwischen 0,1 bar und 10 bar - durchzuführen.

Aminobenzamide der Formel (II) sind bekannt (vgl. z.B. M. J. Kornet, J. Heterocyl. Chem. 1992, 29, 103-105; G. P. Lahm et al., Bioorg. Med. Chem. Letters 2005, 15, 4898-4906; WO 2003/016284, WO 2006/062978).

Pyrazolcarbonsäurechloride der Formel (III) sind neu. Sie lassen sich beispielsweise herstellen, indem man
(D) Pyrazolcarbonsäure-Derivate der Formel (IV) 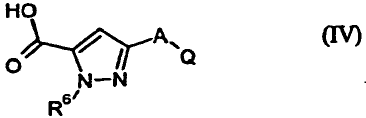 in welcher A, Q und R⁶ die oben angegebenen Bedeutungen haben,
   mit einem Chlorierungsmittel (z.B. Thionylchlorid und Oxalylchlorid) in Gegenwart eines inerten Verdünnungsmittels (z.B. Toluol und Dichlormethan) in Gegenwart von einer katalytischen Menge von N,N-Dimethylformamid umsetzt.
   Pyrazolcarbonsäure-Derivate der Formel (IV) sind neu. Sie lassen sich beispielsweise herstellen, indem man
(E) Pyrazolcarbonsäureester der Formel (VI) 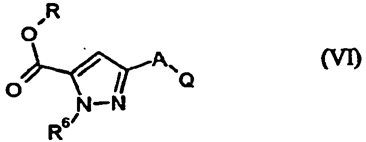 in welcher A, Q und R⁶ die oben angegebenen Bedeutungen haben, und R für C 1-C6-Alkyl steht,
   mit einem Alkalimetallhydroxid (z.B. Natriumhydroxid oder Kaliumhydroxid) in Gegenwart eines inerten Verdünnungsmittels (z.B. Dioxan/Wasser oder Ethanol/Wasser) umsetzt.
   Pyrazolcarbonsäureester der Formel (VI) sind neu. Sie lassen sich beispielsweise herstellen, indem man
(F) Pyrazolcarbonsäureester-Derivate der Formel (VII) 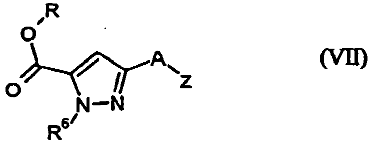 in welcher A, R⁶ und R die oben angegebenen Bedeutungen haben und Z für Chlor, Brom, Iod, Methylsulfonyl oder Toluolsulfonyl steht, mit einem Heteroaromaten der Formel (VIII) oder einer Boronsäure bzw. einem Boronsäureester der Formel (IX), in welcher R' für H, CH₃, C₂H₅ bzw. R'-R' für C(CH₃)₂C(CH₃)₂ steht und Q die oben angegebenen Bedeutungen hat, in Gegenwart eines Übergangsmetalls (z.B. Tetrakis(triphenylphosphin)palladium(0)) und einer Base (z. B. Kaliumcarbonat oder Natriumcarbonat) in Gegenwart eines Lösungsmittels (z.B. Tetrahydrofuran, Acetonitril, oder Dioxan) umsetzt.

   Q-H (VIII)

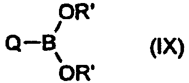

   Heteroaromaten und Heterocyclen der Formel (VIII) sind bekannt, teilweise sogar kommerziell erhältlich oder können nach bekannten Verfahren erhalten werden (vgl. z. B. H. V. Dias et al., Organometallics 1996, 15, 5374-5379; M. D. Threadgill et al., J. Fluorine Chem. 1993, 65, 21-23; M. Abdul-Ghani et al., J. Fluorine Chem. 1990, 48, 149-152; T. Khazaki, Chem. Pharm. Bull. 1996, 44, 314-327; DE 1995-19504627; WO 2004080984, WO 2005495351).
   Heterocyclische Boronsäuren oder Boronate der Formel (IX) sind bekannt, teilweise sogar kommerziell erhältlich oder können nach bekannten Verfahren erhalten werden (vgl. z. B. W. Li, D. P. Nelson, M. S. Jensen, R. S. Hoermer, D. Cai, R. D. Larsen, P. J. Reider, J. Org. Chem. 2002, 67, 5394-5397).
   Pyrazolcarbonsäureester-Derivate der Formel (VII) lassen sich beispielsweise herstellen, indem man
(G) Alkohole der Formel (X) 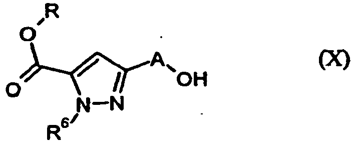 in welcher R, R⁶ und A die oben angegebenen Bedeutungen haben, mit einem Sulfonylchlorid (z.B. Methylsulfonsäurechloid oder Toluolsulfonsäurechlorid) oder einem Halogenierungsmittel (z.B. Thionylchlorid) gegebenenfalls unter Anwesenheit eines Lösungsmittels (z.B. Dichlormethan) und gegebenenfalls unter Anwesenheit einer Base (z.B. Triethylamin oder Pyridin) umsetzt.
   Die Reaktionstemperaturen können bei der Durchführung des erfindungsgemäßen Verfahrens (G) in einem größeren Bereich variiert werden. Im Allgemeinen arbeitet man bei Temperaturen von 0°C bis 150°C, vorzugsweise bei Temperaturen von 0°C bis 60°C.
   Alkohole der Formel (X) lassen sich beispielsweise herstellen, indem man
(H) Pyrazoldicarbonsäureester der Formel (XI) 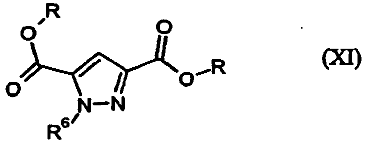 in welcher R und R⁶ die oben angegebenen Bedeutungen haben, mit einem Reduktionsmittel (z.B. Lithiumaluminiumhydrid oder Diisobutylaluminiumhydrid) unter Anwesenheit eines Lösungsmittels (z.B. Tetrahydrofuran oder Diethylether) umsetzt.
   Die Reaktionstemperaturen können bei der Durchführung des erfindungsgemäßen Verfahrens (H) in einem größeren Bereich variiert werden. Im Allgemeinen arbeitet man bei Temperaturen von -100°C bis 20°C, vorzugsweise bei Temperaturen von -78°C bis 0°C.
   Pyrazoldicarbonsäureester der Formel (XI) lassen sich beispielsweise herstellen, indem man
(I) Hydrazine oder deren entsprechenden Salze der Formel (XII) 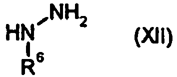 in welcher R⁶ die oben angegebenen Bedeutung hat, mit einem Triketon (XIII) der Formel (XIII) 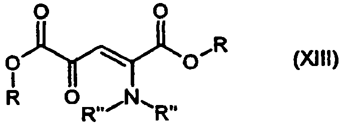 in welcher R die oben angegebenen Bedeutung hat, und R" für Methyl oder Ethyl bzw. R"-R" für (CH₂)₄ oder (CH₂)₂O(CH₂)₂ steht, unter Anwesenheit eines Lösungsmittels (z.B. Methanol oder Ethanol) umsetzt.

Hydrazine oder deren entsprechenden Salze der Formel (XII) sind bekannt, teilweise kommerziell erhältlich oder können nach allgemeinen Synthesemethoden hergestellt werden (vgl. z.B. Advanced Organic Chemistry, Vierte Ausgabe, Jerry March, John Wiley &Sons, Inc. New York, 1992, Seite 1288).

Triketone der Formel (XIII) sind bekannt und können nach allgemeinen Synthesemethoden hergestellt werden (vgl. z.B. Cvetovich, Raymond J.; Pipik, Brenda; Hartner, Frederick W.; Grabowski, Edward J. J.; Tetrahedron Lett 2003, 44, 5867 - 5870).

Die Reaktionstemperaturen können bei der Durchführung des erfindungsgemäßen Verfahrens (I) in einem größeren Bereich variiert werden. Im Allgemeinen arbeitet man bei Temperaturen von 0°C bis 80°C, vorzugsweise bei Temperaturen von 40°C bis 60°C.

### Verfahren (B)

Verwendet man beispielsweise 2-Amino-5-chlor-3,*N*-dimethyl-benzamid und 5-(3,5-Bis-trifluormethyl-pyrazol-1-ylmethyl)-2-(3-chlor-pyridin-2-yl)-2*H*-pyrazol-3-carbonsäure als Ausgangsstoffe, so kann der Verlauf des erfindungsgemäßen Verfahrens (B) durch das folgende Formelschema veranschaulicht werden.

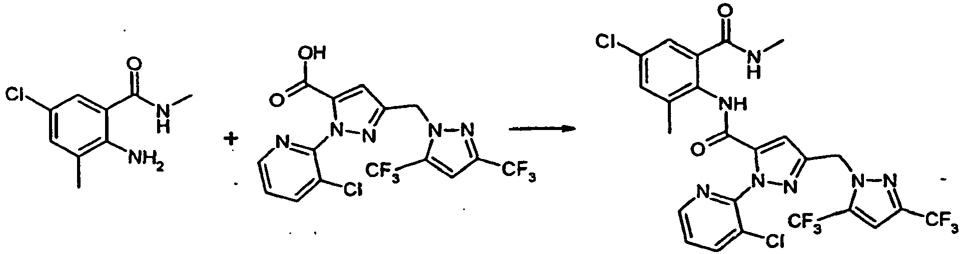

Die bei der Durchführung des erfindungsgemäßen Verfahrens (B) als Ausgangsstoffe benötigten Anthranilamide der Formel (II) sind bereits in Zusammenhang mit dem erfindungsgemäßen Verfahren (A) beschrieben worden.

Die bei der Durchführung des erfindungsgemäßen Verfahrens (B) weiterhin als Ausgangsstoffe benötigten heterocyclischen Carbonsäuren sind durch die Formel (IV) allgemein definiert. In dieser Formel (IV) stehen R⁶, A und Q bevorzugt, besonders bevorzugt, ganz besonders bevorzugt bzw. insbesondere bevorzugt für diejenigen Bedeutungen, die bereits im Zusammenhang mit der Beschreibung der erfindungsgemäßen Stoffe der Formel (I) als bevorzugt, besonders bevorzugt usw. für diese Reste genannt wurden.

Das erfindungsgemäße Verfahren (B) wird in Gegenwart eines Kondensationsmittels durchgeführt. Hierzu eignen sich alle für solche Kupplungsreaktionen üblichen Mittel. Beispielhaft genannt seien Säurehalogenidbildner wie Phosgen, Phosphortribromid, Phosphortrichlorid, Phosphorpentachlorid, Phosphoroxychlorid oder Thionylchlorid; Anhydridbildner wie Chlorameisensäureethylester, Chlorameisensäuremethylester, Chlorameisensäureisopropylester, Chlorameisensäureisobutylester oder Methansulfonylchlorid; Carbodiimide, wie N,N'-Dicyclohexylcarbodiimid (DCC) oder andere übliche Kondensationsmittel, wie Phosphorpentoxid, Polyphosphorsäure, 1,1'-Carbonyldiimidazol, 2-Ethoxy-N-ethoxycarbonyl-1,2-dihydrochinolin (EEDQ), Triphenylphosphin/Tetrachlorkohlenstoff, Brom-tripyrrolidinophosphonium-hexafluorophosphat, Bis(2-oxo-3-oxazolidinyl)phosphinchlorid oder Benzotriazol-1-yloxytris(dimethylamino)-phosphonium-hexafluorphosphat. Auf Polymer gestützte Reagenzien, wie z.B. polymer-gebundenes Cyclohexylcarbodiimid, können ebenfalls verwendet werden.

Das erfindungsgemäße Verfahren (B) wird gegebenenfalls in Gegenwart eines Katalysators durchgeführt. Beispielsweise genannt seien 4-Dimethylaminopyridin, 1-Hydroxy-benzotriazol oder Dimethylformamid.

Das erfindungsgemäße Verfahren (B) kann gegebenenfalls in Gegenwart eines für solche Reaktionen üblichen inerten organischen Verdünnungsmittel durchgeführt werden. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-amylether, Dioxan, Tetrahydrofuran, 1,2- Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dime-thylformamid, N,N-Dimethylacetamid, N-Methylformanilid, N-Methylpyrrolidon oder Hexamethylphosphorsäuretriamid; deren Gemische mit Wasser oder reines Wasser. Besonders bevorzugt verwendbar sind Dichlormethan und N,N-Dimethylformamid.

Die Reaktionstemperaturen können bei der Durchführung des erfindungsgemäßen Verfahrens (B) in einem größeren Bereich variiert werden. Im Allgemeinen arbeitet man bei Temperaturen von 0°C bis 150°C, vorzugsweise bei Temperaturen von 0°C bis 80°C.

Das erfindungsgemäße Verfahren wird im Allgemeinen unter Normaldruck durchgeführt. Es ist jedoch auch möglich, das erfindungsgemäße Verfahren unter erhöhtem oder vermindertem Druck - im Allgemeinen zwischen 0,1 bar und 10 bar - durchzuführen.

### Verfahren (C)

Verwendet man 2-[3-{[3,5-bis(trifluoromethyl)-1H-pyrazol-1-yl]methyl}-1-(3-chloropyridin-2-yl)-1H-pyazol-5-yl]-6-chlor-8-methyl-1H-3,1-benzoxazin-4-on und Methylamin so kann der Verlauf des erfindungsgemäßen Verfahrens (C) durch das folgende Formelschema veranschaulicht werden.

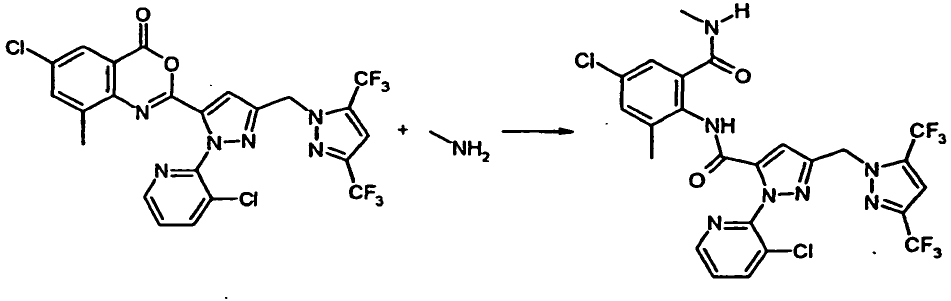

Die bei der Durchführung des erfindungsgemäßen Verfahrens (C) als Ausgangsstoffe benötigten Benzoxazinone sind durch die Formel (V) allgemein definiert. In dieser Formel (V) stehen R⁴, R⁵, R⁶ A, Q und n bevorzugt, besonders bevorzugt, ganz besonders bevorzugt bzw. insbesondere bevorzugt für diejenigen Bedeutungen, die bereits im Zusammenhang mit der Beschreibung der erfindungsgemäßen Stoffe der Formel (I) als bevorzugt, besonders bevorzugt usw. für diese Reste genannt wurden.

Benzoxazinone der Formel (V) sind neu. Sie werden beispielsweise erhalten, indem man
(J) Pyrazolcarbonsäure-Derivate der Formel (IV) 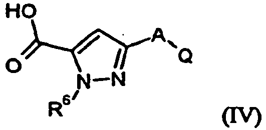 in welcher R⁶, A und Q die oben angegebenen Bedeutungen haben,
   mit Anthranilsäuren der Formel (XIV) 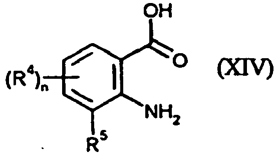 in welcher R⁴, R⁵ und n die oben angegebenen Bedeutungen haben, in Anwesenheit einer Base (z.B. Triethylamin oder Pyridin) und in Gegenwart eines Sulfonsäurechlorids (z.B. Methansulfonsäurechlorid) sowie gegebenenfalls in Gegenwart eines Verdünnungsmittels (z.B. Acetonitril) umsetzt.

Die bei der Durchführung des erfindungsgemäßen Verfahrens (J) als Ausgangsstoffe benötigten Pyrazolcarbonsäure-Derivate der Formel (IV) sind bereits oben in Zusammenhang mit dem erfindungsgemäßen Verfahren (A) beschrieben worden.

Die bei der Durchführung des erfindungsgemäßen Verfahrens (J) weiterhin als Ausgangsstoffe benötigten Anthranilsäuren sind durch die Formel (XIV) allgemein definiert. In dieser Formel (XIV) stehen R⁴, R⁵ und n bevorzugt, besonders bevorzugt, ganz besonders bevorzugt bzw. insbesondere bevorzugt für diejenigen Bedeutungen, die bereits im Zusammenhang mit der Beschreibung der erfindungsgemäßen Stoffe der Formel (I) als bevorzugt, besonders bevorzugt usw. für diese Reste genannt wurden.

Anthranilsäuren der Formel (XIV) sind bekannt und können nach allgemeinen Synthesemethoden hergestellt werden (vgl. z. B. Baker et al. J. Org. Chem. 1952, 149-153; G. Reissenweber et al., Angew. Chem 1981, 93, 914-915, PJ. Montoya-Pelaez, J. Org. Chem. 2006, 71, 5921-5929; F. E. Sheibley, J. Org. Chem. 1938, 3, 414-423, WO 2006023783).

Die Verbindungen der Formel (I) können gegebenenfalls in verschiedenen polymorphen Formen oder als Mischung verschiedener polymorpher Formen vorliegen. Sowohl die reinen Polymorphe als auch die Polymorphgemische sind Gegenstand der Erfindung und können erfindungsgemäß verwendet werden.

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Forsten, in Gärten-und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:
Aus der Ordnung der Anoplura (Phthiraptera) z.B. Damalinia spp., Haematopinus spp., Linognathus spp., Pediculus spp., Trichodectes spp..

Aus der Klasse der Arachnida z.B. Acarus siro, Aceria sheldoni, Aculops spp., Aculus spp., Amblyomma spp., Argas spp., Boophilus spp., Brevipalpus spp., Bryobia praetiosa, Chorioptes spp., Dermanyssus gallinae, Eotetranychus spp., Epitrimerus pyri, Eutetranychus spp., Eriophyes spp., Hemitarsonemus spp., Hyalomma spp., Ixodes spp., Latrodectus mactans, Metatetranychus spp., Oligonychus spp., Ornithodoros spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vasates lycopersici.

Aus der Klasse der Bivalva z.B. Dreissena spp..

Aus der Ordnung der Chilopoda z.B. Geophilus spp., Scutigera spp..

Aus der Ordnung der Coleoptera z.B. Acanthoscelides obtectus, Adoretus spp., Agelastica alni, Agriotes spp., Amphimallon solstitialis, Anobium punctatum, Anoplophora spp., Anthonomus spp., Anthrenus spp., Apogonia spp., Atomaria spp., Attagenus spp., Bruchidius obtectus, Bruchus spp., Ceuthorhynchus spp., Cleonus mendicus, Conoderus spp., Cosmopolites spp., Costelytra zealandica, Curculio spp., Cryptorhynchus lapathi, Dermestes spp., Diabrotica spp., Epilachna spp., Faustinus cubae, Gibbium psylloides, Heteronychus arator, Hylamorpha elegans, Hylotrupes bajulus, Hypera postica, Hypothenemus spp., Lachnosterna consanguinea, Leptinotarsa decemlineata, Lissorhoptrus oryzophilus, Lixus spp., Lyctus spp., Meligethes aeneus, Melolontha melolontha, Migdolus spp., Monochamus spp., Naupactus xanthographus, Niptus hololeucus, Oryctes rhinoceros, Oryzaephilus surinamensis, Otiorrhynchus sulcatus, Oxycetonia jucunda, Phaedon cochleariae, Phyllophaga spp., Popillia japonica, Premnotrypes spp., Psylliodes chrysocephala, Ptinus spp., Rhizobius ventralis, Rhizopertha dominica, Sitophilus spp., Sphenophorus spp., Sternechus spp., Symphyletes spp., Tenebrio molitor, Tribolium spp., Trogoderma spp., Tychius spp., Xylotrechus spp., Zabrus spp..

Aus der Ordnung der Collembola z.B. Onychiurus armatus.

Aus der Ordnung der Dermaptera z.B. Forficula auricularia.

Aus der Ordnung der Diplopoda z.B. Blaniulus guttulatus.

Aus der Ordnung der Diptera z.B. Aedes spp., Anopheles spp., Bibio hortulanus, Calliphora erythrocephala, Ceratitis capitata, Chrysomyia spp., Cochliomyia spp., Cordylobia anthropophaga, Culex spp., Cuterebra spp., Dacus oleae, Dermatobia hominis, Drosophila spp., Fannia spp., Gastrophilus spp., Hylemyia spp., Hyppobosca spp., Hypoderma spp., Liriomyza spp.. Lucilia spp., Musca spp., Nezara spp., Oestrus spp., Oscinella frit, Pegomyia hyoscyami, Phorbia spp., Stomoxys spp., Tabanus spp., Tannia spp., Tipula paludosa, Wohlfahrtia spp.

Aus der Klasse der Gastropoda z.B. Arion spp., Biomphalaria spp., Bulinus spp., Deroceras spp., Galba spp., Lymnaea spp., Oncomelania spp., Succinea spp..

Aus der Klasse der Helminthen z.B. Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp, Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp, Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.

Weiterhin lassen sich Protozoen, wie Eimeria, bekämpfen.

Aus der Ordnung der Heteroptera z.B. Anasa tristis, Antestiopsis spp., Blissus spp., Calocoris spp., Campylomma livida, Cavelerius spp., Cimex spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Nezara spp., Oebalus spp., Pentomidae, Piesma quadrata, Piezodorus spp., Psallus seriatus, Pseudacysta persea, Rhodnius spp., Sahlbergella singularis, Scotinophora spp., Stephanitis nashi, Tibraca spp., Triatoma spp.

Aus der Ordnung der Homoptera z.B. Acyrthosipon spp., Aeneolamia spp., Agonoscena spp., Aleurodes spp., Aleurolobus barodensis, Aleurothrixus spp., Amrasca spp., Anuraphis cardui, Aonidiella spp., Aphanostigma piri, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Doralis spp., Drosicha spp., Dysaphis spp., Dysmicoccus spp., Empoasca spp., Eriosoma spp., Erythroneura spp., Euscelis bilobatus, Geococcus coffeae, Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva fimbriolata, Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes vaporariorum, Trioza spp., Typhlocyba spp., Unaspis spp., Viteus vitifolii.

Aus der Ordnung der Hymenoptera z.B. Diprion spp., Hoplocampa spp., Lasius spp., Monomorium pharaonis, Vespa spp..

Aus der Ordnung der Isopoda z.B. Armadillidium vulgare, Oniscus asellus, Porcellio scaber.

Aus der Ordnung der Isoptera z.B. Reticulitermes spp., Odontotermes spp..

Aus der Ordnung der Lepidoptera z.B. Acronicta major, Aedia leucomelas, Agrotis spp., Alabama argillacea, Anticarsia spp., Barathra brassicae, Bucculatrix thurberiella, Bupalus piniarius, Cacoecia podana, Capua reticulana, Carpocapsa pomonella, Cheimatobia brumata, Chilo spp., Choristoneura fumiferana, Clysia ambiguella, Cnaphalocerus spp., Earias insulana, Ephestia kuehniella, Euproctis chrysorrhoea, Euxoa spp., Feltia spp., Galleria mellonella, Helicoverpa spp., Heliothis spp., Hofmannophila pseudospretella, Homona magnanima, Hyponomeuta padella, Laphygma spp., Lithocolletis blancardella, Lithophane antennata, Loxagrotis albicosta, Lymantria spp., Malacosoma neustria, Mamestra brassicae, Mocis repanda, Mythimna separata, Oria spp., Oulema oryzae, Panolis flammea, Pectinophora gossypiella, Phyllocnistis citrella, Pieris spp., Plutella xylostella, Prodenia spp., Pseudaletia spp., Pseudoplusia includens, Pyrausta nubilalis, Spodoptera spp., Thermesia gemmatalis, Tinea pellionella, Tineola bisselliella, Tortrix viridana, Trichoplusia spp..

Aus der Ordnung der Orthoptera z.B. Acheta domesticus, Blatta orientalis, Blattella germanica, Gryllotalpa spp., Leucophaea maderae, Locusta spp., Melanoplus spp., Periplaneta americana, Schistocerca gregaria.

Aus der Ordnung der Siphonaptera z.B. Ceratophyllus spp., Xenopsylla cheopis.

Aus der Ordnung der Symphyla z.B. Scutigerella immaculata.

Aus der Ordnung der Thysanoptera z.B. Baliothrips biformis, Enneothrips flavens, Frankliniella spp., Heliothrips spp., Hercinothrips femoralis, Kakothrips spp., Rhipiphorothrips cruentatus, Scirtothrips spp., Taeniothrips cardamoni, Thrips spp..

Aus der Ordnung der Thysanura z.B. Lepisma saccharina.

Zu den pflanzenparasitären Nematoden gehören z.B. Anguina spp., Aphelenchoides spp., Belonoaimus spp., Bursaphelenchus spp., Ditylenchus dipsaci, Globodera spp., Heliocotylenchus spp., Heterodera spp., Longidorus spp., Meloidogyne spp., Pratylenchus spp., Radopholus similis, Rotylenchus spp., Trichodorus spp., Tylenchorhynchus spp., Tylenchulus spp., Tylenchulus semipenetrans, Xiphinema spp..

Die erfindungsgemäßen Verbindungen können gegebenenfalls in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasmalike-organism) und RLO (Rickettsia-like-organism) verwendet werden. Sie lassen sich gegebenenfalls auch als Zwischen- oder Vorprodukte für die Synthese weiterer Wirkstoffe einsetzen.

Die Wirkstoffe können in die üblichen Formulierungen überführt werden, wie Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-Konzentrate, Wirkstoff-imprägnierte Naturstoffe, Wirkstoff-imprägnierte synthetische Stoffe, Düngemittel sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Die Herstellung der Formulierungen erfolgt entweder in geeigneten Anlagen oder auch vor oder während der Anwendung.

Als Hilfsstoffe können solche Stoffe Verwendung finden, die geeignet sind, dem Mittel selbst oder und/oder davon abgeleitete Zubereitungen (z.B. Spritzbrühen, Saatgutbeizen) besondere Eigenschaften zu verleihen, wie bestimmte technische Eigenschaften und/oder auch besondere biologische Eigenschaften. Als typische Hilfsmittel kommen in Frage: Streckmittel, Lösemittel und Trägerstoffe.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (poly-)Ether, der einfachen und substituierten Amine, Amide, Lactame (wie N-Alkylpyrrolidone) und Lactone, der Sulfone und Sulfoxide (wie Dimethylsysulfoxid).

Im Falle der Benutzung von Wasser als Streckmittel können z.B. auch organische Lösemittel als Hilfslösangsmittel verwendet werden. Als flüssige Lösemittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organische Material wie Papier, Sägemehl, Kokosnußschalen, Maiskolben und Tabakstengeln, als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage nicht-ionische und/oder ionische Stoffe, z.B. aus den Klassen der Alkohol-POE- und/oder POP-Ether, Säure- und/oder POP- POE-Ester, Alkyl-Aryl- und/oder POP-POE-Ether, Fett- und/oder POP- POE-Addukte, POE- und/oder POP-Polyol Derivate, POE- und/oder POP-Sorbitan- oder-Zucker-Addukte, Alky- oder Aryl-Sulfate, Sulfonate und Phosphate oder die entsprechenden PO-Ether-Addukte. Ferner geeignete Oligo- oder Polymere, z.B. ausgehend von vinylischen Monomeren, von Acrylsäure, aus EO und/oder PO allein oder in Verbindung mit z.B. (poly-) Alkoholen oder (poly-) Aminen. Ferner können Einsatz finden Lignin und seine Sulfonsäure-Derivate, einfache und modifizierte Cellulosen, aromatische und/oder aliphatische Sulfonsäuren sowie deren Addukte mit Formaldehyd.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Weitere Additive können Duftstoffe, mineralische oder vegetabile gegebenenfalls modifizierte Öle, Wachse und Nährstoffe (auch Spurennährstoffe), wie Salze von Eisen, Mangan, Bor, Kupfer, Kobalt, Molybdän und Zink sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel.

Die Formulierungen enthalten im allgemeinen zwischen 0,01 und 98 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen wie Insektiziden, Lockstoffen, Sterilantien, Bakteriziden, Akariziden, Nematiziden, Fungiziden, wachstumsregulierenden Stoffen, Herbiziden, Safenern, Düngemitteln oder Semiochemicals vorliegen.

Besonders günstige Mischpartner sind z.B. die folgenden:
Fungizide:
   Inhibitoren der Nucleinsäure Synthese
      Benalaxyl, Benalaxyl-M, Bupirimat, Chiralaxyl, Clozylacon, Dimethirimol, Ethirimol, Furalaxyl, Hymexazol, Metalaxyl, Metalaxyl-M, Ofurace, Oxadixyl, Oxolinsäure
   Inhibitoren der Mitose und Zellteilung
      Benomyl, Carbendazim, Diethofencarb, Fuberidazole, Pencycuron, Thiabendazol, Thiophanat-methyl, Zoxamid
   Inhibitoren der Atmungskette Komplex I
      Diflumetorim
   Inhibitoren der Atmungskette Komplex II
      Boscalid, Carboxin, Fenfuram, Flutolanil, Furametpyr, Mepronil, Oxycarboxin, Penthiopyrad, Thifluzamid
   Inhibitoren der Atmungskette Komplex III
      Azoxystrobin, Cyazofamid, Dimoxystrobin, Enestrobin, Famoxadon, Fenamidon, Fluoxastrobin, Kresoximmethyl, Metominostrobin, Orysastrobin, Pyraclostrobin, Picoxystrobin, Trifloxystrobin
   Entkoppler
      Dinocap, Fluazinam
   Inhibitoren der ATP Produktion
      Fentinacetat, Fentinchlorid, Fentinhydroxid, Silthiofam
   Inhibitoren der Aminosäure- und Proteinbiosynthese
      Andoprim, Blasticidin-S, Cyprodinil, Kasugamycin, Kasugamycinhydrochlorid Hydrat, Mepanipyrim, Pyrimethanil
   Inhibitoren der Signal-Transduktion
      Fenpiclonil, Fludioxonil, Quinoxyfen
   Inhibitoren der Fett- und Membran Synthese
      Chlozolinat, Iprodion, Procymidon, Vinclozolin
      Ampropylfos, Kalium-Ampropylfos, Edifenphos, Iprobenfos (IBP), Isoprothiolan, Pyrazophos Tolclofos-methyl, Biphenyl
      Iodocarb, Propamocarb, Propamocarb hydrochlorid
   Inhibitoren der Ergosterol Biosynthese
      Fenhexamid,
      Azaconazol, Bitertanol, Bromuconazol, Cyproconazol, Diclobutrazol, Difenoconazol, Diniconazol, Diniconazol-M, Epoxiconazol, Etaconazol, Fenbuconazol, Fluquinconazol. Flusilazol, Flutriafol, Furconazol, Furconazol-cis, Hexaconazol, Imibenconazol, Ipconazol, Metconazol, Myclobutanil, Paclobutrazol, Penconazol, Propiconazol, Prothioconazol, Simeconazol, Tebuconazol, Tetraconazol, Triadnmefon, Triadimenol, Triticonazol, Uniconazol, Voriconazol, Imazalil, Imazalilsulfat, Oxpoconazol, Fenarimol, Flurprimidol, Nuarimol, Pyrifenox, Triforin, Pefurazoat, Prochloraz, Triflumizol, Viniconazol,
      Aldimorph, Dodemorph, Dodemorphacetat, Fenpropimorph, Tridemorph, Fenpropidin, Spiroxamin,
      Naftifin, Pyributicarb, Terbinafin
   Inhibitoren der Zellwand Synthese
      Benthiavalicarb, Bialaphos, Dimethomorph, Flumorph, Iprovalicarb, Polyoxins, Polyoxorim, Validamycin A
   Inhibitoren der Melanin Biosynthese
      Capropamid, Diclocymet, Fenoxanil, Phtalid, Pyroquilon, Tricyclazol
   Resistenzinduktion
      Acibenzolar-S-methyl, Probenazol, Tiadinil
   Multisite
      Captafol, Captan, Chlorothalonil, Kupfersalze wie: Kupferhydroxid, Kupfernaphthenat, Kupferoxychlorid, Kupfersulfat, Kupferoxid, Oxin-Kupfer und Bordeaux Mischung, Dichlofluanid, Dithianon, Dodin, Dodin freie Base, Ferbam, Folpet, Fluorofolpet, Guazatin, Guazatinacetat, Iminoctadin, Iminoctadinalbesilat, Iminoctadintriacetat, Mankupfer, Mancozeb, Maneb, Metiram, Metiram Zink, Propineb, Schwefel und Schwefelpräparate enthaltend Calciumpolysulphid, Thiram, Tolylfluanid, Zineb, Ziram
   Unbekannter Mechanismus
      Amibromdol, Benthiazol, Bethoxazin, Capsimycin, Carvon, Chinomethionat, Chloropicrin, Cufraneb, Cyflufenamid, Cymoxanil, Dazomet, Debacarb, Diclomezine, Dichlorophen, Dicloran, Difenzoquat, Difenzoquat Methylsulphat, Diphenylamin, Ethaboxam, Ferimzon, flumetover, Flusulfamid, Fluopicolid, Fluoroimid, Hexachlorobenzol, 8-Hydroxychinolinsulfat, Irumamycin, Methasulphocarb, Metrafenon, Methyl Isothiocyanat, Mildiomycin, Natamycin, Nickel dimethyldithiocaibamat, Nitrothal-isopropyl, Octhilinon, Oxamocarb, Oxyfenthiin, Pentachlorophenol und Salze, 2-Phenylphenol und Salze, Piperalin, Propanosin -Natrium, Proquinazid, Pyrrolnitrin, Quintozen, Tecloftalam, Tecnazen, Triazoxid, Trichlamid, Zarilamid und 2,3,5,6-Tetrachlor-4-(methylsulfonyl)-pyridin, N-(4-Chlor-2-nitrophenyl)-N-ethyl-4-methyl-benzenesulfonamid, 2-Amino-4-methyl-N-phenyl-5-thiazolecarboxamid, 2-Chlor-N-(2,3-dihydro-1,1,3-trimethyl-1H-inden-4-yl)-3-pyridincarboxamid, 3-[5-(4-Chlorphenyl)-2,3-dimethylisoxazolidin-3-yl]pyridin, cis-1-(4-Chlorphenyl)-2-(1H-1,2,4 triazol-1-yl)-cycloheptanol, 2,4-Dihydro-5-methoxy-2-methyl-4-[[[[1-[3-(trifluoromethyl)-phenyl]-ethyliden]-amino]-oxy]-methyl]-phenyl]-3H-1,2,3-triazol-3-on (185336-79-2), Methyl 1-(2,3-dihydro-2,2-dimethyl-1H-inden-1-yl)-1H-imidazole-5-carboxylat, 3,4,5-Trichlor-2,6-pyridindicarbonitril, Methyl 2-[[[cyclopropyl[(4-methoxyphenyl) imino]methyl]thio]methyl]-.alpha.-(methoxymethylen)- benzacetat, 4-Chlor-alpha-propinyloxy-N-[2-[3-methoxy-4-(2-propinyloxy)phenyl]ethyl]-benzacetamide, (2S)-N-[2-[4-[[3-(4-chlorophenyl)-2-propinyl]oxy]-3-methoxyphenyl]ethyl]- 3-methyl-2-[(methylsulfonyl)amino]-butanamid, 5-Chlor-7-(4-methylpiperidin-1-yl)-6-(2,4,6-trifluoro-phenyl)[1,2,4]triazolo[1,5-a]pyrimidin, 5-Chlor-6-(2,4,6-trifluorophenyl)-N-[(1R)-1,2,2-trimethylpropyl][1,2,4]triazolo[1,5-a]pyrimidin-7-amin, 5-Chlor-N-[(1R)-1,2-dimethyl-propyl]-6-(2,4,6-trifluorophenyl) [1,2,4]triazolo[1,5-a]pyrimidin-7-aimne, N-[1-(5-Brom-3-chloropyridin-2-yl)ethyl]-2,4-dichloronicotinamid, N-(5-Brom-3-chlorpyridin-2-yl)methyl-2,4-dichlornicotinamid, 2-Butoxy-6-iod-3-propyl-benzopyranon-4-on, N-{(Z)-[(cyclopropyl-methoxy) imino][6-(difluormethoxy)-2,3-difluorphenyl]methyl}-2-benzacetamid, N-(3-Ethyl-3,5,5-trimethyl-cyclohexyl)-3-formylamino-2-hydroxy-benzamid, 2-[[[[1-[3(1Fluor-2-phenylethyl)oxy] phenyl] ethyliden]amino]oxy]methyl]-alpha-(methoxyimino)-N-methyl-alphaE-benzacetamid, N-{2-[3-Chlor-5-(trifluormethyl)pyridin-2-yl]ethyl}-2-(trifluoro-methyl)benzamid, N-(3',4'dichlor-5-fluorbiphenyl-2-yl)-3(difluormethyl)-1-methyt-1H-pyrazol-4carboxamid, N-(6-Methoxy-3-pyridinyl)-cyclopropan carboxamid, 1-[(4-Methoxyphenoxy)methyl]-2,2-dimethylpropyl-1H-imidazol-1- carbonsäure, 0-[1-[(4-Methoxyphenoxy)methyl]-2,2-dimethylpropyl]-1H-imidazol- 1- carbothioic acid, 2-(2-{[6-(3-Chlor-2-methylphenoxy)-S-fluorpyrimidin-4-yl]oxy}phenyl)-2-(methoxyimino)-N-methylacetamid
**Bakterizide:**
   Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.
**Insektizide / Akarizide / Nematizide:**
   Acetylcholinesterase (AChE) Inhibitoren
      Carbamate,
         zum Beispiel Alanycarb, Aldicarb, Aldoxycarb, Allyxycarb, Aminocarb, Bendiocarb, Benfuracarb, Bufencarb, Butacarb, Butocarboxim, Butoxycarboxim, Carbaryl, Carbofuran, Carbosulfan, Cloethocarb, Dimetilan, Ethiofencarb, Fenobucarb, Fenothiocarb, Formetanate, Furathiocarb, Isoprocarb, Metam-sodium, Methiocarb, Methomyl, Metolcarb, Oxamyl, Pirimicarb, Promecarb, Propoxur, Thiodicarb, Thiofanox, Trimethacarb, XMC, Xylylcarb, Triazamate
      Organophosphate,
         zum Beispiel Acephate, Azamethiphos, Azinphos (-methyl, -ethyl), Bromophos-ethyl, Bromfenvinfos (-methyl), Butathiofos, Cadusafos, Carbophenothion, Chlorethoxyfos, Chlorfenvinphos, Chlormephos, Chlorpyrifos (-methyl/-ethyl), Coumaphos, Cyanofenphos, Cyanophos, Chlorfenvinphos, Demeton-*S*-methyl, Demeton-*S*-methylsulphon, Dialifos, Diazinon, Dichlofenthion, Dichlorvos/DDVP, Dicrotophos, Dimethoate, Dimethylvinphos, Dioxabenzofos, Disulfoton, EPN, Ethion, Ethoprophos, Etrimfos, Famphur, Fenamiphos, Fenitrothion, Fensulfothion, Fenthion, Flupyrazofos, Fonofos, Formothion, Fosmethilan, Fosthiazate, Heptenophos, Iodofenphos, Iprobenfos, Isazofos, Isofenphos, Isopropyl O-salicylate, Isoxathion, Malathion, Mecarbam, Methacrifos, Methamidophos, Methidathion, Mevinphos, Monocrotophos, Naled, Omethoate, Oxydemeton-methyl, Parathion (-methyl/ -ethyl), Phenthoate, Phorate, Phosalone, Phosmet, Phosphamidon, Phosphocarb, Phoxim, Pirimiphos (-methyl/-ethyl), Profenofos, Propaphos, Propetamphos, Prothiofos, Prothoate,
      Pyraclofos, Pyridaphenthion, Pyridathion, Quinalphos, Sebufos, Sulfotep, Sulprofos, Tebupirimfos, Temephos, Terbufos, Teorachlorvinphos, Thiometon, Triazophos, Triclorfon, Vamidothion
   Natrium-Kanal-Moduiatoren / Spannungsabhängige Natrium-Kanal-Blocker
      Pyrethroide,
         zum Beispiel Acrinathrin, Allethrin (d-cis-trans, d-trans), Beta-Cyfluthrin, Bifenthrin, Bioallethrin, Bioallethrin-S-cyclopentyl-isomer, Bioethanomethrin, Biopermethrin, Bioresmethrin, Chlovaporthrin, Cis-Cypermethrin, Cis-Resmethrin, Cis-Permethrin, Clocythrin, Cycloprothrin, Cyfluthrin, Cyhalothrin, Cypermethrin (alpha-, beta-, theta-, zeta-), Cyphenothrin, Deltamethrin, Empenthrin (IR-isomer), Esfenvaleraie, Etofenprox, Fenfluthrin, Fenpropathrin, Fenpyrithrin, Fenvalerate, Flubrocythrinate, Flucythrinate, Flufenprox. Flumethrin, Fluvalinate, Fubfenprox, Gamma-Cyhalothrin, Imiprothrin, Kadethrin, Lambda- Cyhalothrin, Metofluthrin, Permethrin (cis-, trans-), Phenothrin (IR-trans isomer), Prallethrin, Profluthrin, Protrifenbute, Pyresmethrin, Resmethrin, RU 15525, Silafluofen, Tau-Fluvalinate, Tefluthrin, Terallethrin, Tetramethrin (-1R- isomer), Tralomethrin, Transfluthrin, ZXI 8901, Pyrethrins (pyrethrum)
      DDT
      Oxadiazine,
         zum Beispiel Indoxacarb
      Semicarbazon,
         zum Beispiel Metafhunizon (BAS3201)
   Acetylcholin-Rezeptor-Agonisten/-Antagonisten
      Chloronicotinyle,
         zum Beispiel Acetamiprid, Clothianidin, Dinotefuran, Imidacloprid, Nitenpyram, Nithiazine, Thiacloprid, Thiamethoxam
      Nicotine, Bensultap, Cartap
   Acetylcholin-Rezeptor-Modulatoren
      Spinosyne,
         zum Beispiel Spinosad
   GABA-gesteuerte Chlorid-Kanal-Antagonisten
      Organochlorine,
         zum Beispiel Camphechlor, Chlordane, Endosulfan, Gamma-HCH, HCH, Heptachlor, Lindane, Methoxychlor
      Fiprole,
         zum Beispiel Acetoprole, Ethiprole, Fipronil, Pyrafluprole, Pyriprole, Vaniliprole
   Chlorid-Kanal-Aktivatoren
      Mectine,
         zum Beispiel Abamectin, Emamectin, Emamectin-benzoate, Ivermectin, Lepimectin, Milbemycin
   Juvenilhormon-Mimetika,
      zum Beispiel Diofenolan, Epofenonane, Fenoxycarb, Hydroprene, Kinoprene, Methoprene, Pyriproxifen, Triprene
   Ecdysonagonisten/disruptoren
      Diacylhydrazine,
         zum Beispiel Chromafenozide, Halofenozide, Methoxyfenozide, Tebufenozide
   Inhibitoren der Chitinbiosynthese
      Benzoylharnstoffe,
         zum Beispiel Bistrifhiron, Chlofluazuron, Diflubenzuron, Fluazuron, Flucycloxuron, Flufenoxuron, Hexaflumuron, Lufenuron, Novaluron, Noviflumuron, Penfluron, Teflubenzuron, Triflumuron
      Buprofezin
      Cyromazine
   Inhibitoren der oxidativen Phosphorylierung, ATP-Disraptoren
      Diafenthiuron
      Organozinriverbindungen,
         zum Beispiel Azocyclotin, Cyhexatin, Fenbutatin-oxide
   Entkoppler der oxidativen Phoshorylierung durch Unterbrechung des H-Protongradienten
      Pyrrole,
         zum Beispiel Chlorfenapyr
      Dinitrophenole,
         zum Beispiel Binapacyrl, Dinobuton, Dinocap, DNOC
   Site-I-Elektronentransportinhibitoren
      METI's,
         zum Beispiel Fenazaquin, Fenpyroximate, Pyrimidifen, Pyridaben, Tebufenpyrad, Tolfenpyrad
      Hydrameihylnon
      Dicofol
   Site-II-Elektronentransportinhibitoren
      Rotenone
   Site-III-Elektronentransportinhibitoren
      Acequinocyl, Fluacrypyrim
   Mikrobielle Disruptoren der Insektendarmmembran
      Bacillus thuringiensis-Stämme
   Inhibitoren der Fettsynthese
      Tetronsäuren,
      zum Beispiel Spirodiclofen, Spiromesifen,
      Tetramsäuren,
      zum Beispiel Spirotetramat, cis-3-(2,5-dimethylphenyl)-4-hydroxy-8-methoxy-1-azaspiro[4.5]dec-3-en-2-on
      Carboxamide,
      zum Beispiel Flonicamid
      Oktopaminerge Agonisten,
      zum Beispiel Amitraz
   Inhibitoren der Magnesium-stimulierten ATPase,
      Propargite
      Nereistoxin-Analoge,
      zum Beispiel Thiocyclam hydrogen oxalate, Thiosultap-sodium
   Agonisten des Ryanodin-Rezeptors,
      Benzoesäuredicarboxamide,
      zum Beispiel Flubendiamid
      Anthranilamide,
      zum Beispiel Rynaxypyr (3-bromo-N-{4-chloro-2-methyl-6-[(methylamino)carbonyl]phenyl}-1-(3-chloropyridin-2-yl)-1H-pyrazole-5-carboxamide)
   Biologika, Hormone oder Pheromone
      Azadirachtin, Bacillus spec., Beauveria spec., Codlemone, Metarrhizium spec., Paecilomyces spec., Thuringiensin, Verticillium spec.
   Wirkstoffe mit unbekannten oder nicht spezifischen Wirkmechanismen
      Begasungsmittel,
         zum Beispiel Aluminium phosphide, Methyl bromide, Sulfuryl fluoride
      Fraßhemmer,
         zum Beispiel Cryolite, Flonicamid, Pymetrozine
      Milbenwachstumsinhibitoren,
         zum Beispiel Clofentezine, Etoxazole, Hexythiazox
      Amidoflumet, Benclothiaz, Benzoximate, Bifenazate, Bromopropylate, Buprofezin, Chinomethionat, Chlordimeform, Chlorobenzilate, Chloropicrin, Clothiazoben, Cycloprene, Cyflumetofen, Dicyclanil, Fenoxacrim, Fentrifanil, Flubenzimine, Flufenerim, Flutenzin, Gossyplure, Hydramethylnone, Japonilure, Metoxadiazone, Petroleum, Piperonyl butoxide, Potassium oleate, Pyridalyl, Sulfluramid, Tetradifon, Tetrasul, Triarathene,Verbutin

Auch eine Mischung mit anderen bekannten Wirkstoffen, wie Herbiziden, Düngemitteln, Wachstumsregulatoren, Safenern, Semiochemicals, oder auch mit Mitteln zur Verbesserung der Pflanzeneigenschaften ist möglich.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß. Insbesondere können Ammonium- oder Phosphoniumsalze und/oder Penetrationsforderer zur Wirkungssteigerung zugegeben werden.

Die erfindungsgemäßen Wirkstoffe können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Der Wirkstoffgehalt der aus den handelsüblichen Formulierungen bereiteten Anwendungsformen kann in weiten Bereichen variieren. Die Wirkstoffkonzentration der Anwendungsformen kann von 0,00000001 bis zu 95 Gew.-% Wirkstoff, vorzugsweise zwischen 0,00001 und 1 Gew.-% liegen.

Die Anwendung geschieht in einer den Anwendungsformen angepaßten üblichen Weise.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Saatgut sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Saatgut.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausf-uhrungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Emährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Zuckerrüben, Tomaten, Erbsen und andere Gemüsesorten, Baurnwolle, Tabak, Raps, sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid-tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, zB. Raps), IMI® (Toleranz gegen Imidazolinone) und STS® (Toleranz gegen Sulfonylharnstoffe z.B. Mais) vertrieben werden. Als Herbizid- resistente (konventionell auf Herbizid-Toleranz gezüchtete) Pflanzen seien auch die unter der Bezeichnung ClearHeld® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel I bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ekto- und Endoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:
Aus der Ordnung der Anoplurida z.B. Haematopinus spp., Linognathus spp., Pediculus spp., Phtirus spp., Solenopotes spp..

Aus der Ordnung der Mallophagida und den Unterordnungen Amblycerina sowie Ischnocerina z.B. Trimenopon spp., Menopon spp., Trinoton spp., Bovicola spp., Wemeckiella spp., Lepikentron spp., Damalina spp., Trichodectes spp., Felicola spp..

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopata spp., Philipomyia spp., Braula spp., Musca spp., Hydrotaea spp., Stomoxys spp., Haernatobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..

Aus der Ordnung der Siphonapterida z.B. Pulex spp., Ctenocephalides spp., Xenopsylla spp., Ceratophyllus spp..

Aus der Ordnung der Heteropterida z.B. Cimex spp., Triatoma spp., Rhodnius sps., Panstrongylus spp..

Aus der Ordnung der Blattarida z.B. Blatta orientalis, Periplaneta americana, Blattela germanica, Supella spp..

Aus der Unterklasse der Acari (Acarina) und den Ordnungen der Meta- sowie Mesostigmata z.B. Argas spp., Omithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia sps, Pneumonyssus spp., Sternostoma spp., Varroa spp..

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der *erfindungsgemäßen* Wirkstoffe eine wirtschaftlichere und einfachere Tierhaltimg möglich ist

Die Anwendung der erfindungsgemäßen Wirkstoffe geschieht im Veterinärsektor und bei der Tierhaltung in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändem, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, Sießfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus;
Hautflügler wie Sirex juvencus, Urocerus gigas, Urocerus gigas taignus, Urocerus augur;
Termiten wie Kalotermes flavicollis, Cryptotermes brevis, Heterotermes indicola, Reticulitermes flavipes, Reticulitermes santonensis, Reticulitermes lucifugus, Mastotermes darwiniensis, *Zootermopsis* nevadensis, *Coptotermes* formosanus;
Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Die anwendungsfertigen Mittel können gegebenenfalls noch weitere Insektizide und gegebenenfalls noch ein oder mehrere Fungizide enthalten.

Hinsichtlich möglicher zusätzlicher Zumischpartner sei auf die oben genannten Insektizide und Fungizide verwiesen.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Weiter können die erfindungsgemäßen Verbindungen allein oder in Kombinationen mit anderen Wirkstoffen als Antifouling-Mittel eingesetzt werden.

Die Wirkstoffe eignen sich auch zur Bekämpfung von tierischen Schädlingen im Haushalts-, Hygiene- und Vorratsschutz, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:
Aus der Ordnung der Scorpionidea z.B. Buthus occitanus.

Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.

Aus der Ordnung der Araneae z.B. Aviculariidae, Araneidae.

Aus der Ordnung der Opiliones z.B. Pseudoscorpiones chelifer, Pseudoscorpiones cheiridium, Opiliones phalangium.

Aus der Ordnung der Isopoda z.B. Oniscus asellus, Porcellio scaber.

Aus der Ordnung der Diplopoda z.B. Blaniulus guttulatus, Polydesmus spp..

Aus der Ordnung der Chilopoda z.B. Geophilus spp..

Aus der Ordnung der Zygentoma z.B. Ctenolepisma spp., Lepisma saccharina, Lepismodes inquilinus.

Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.

Aus der Ordnung der Saltatoria z.B. Acheta domesticus.

Aus der Ordnung der Dermaptera z.B. Forficula auricularia.

Aus der Ordnung der Isoptera z.B. Kalotermes spp., Reticulitermes spp.

Aus der Ordnung der Psocoptera z.B. Lepinatus spp., Liposcelis spp.

Aus der Ordnung der Coleoptera z.B. Anthrenus spp., Attagenus spp., Dermestes spp., Latheticus oryzae, Necrobia spp., Ptinus spp., Rhizopertha dominica, Sitophilus granarius, Sitophilus oryzae. Sitophilus zeamais, Stegobium paniceum.

Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.

Aus der Ordnung der Lepidoptera z.B. Achroia grisella, Galleria mellonella, Plodia interpunctella, Tinea cloacella, Tinea pellionella, Tineola bisselliella.

Aus der Ordnung der Siphonaptera z.B. Ctenocephalides canis, Ctenocephalides felis, Pulex irritans, Tunga penetrans, Xenopsylla cheopis.

Aus der Ordnung der Hymenoptera z.B. Camponotus herculeanus, Lasius fuliginosus, Lasius niger, Lasius umbratus, Monomorium pharaonis, Paravespula spp., Tetramorium caespitum.

Aus der Ordnung der Anoplura z.B. Pediculus humanus capitis, Pediculus humanus corporis, Pemphigus spp., Phylloera vastatrix, Phthirus pubis.

Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsänreestern, Carbamaten, Pyrethroiden, Neo-nicotinoiden, Wachstumsregulatoren oder Wirkstoffen aus anderen bekannten Insektizidklassen.

Die Anwendung erfolgt in Aerosolen, drucklosen Sprührrutteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

Die folgenden Herstellungs- und Verwendungsbeispiele illustrieren die Erfindung, ohne sie zu beschränken.

### Herstellungsbeispiele

### Beispiel 1

### 5-(3.5-Bis-trifluoromethyl-pyrazol-1-ylmethyl)-2-(3-chlor-pyridin-2-yl)-2H-pyrazol-3-carbonsäure (4-chlor-2-methyl-6-methlylcarbamoyl-phenyl)-amid (1-1-1):

Man legt 300 mg (509 µmol) 2-[5-(3,5-Bis-trifluoromethyl-pyrazol-1-ylmethyl)-2-(3-chlor-pyridin-2-yl)-2H-pyrazol-3-yll-6-chlor-8-methyl-benzo[d][1,3]oxazin-4-on in 3.3 ml Tetrahydrofuran vor und tropft 764 µl (1.53 mmol) einer 2 M Lösung aus Methylamin in Tetrahydrofuran hinzu. Es wird 1h bei 50°C gerührt, nach dem Abkühlen das Lösungsmittel i. Vak. entfernt und der Rückstand an Kieselgel (Cyclohexan/Ethylacetat = 2 : 1 → 1 :1) gereinigt.
Ausbeute: 200 mg (logP: 3.67)

Analog zu dem oben aufgefuhrten Beispiel (I-1-1) sowie der allgemeinen Beschreibung werden folgende Verbindungen der Formel (I-1) erhalten.

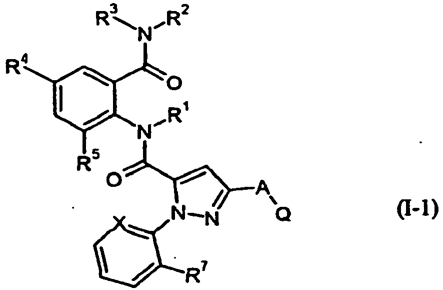

**Tabelle 1**

| **Nr.** | **R¹** | **R²** | **R³** | **R⁴** | **R⁵** | **A** | **Q** | **X** | **R⁷** | **logP** |
|---|---|---|---|---|---|---|---|---|---|---|
| I-1-2 | H | H | *i*-Pr | Cl | CH₃ | CH₂ | 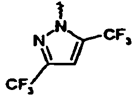 | N | Cl | 4.08 |
| I-1-3 | H | H | CH₃ | Cl | CH₃ | CH₂ | 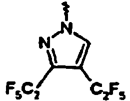 | N | Cl | 4.39 |
| I-1-4 | H | H | CH₃ | Cl | CH₃ | CH₂ | 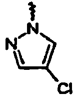 | N | Cl | 2.54 |
| I-1-5 | H | H | *i*-Pr | Cl | CH₃ | CH₂ | 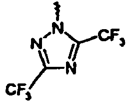 | N | Cl | 3.88 |
| I-1-6 | H | H | *i*-Pr | Cl | CH₃ | CH₂ | 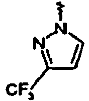 | N | Cl | 3.27 |
| I-1-7 | H | H | *i*-Pr | Cl | CH₃ | CH₂ | 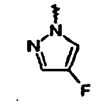 | N | Cl | 2.70 |
| I-1-8 | H | H | *i*-Pr | Cl | CH₃ | CH₂ | 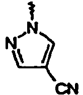 | N | Cl | 2.57 |
| I-1-9 | H | H | *i*-Pr | Cl | CH₃ | CH₂ | 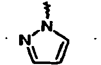 | N | Cl | 2.45 |
| I-1-10 | H | H | *i*-Pr | Cl | CH₃ | CH₂ | 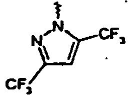 | CH | Cl | 4.70 |
| I-1-11 | H | H | CH₃ | Cl | CH₃ | CH₂ | 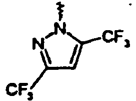 | CH | Cl | 4.12 |
| I-1-12 | H | H | *i*-Pr | Cl | CH₃ | CH₂ | 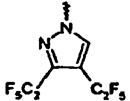 | N | Cl | 4.89 |
| I-1-13 | H | H | *i*-Pr | Br | CH₃ | CH₂ | 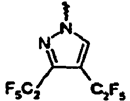 | N | Cl | 4.98 |
| I-1-14 | H | H | *i*-Pr | Cl | CH₃ | CH₂ | 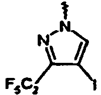 | N | Cl | 4.29 |
| I-1-15 | H | H | *i*-Pr | Cl | CH₃ | CH₂ | 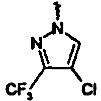 | N | Cl | 3.85 |
| I-1-16 | H | H | CH₃ | Cl | CH₃ | CH₂ | 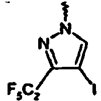 | N | Cl | 3.79 |
| I-1-17 | H | H | CH₃ | Cl | CH₃ | CH₂ | 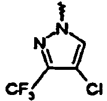 | N | Cl | 3.35 |
| I-1-18 | H | H | *i*-Pr | Cl | CH₃ | CH₂ | 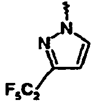 | N | Cl | 3.75 |
| I-1-19 | H | H | CH₃ | Cl | CH₃ | CH₂ | 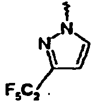 | N | Cl | 3.29 |
| I-1-20 | H | H | i-Pr | Br | CH₃ | CH₂ | 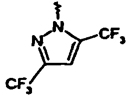 | N | Cl | 4.16 |
| I-1-21 | H | H | i-Pr | Cl | CH₃ | CH₂ | 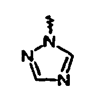 | N | Cl | 2.02 |
| I-1-22 | H | H | i-Pr | Br | CH₃ | CH₂ | 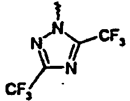 | N | Cl | 3.91 |
| I-1-23 | H | H | i-Pr | Cl | CH₃ | CH₂ | 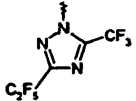 | N | Cl | 4.25 |
| I-1-24 | H | H | CH₃ | Br | CH₃ | CH₂ | 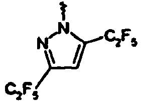 | N | Cl | 4.49 |
| I-1-25 | H | H | CH₃ | Br | CH₃ | CH₂ | 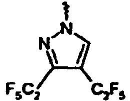 | N | Cl | 4.39 |
| I-1-26 | H | H | CH₃ | Cl | CH₃ | CH₂ | 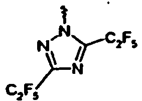 | N | Cl | 4.17 |
| I-1-27 | H | H | CH₃ | Cl | CH₃ | CH₂ | 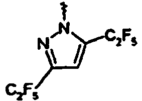 | N | Cl | 4.45 |
| I-1-28 | H | H | i-Pr | Cl | CH₃ | CH₂ | 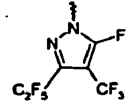 | N | Cl | 4.63 |
| I-1-29 | H | H | *i*-Pr | Cl | CH₃ | CH₂ | 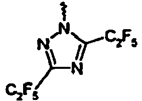 | N | Cl | 4.64 |
| I-1-30 | H | H | CH₃ | Cl | CH₃ | CH₂ | 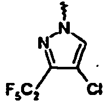 | N | Cl | 3.77 |
| I-1-31 | H | H | *i*-Pr | Cl | CH₃ | CH₂ | 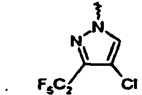 | N | Cl | 4.21 |
| I-1-32 | H | H | CH₂CN | Cl | CH₃ | CH₂ | 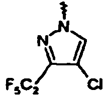 | N | Cl | 3.70 |
| I-1-33 | H | H | CH₂CN | Br | CH₃ | CH₂ | 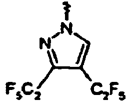 | N | Cl | 4.28 |
| I-1-34 | H | H | CH₃ | Cl | CH₃ | CH₂ | 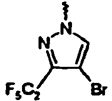 | N | Cl | 3,80 |
| I-1-35 | H | H | CH₃ | Cl | CH₃ | CH₂ | 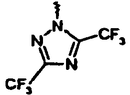 | N | Cl | 3,43 |
| I-1-36 | H | H | CH₃ | Br | CH₃ | CH₂ | 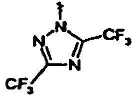 | N | Cl | 3,52 |
| I-1-37 | H | H | *i*-Pr | Br | CH₃ | CH₂ | 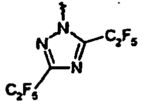 | N | Cl | 4,73 |
| I-1-38 | H | H | CH₃ | Cl | CH₃ | CH₂ | 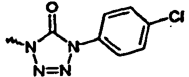 | N | Cl | 3,28 |
| I-1-39 | H | H | CH₃ | Br | CH₃ | CH₂ | 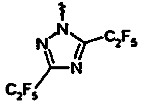 | N | Cl | 4,31 |
| I-1-40 | H | H | *i*-Pr | Cl | CH₃ | CH₂ | 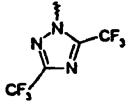 | CCl | Cl | 4,58 |
| I-1-41 | H | H | CH₃ | Cl | CH₃ | CH₂ | 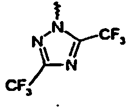 | CCl | Cl | 4,13 |
| I-1-42 | H | H | CH₃ | Cl | CH₃ | CH₂ | 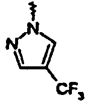 | N | Cl | 2,76 |
| I-1-43 | H | H | *i*-Pr | Cl | CH₃ | CH₂ | 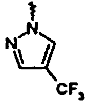 | N | Cl | 3,20 |
| I-1-44 | H | H | CH₃ | Cl | CH₃ | CH₂ | 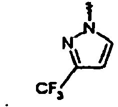 | N | Cl | 2,92 |
| I-1-45 | H | H | CH₃ | Cl | CH₃ | CH₂ | 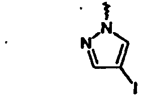 | N | Cl | 2,67 |
| I-1-46 | H | H | c-Pr | Cl | CH₃ | CH₂ | 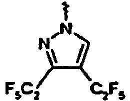 | N | Cl | 4,58 |
| I-1-47 | H | H | CH₂CN | Cl | CH₃ | CH₂ | 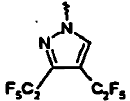 | N | Cl | 4,31 |
| I-1-48 | H | H | H | Cl | CH₃ | CH₂ | 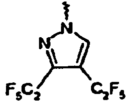 | N | Cl | 4,15 |
| I-1-49 | H | H | CH₂CN | Cl | CH₃ | CH₂ | 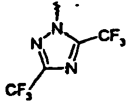 | N | Cl | 3,97 |
| I-1-50 | H | H | H | Cl | CH₃ | CH₂ | 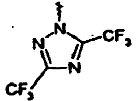 | N | Cl | 3,88 |
| I-1-51 | H | H | *i*-Pr | Cl | CH₃ | CH₂ | 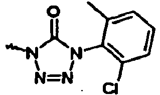 | N | Cl | 3,52 |
| I-1-52 | H | H | *c*-Pr | Br | CH₃ | CH₂ | 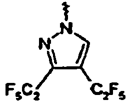 | N | Cl | 4,59 |
| I-1-53 | H | H | H | Br | CH₃ | CH₂ | 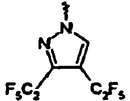 | N | Cl | 4,17 |
| I-1-54 | H | H | *c*-Pr | Cl | CH₃ | CH₂ | 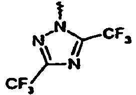 | N | Cl | 3,61 |
| I-1-55 | H | H | CH₂CN | Cl | CH₃ | CH₂ | 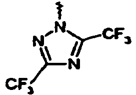 | N | Cl | 3,40 |
| I-1-56 | H | H | CH₂CN | Br | CH₃ | CH₂ | 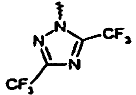 | N | Cl | 3,44 |
| I-1-57 | H | H | *c*-Pr | Br | CH₃ | CH₂ | 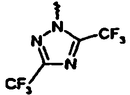 | N | Cl | 3,67 |
| I-1-58 | H | H | CH₃ | Br | CH₃ | CH₂ | 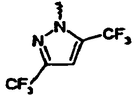 | N | Cl | 3,76 |
| I-1-59 | H | H | CH₂CN | Br | CH₃ | CH₂ | 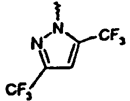 | N | Cl | 3,65 |
| I-1-60 | H | H | *c*-Pr | Br | CH₃ | CH₂ | 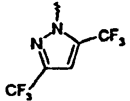 | N | Cl | 3,91 |
| I-1-61 | H | H | H | Br | CH₃ | CH₂ | 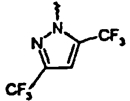 | N | Cl | 3,53 |
| I-1-62 | H | H | CH₃ | Cl | CH₃ | CH₂ | 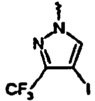 | N | Cl | 3,41 |
| I-1-63 | H | H | *i*-Pr | Cl | CH₃ | CH₂ | 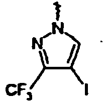 | N | Cl | 3,85 |
| I-1-64 | H | H | CH₃ | Cl | CH₃ | CH₂ | 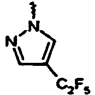 | N | Cl | 3,21 |
| I-1-65 | H | H | *i*-Pr | Cl | CH₃ | CH₂ | 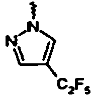 | N | Cl | 3,67 |
| I-1-66 | H | H | CH₃ | Cl | CH₃ | CH₂ | 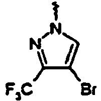 | N | Cl | 3,38 |
| I-1-67 | H | H | *i*-Pr | Cl | CH₃ | CH₂ | 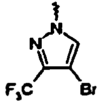 | N | Cl | 3,78 |
| I-1-68 | H | H | CH₃ | Br | CH₃ | CH₂ | 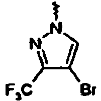 | N | Cl | 3,46 |
| I-1-69 | H | H | *i*-Pr | Br | CH₃ | CH₂ | 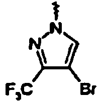 | N | Cl | 3,92 |
| I-1-70 | H | H | CH₃ | Br | CH₃ | CH₂ | 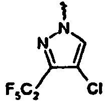 | N | Cl | 3,94 |
| I-1-71 | H | H | *i*-Pr | Br | CH₃ | CH₂ | 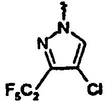 | N | Cl | 4,40 |
| I-1-72 | H | H | *c*-Pr | Br | CH₃ | CH₂ | 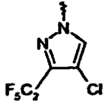 | N | Cl | 4,14 |
| I-1-73 | H | H | CH₂CN | Br | CH₃ | CH₂ | 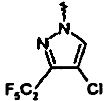 | N | Cl | 3,87 |
| I-1-74 | H | H | H | Br | CH₃ | CH₂ | 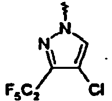 | N | Cl | 3,68 |
| I-1-75 | H | H | (*S*)-CH(CH₃) CH₂SCH₃ | Cl | CH₃ | CH₂ | 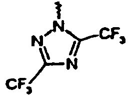 | N | Cl | 4,04 |
| I-1-76 | H | H | C(CH₃)₂ CH₂SCH₃ | Cl | CH₃ | CH₂ | 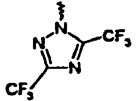 | N | Cl | 4,49 |
| I-1-77 | H | H | (*S)*-CH(CH₃) CH₂O(C=O) NHC₂H₅ | Cl | CH₃ | CH₂ | 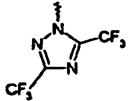 | N | Cl | 3,58 |
| I-1-78 | H | H | (*S*)-CH(CH₃) CH₂O(C=O) NHCH₃ | Cl | CH₃ | CH₂ | 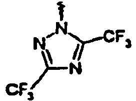 | N | Cl | 3,36 |
| I-1-79 | H | H | CH₂CN | Cl | CH₃ | CH₂ | 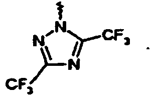 | N | Cl | 3,62 |
| I-1-80 | H | H | CH₃ | Br | CH₃ | CH₂ | 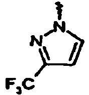 | N | Cl | 2,99 |
| I-1-81 | H | H | *i*-Pr | Br | CH₃ | CH₂ | 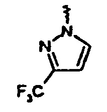 | N | Cl | 3,46 |
| I-1-82 | H | H | *c*-Pr | Br | CH₃ | CH₂ | 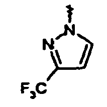 | N | Cl | 3,18 |
| I-1-83 | H | H | H | Br | CH₃ | CH₂ | 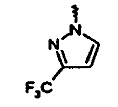 | N | Cl | 2,75 |
| I-1-84 | H | H | (*S*)-CH(CH₃) CH₂SCH₃ | H | CH₃ | CH₂ | 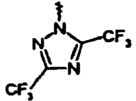 | N | Cl | 3,62 |
| I-1-85 | H | H | CH₂CN | Br | CH₃ | CH₂ | 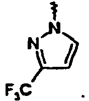 | N | Cl | 2,98 |
| I-1-86 | H | H | CH₃ | Cl | CH₃ | CH₂ | 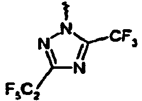 | N | Cl | 3,78 |
| I-1-87 | H | H | (*S*)-CH(CH₃) CH₂SO₂CH₃ | H | CH₃ | CH₂ | 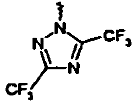 | N | Cl | 2,92 |
| I-1-88 | H | H | CH₃ | Cl | CH₃ | CH₂ | 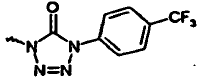 | N | Cl | 3,61 |
| I-1-89 | H | H | *i*-Pr | Cl | CH₃ | CH₂ | 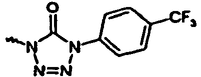 | N | Cl | 3,96 |
| I-1-90 | H | H | CH₃ | Cl | CH₃ | CH₂ | 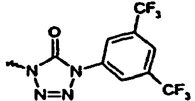 | N | Cl | 4,08 |
| I-1-91 | H | H | *i*-Pr | Cl | CH₃ | CH₂ | 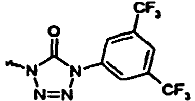 | N | Cl | 4,51 |
| I-1-92 | H | H | (*S*)-CH(CH3) CH₂SOCH₃ | H | CH₃ | CH₂ | 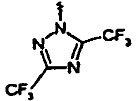 | N | Cl | 2,65 |
| I-1-93 | H | H | *c*-Pr | Br | CH₃ | CH₂ | 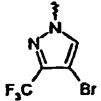 | N | Cl | 3,69 |
| I-1-94 | H | H | H | Cl | CH₃ | CH₂ | 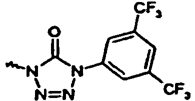 | N | Cl | 3,85 |
| I-1-95 | H | H | CH₂CN | Cl | CH₃ | CH₂ | 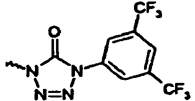 | N | Cl | 4,01 |
| I-1-96 | H | H | CH₃ | Cl | CH₃ | CH₂ | 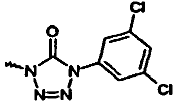 | N | Cl | 3,90 |
| I-1-97 | H | H | CH₂CN | Br | CH₃ | CH₂ | 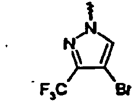 | N | Cl | 3,45 |
| I-1-98 | H | H | C(CH₃)₂ CH₂SCH₃ | Br | CH₃ | CH₂ | 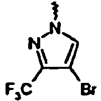 | N | Cl | 4,47 |
| I-1-99 | H | H | (*S*)-CH(CH₃) CH₂SCH₃ | Br | CH₃ | CH₂ | 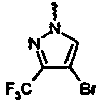 | N | Cl | 4,10 |
| I-1-100 | H | H | H | Br | CH₃ | CH₂ | 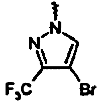 | N | Cl | 3,24 |
| I-1-101 | H | H | (*S*)-CH(CH₃) CH₂SO₂CH₃ | Br | CH₃ | CH₂ | 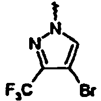 | N | Cl | 3,32 |
| I-1-102 | H | H | CH₃ | Cl | CH₃ | CH₂ | 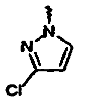 | N | Cl | 2,51 |
| I-1-103 | H | H | *i*-Pr | Cl | CH₃ | CH₂ | 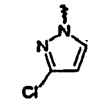 | N | Cl | 2,96 |
| I-1-104 | H | H | CH₃ | Cl | CH₃ | CH₂ | 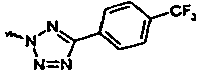 | N | Cl | 3,79 |
| I-1-105 | H | H | *i*-Pr | Cl | CH₃ | CH₂ | 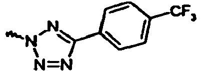 | N | Cl | 4,14 |
| I-1-106 | H | H | H | Cl | CH₃ | CH₂ | 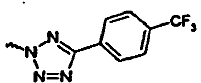 | N | Cl | 3,46 |
| I-1-107 | H | H | CH₂CN | Cl | CH₃ | CH₂ | 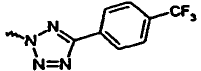 | N | Cl | 3,65 |
| I-1-108 | H | H | CH₃ | Cl | CH₃ | CH₂ | 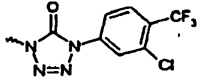 | N | Cl | 3,90 |
| I-1-109 | H | H | CH₃ | Cl | CH₃ | CH₂ | 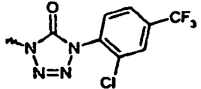 | N | Cl | 3,51 |
| I-1-110 | H | H | CH₃ | Cl | CH₃ | CH₂ | 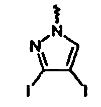 | N | Cl | 3,13 |
| I-1-111 | H | H | *i*-Pr | Cl | CH₃ | CH₂ | 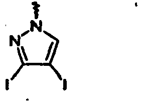 | N | Cl | 3,58 |
| I-1-112 | H | H | CH₃ | Cl | CH₃ | CH₂ | 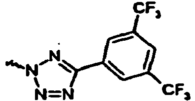 | N | Cl | 4,28 |
| I-1-113 | H | H | (*S*)-CH(CH₃) CH₂SOCH₃ | Br | CH₃ | CH₂ | 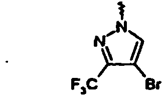 | N | Cl | 2,97 |
| I-1-114 | H | H | H | Cl | CH₃ | CH₂ | 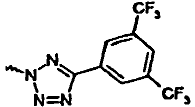 | N | Cl | 4,03 |
| I-1-115 | H | H | *i*-Pr | Cl | CH₃ | CH₂ | 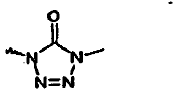 | N | Cl | 2,31 |
| I-1-116 | H | H | CH₃ | Cl | CH₃ | CH₂ | 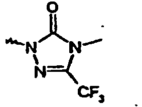 | N | Cl | 2,44 |
| I-1-117 | H | H | *i*-Pr | Cl | CH₃ | CH₂ | 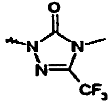 | N | Cl | 2,85 |
| I-1-118 | H | H | CH₃ | Cl | CH₃ | CH₂ | 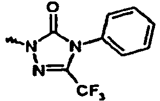 | N | Cl | 3,10 |
| I-1-119 | H | H | *i*-Pr | Cl | CH₃ | CH₂ | 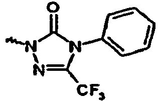 | N | Cl | 3,54 |
| I-1-120 | H | H | (*S*)-CH(CH₃) CH₂SO(=NH) CH₃ | Cl | CH₃ | CH₂ | 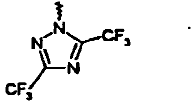 | N | Cl | 2,75 |
| I-1-121 | H | H | C(CH₃)₂ CH₂SO₂CH₃ | Br | CH₃ | CH₂ | 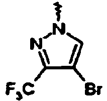 | N | Cl | 3,57 |
| I-1-122 | H | H | CH₃ | Cl | CH₃ | CH₂ | 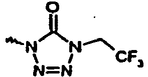 | N | Cl | 2,51 |
| I-1-123 | H | H | *i*-Pr | Cl | CH₃ | CH₂ | 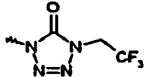 | N | Cl | 2,92 |
| I-1-124 | H | H | (*S*)-CH(CH₃) CH₂SCH₃ | Cl | CH₃ | CH₂ | 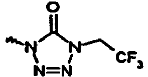 | N | Cl | 3,13 |
| I-1-125 | H | H | CH₃ | Cl | CH₃ | CH₂ | 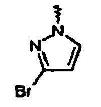 | N | Cl | 2,56 |
| I-1-126 | H | H | *i*-Pr | Cl | CH₃ | CH₂ | 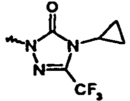 | N | Cl | 3,18 |
| I-1-127 | H | H | CH₃ | Cl | CH₃ | CH₂ | 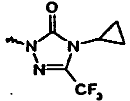 | N | Cl | 2,74 |
| I-1-128 | H | H | C(CH₃)₂ CH₂SOCH₃ | Cl | CH₃ | CH₂ | 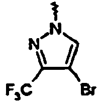 | N | Cl | 3,25 |
| I-1-129 | H | H | (*S*)-CH(CH₃) CH₂SO₂CH₃ | Cl | CH₃ | CH₂ | 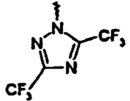 | N | Cl | 3,27 |
| I-1-130 | H | H | (*S*)-CH(CH₃) CH₂SCH₃ | Cl | CH₃ | CH₂ | 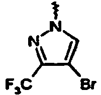 | N | Cl | 4,11 |
| I-1-131 | H | H | C(CH₃)2 CH₂SCH₃ | Cl | CH₃ | CH₂ | 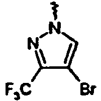 | N | Cl | 4,48 |
| I-1-132 | H | H | CH₃ | Cl | CH₃ | CH₂ | 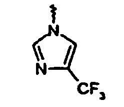 | N | Cl | 2,39 |
| I-1-133 | H | H | *i*-Pr | Cl | CH₃ | CH₂ | 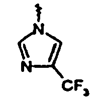 | N | Cl | 2,77 |
| I-1-134 | H | H | C(CH₃)₂ CH₂SCH₃ | Br | CH₃ | CH₂ | 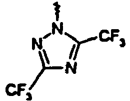 | N | Cl | 4,63 |
| I-1-135 | H | H | (*S*)-CH(CH₃) CH₂SCH₃ | Br | CH₃ | CH₂ | 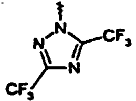 | N | Cl | 4,24 |
| I-1-136 | H | H | C(CH₃)2 CH₂SOCH₃ | Cl | CH₃ | CH₂ | 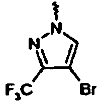 | N | Cl | 3,23 |
| I-1-137 | H | H | C(CH₃)₂ CH₂SOCH₃ | Cl | CH₃ | CH₂ | 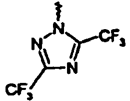 | N | Cl | 3,22 |
| I-1-138 | H | H | (*S*)-CH(CH₃) CH₂SOCH₃ | Cl | CH₃ | CH₂ | 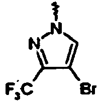 | N | Cl | 2,91 |
| I-1-139 | H | H | C(CH₃)2 CH₂SO₂CH₃ | Cl | CH₃ | CH₂ | 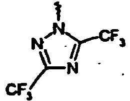 | N | Cl | 3,50 |
| I-1-140 | H | H | (*S*)-CH(CH₃) CH₂SOCH₃ | Br | CH₃ | CH₂ | 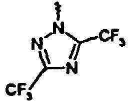 | N | Cl | 3,05 |
| I-1-141 | H | H | (*S*)-CH(CH₃) CH₂SO₂CH₃ | Cl | CH₃ | CH₂ | 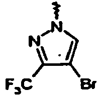 | N | Cl | 3,26 |
| I-1-142 | H | H | C(CH₃)2 CH₂SO₂CH₃ | Br | CH₃ | CH₂ | 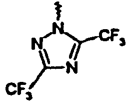 | N | Cl | 3,67 |
| I-1-143 | H | H | C(CH₃)₂ CH₂SOCH₃ | Br | CH₃ | CH₂ | 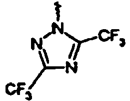 | N | Cl | 3,20 |
| I-1-144 | H | H | C(CH₃)₂ CH₂SOCH₃ | Br | CH₃ | CH₂ | 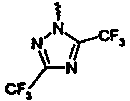 | N | Cl | 3,38 |
| I-1-145 | H | H | C(CH₃)₂ CH₂SO₂CH₃ | Cl | CH₃ | CH₂ | 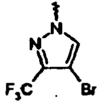 | N | Cl | 3,56 |
| I-1-146 | H | H | (*S*)-CH(CH₃) CH₂SO₂CH₃ | Br | CH₃ | CH₂ | 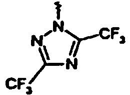 | N | Cl | 3,36 |
| I-1-147 | H | H | (*S*)-CH(CH₃) CH₂SOCH₃ | Cl | CH₃ | CH₂ | 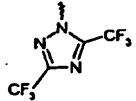 | N | Cl | 2,95 |
| I-1-148 | H | H | CH₃ | Cl | CH₃ | CH₂ | 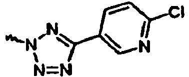 | N | Cl | 2,91 |
| I-1-149 | H | H | *i*-Pr | Cl | CH₃ | CH₂ | 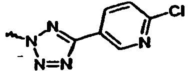 | N | Cl | 3,41 |
| I-1-150 | H | H | CH₃ | Cl | CH₃ | CH₂ | 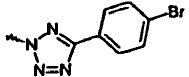 | N | ci | 3,70 |
| I-1-151 | H | H | *i*-Pr | Cl | CH₃ | CH₂ | 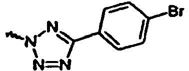 | N | Cl | 4,20 |
| I-1-152 | H | H | (*S*)-CH(CH₃) CH₂SCH₃ | Cl | CH₃ | CH₂ | 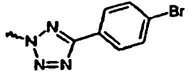 | N | Cl | 4,41 |
| I-1-153 | H | H | CH₃ | Cl | CH₃ | CH₂ | 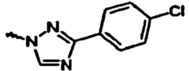 | N | Cl | 3,07 |
| I-1-154 | H | H | *i*-Pr | Cl | CH₃ | CH₂ | 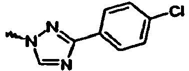 | N | Cl | 3,56 |
| I-1-155 | H | H | CH₃ | Cl | CH₃ | CH₂ | 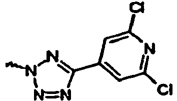 | N | Cl | 3,69 |
| I-1-156 | H | H | *i*-Pr | Cl | CH₃ | CH₂ | 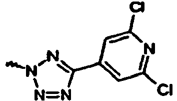 | N | Cl | 4,17 |
| I-1-157 | H | H | (*S*)-CH(CH₃) CH₂SCH₃ | Cl | CH₃ | CH₂ | 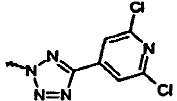 | N | Cl | 4,36 |
| I-1-158 | H | H | (*S*)-CH(CH₃) CH₂SCH₃ | Cl | CH₃ | CH₂ | 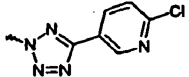 | N | Cl | 3,67 |
| I-1-159 | H | H | (*S*)-CH(CH₃) CH₂SCH₃ | Cl | CH₃ | CH₂ | 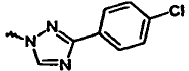 | N | Cl | 3,78 |
| I-1-160 | H | H | CH₃ | Cl | CH₃ | CH₂ | 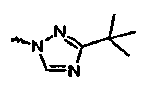 | N | Cl | 2,39 |
| I-1-161 | H | H | *i*-Pr | Cl | CH₃ | CH₂ | 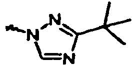 | N | Cl | 2,87 |
| I-1-162 | H | H | (*S*)-CH(CH₃) CH₂SCH₃ | Cl | CH₃ | CH₂ | 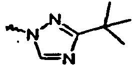 | N | Cl | 3,10 |
| I-1-163 | H | H | CH₃ | Cl | CH₃ | CH₂ | 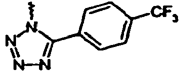 | N | Cl | 3,15 |
| I-1-164 | H | H | NH[S(=O)₂] N(CH₃)₂ | Cl | CH₃ | CH₂ | 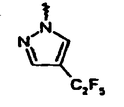 | N | Cl | 3,35 |
| I-1-165 | H | H | CH₃ | Cl | CH₃ | CH₂ | 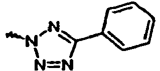 | N | Cl | 3,09 |
| I-1-166 | H | H | *i*-Pr | Cl | CH₃ | CH₂ | 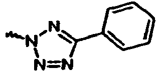 | N | Cl | 3,60 |
| I-1-167 | H | =S(*i-*Pr)₂ | | Cl | CH₃ | CH₂ | 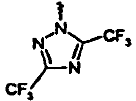 | N | Cl | 4,44 |
| I-1-168 | H | H | *i*-Pr | Cl | CH₃ | CH₂ | 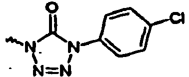 | N | Cl | 3,79 |
| I-1-169 | H | H | *i*-Pr | CN | CH₃ | CH₂ | 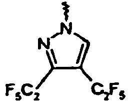 | N | Cl | 4,55 |
| I-1-170 | H | H | CH₃ | CN | CH₃ | CH₂ | 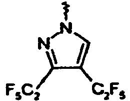 | N | Cl | 4,08 |
| I-1-171 | H | H | CH₃ | CN | CH₃ | CH₂ | 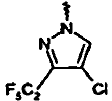 | N | Cl | 3,44 |
| I-1-172 | H | H | *i*-Pr | CN | CH₃ | CH₂ | 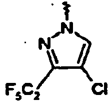 | N | Cl | 3,87 |
| I-1-173 | H | H | *c*-Pr | CN | CH₃ | CH₂ | 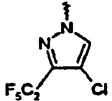 | N | Cl | 3,59 |
| I-1-174 | H | H | H | CN | CH₃ | CH₂ | 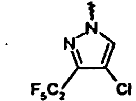 | N | Cl | 3,20 |
| I-1-175 | H | H | CH₂CN | CN | CH₃ | CH₂ | 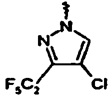 | N | Cl | 3,40 |
| I-1-176 | H | H | CH₃ | CN | CH₃ | CH₂ | 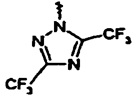 | N | Cl | 3,11 |
| I-1-177 | H | H | *i*-Pr | CN | CH₃ | CH₂ | 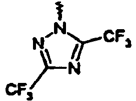 | N | Cl | 3,53 |
| I-1-178 | H | H | *c*-Pr | CN | CH₃ | CH₂ | 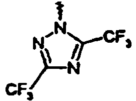 | N | Cl | 3,31 |
| I-1-179 | H | H | H | CN | CH₃ | CH₂ | 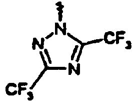 | N | Cl | 2,91 |
| I-1-180 | H | H | CH₂CN | CN | CH₃ | CH₂ | 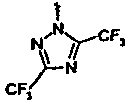 | N | Cl | 3,11 |
| I-1-181 | H | H | CH₃ | CN | CH₃ | CH₂ | 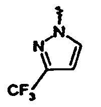 | N | Cl | 2,57 |
| I-1-182 | H | H | *i*-Pr | CN | CH₃ | CH₂ | 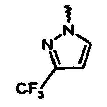 | N | Cl | 2,96 |
| I-1-183 | H | H | *c*-Pr | CN | CH₃ | CH₂ | 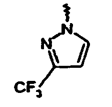 | N | Cl | 2,75 |
| I-1-184 | H | H | H | CN | CH₃ | CH₂ | 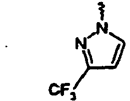 | N | Cl | 2,42 |
| I-1-185 | H | H | CH₂CN | CN | CH₃ | CH₂ | 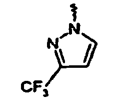 | N | Cl | 2,62 |
| I-1-186 | H | H | CH₃ | CN | CH₃ | CH₂ | 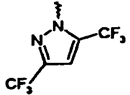 | N | Cl | 3,35 |
| I-1-187 | H | H | *i*-Pr | CN | CH₃ | CH₂ | 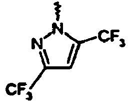 | N | Cl | 3,80 |
| I-1-188 | H | H | *c*-Pr | CN | CH₃ | CH₂ | 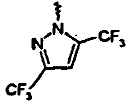 | N | Cl | 3,56 |
| I-1-189 | H | H | H | CN | CH₃ | CH₂ | 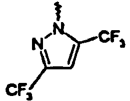 | N | Cl | 3,15 |
| I-1-190 | H | H | CH₂CN | CN | CH₃ | CH₂ | 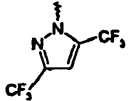 | N | Cl | 3,28 |
| I-1-191 | H | H | *c*-Pr | CN | CH₃ | CH₂ | 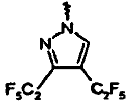 | N | Cl | 4,29 |
| I-1-192 | H | H | H | CN | CH₃ | CH₂ | 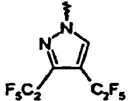 | N | Cl | 3,91 |
| I-1-193 | H | H | CH₂CN | CN | CH₃ | CH₂ | 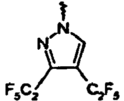 | N | Cl | 4,04 |
| I**-**1-194 | H | | 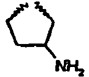 | CN | CH₃ | CH₂ | 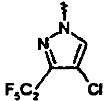 | N | Cl | 2,18 |
| I-1-195 | H | H | (*S*)-CH(CH₃) CH₂SCH₃ | F | CH₃ | CH₂ | 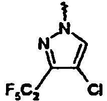 | N | Cl | 4,13 |
| I-1-196 | H | H | 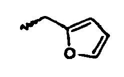 | F | CH₃ | CH₂ | 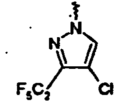 | N | Cl | 3,90 |
| I-1-197 | H | H | 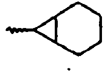 | CN | CH₃ | CH₂ | 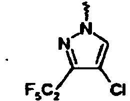 | N | Cl | 4,50 |
| I-1-198 | H | H | 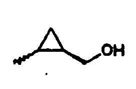 | CN | CH₃ | CH₂ | 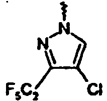 | N | Cl | 3,11 |
| I-1-199 | H | H | 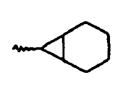 | F | CH₃ | CH₂ | 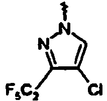 | N | Cl | 4,55 |
| I-1-200 | H | | 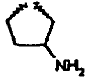 | F | CH₃ | CH₂ | 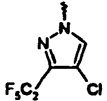 | N | Cl | 2,19 |
| I-1-201 | H | H | 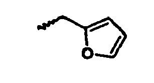 | Cl | CH₃ | CH₂ | 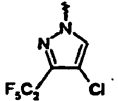 | N | Cl | 4,21 |
| I-1-202 | H | H | 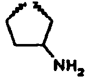 | Cl | CH₃ | CH₂ | 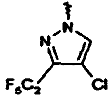 | N | Cl | 2,30 |
| I-1-203 | H | H | 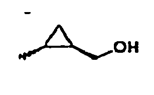 | F | CH₃ | CH₂ | 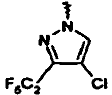 | N | Cl | 3,14 |
| I-1-204 | H | H | (*S*)-CH(CH₃) CH₂SO₂CH₃ | F | CH₃ | CH₂ | 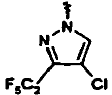 | N | Cl | 3,34 |
| I-1-205 | H | H | 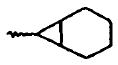 | Cl | CH₃ | CH₂ | 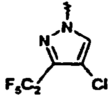 | N | Cl | 4,88 |
| I-1-206 | H | H | (*S*)-CH(CH₃) CH₂SCH₃ | Cl | CH₃ | CH₂ | 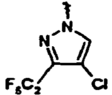 | N | Cl | 4,41 |
| I-1-207 | H | H | 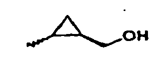 | Cl | CH₃ | CH₂ | 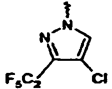 | N | Cl | 3,37 |
| I-1-208 | H | H | 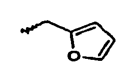 | CN | CH₃ | CH₂ | 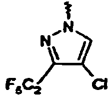 | N | Cl | 3,90 |
| I-1-209 | H | H | (*S*)-CH(CH₃) CH₂SCH₃ | CN | CH₃ | CH₂ | 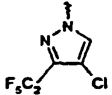 | N | Cl | 4,05 |
| I-1-210 | H | H | (*S*)-CH(CH₃) CH₂SO₂CH₃ | CN | CH₃ | CH₂ | 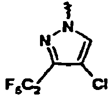 | N | Cl | 3,34 |
| I-1-211 | H | H | (*S*)-CH(CH₃) CH₂SOCH₃ | CN | CH₃ | CH₂ | 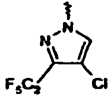 | N | Cl | 3,02 |
| I-1-212 | H | H | (*S*)-CH(CH₃) CH₂SOCH₃ | Cl | CH₃ | CH₂ | 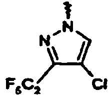 | N | Cl | 3,25 |
| I-1-213 | H | H | (*S*)-CH(CH₃) CH₂SO₂CH₃ | Cl | CH₃ | CH₂ | 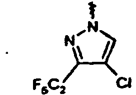 | N | Cl | 3,60 |
| I-1-214 | H | H | 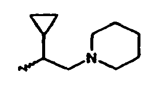 | Cl | CH₃ | CH₂ | 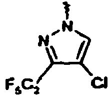 | N | Cl | 2,77 |
| I-1-215 | H | H | 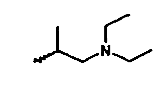 | Cl | CH₃ | CH₂ | 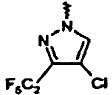 | N | Cl | 2,62 |
| I-1-216 | H | H | (*S*)-CH(CH₃) CH₂SOCH₃ | F | CH₃ | CH₂ | 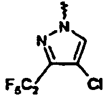 | N | Cl | 3,03 |
| I-1-217 | H | H | *i*-Pr | F | CH₃ | CH₂ | 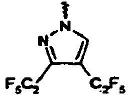 | N | Cl | 4,47 |
| I-1-218 | H | H | *i*-Pr | F | CH₃ | CH₂ | 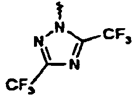 | N | Cl | 3,55 |
| I-1-219 | H | H | CH₃ | F | CH₃ | CH₂ | 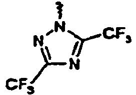 | N | Cl | 3,12 |
| I-1-220 | H | H | CH₃ | F | CH₃ | CH₂ | 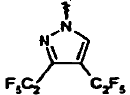 | N | Cl | 4,06 |
| I-1-221 | H | H | CH₃ | F | CH₃ | CH₂ | 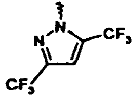 | N | Cl | 3,32 |
| I-1-222 | H | H | CH₃ | F | CH₃ | CH₂ | 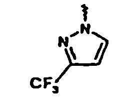 | N | Cl | 2,58 |
| I-1-223 | H | H | CH₃ | F | CH₃ | CH₂ | 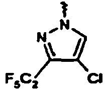 | N | Cl | 3,47 |
| I-1-224 | H | H | *i*-Pr | F | CH₃ | CH₂ | 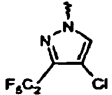 | N | Cl | 3,89 |
| I-1-225 | H | H | *i*-Pr | F | CH₃ | CH₂ | 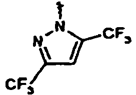 | N | Cl | 3,77 |
| I-1-226 | H | H | *i*-Pr | F | CH₃ | CH₂ | 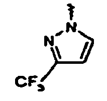 | N | Cl | 3,00 |
| I-1-227 | H | H | CH₃ | I | CH₃ | CH₂ | 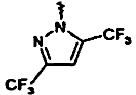 | N | Cl | 3,87 |
| I-1-228 | H | H | C₂H₅ | I | CH₃ | CH₂ | 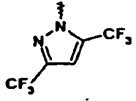 | N | Cl | 4,06 |
| I-1-229 | H | H | *n*-Pr | I | CH₃ | CH₂ | 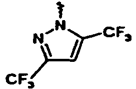 | N | Cl | 4,33 |
| I-1-230 | H | H | *i*-Pr | I | CH₃ | CH₂ | 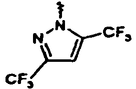 | N | Cl | 4,29 |
| I-1-231 | H | H | c-Pr | I | CH₃ | CH₂ | 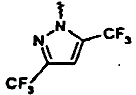 | N | Cl | 4,01 |
| I-1-232 | H | H | CH₂CF₃ | I | CH₃ | CH₂ | 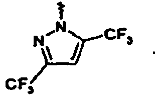 | N | Cl | 5,28 |
| I-1-233 | H | H | 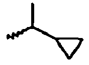 | I | CH₃ | CH₂ | 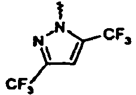 | N | Cl | 4,55 |
| I-1-234 | H | H | H | I | CH₃ | CH₂ | 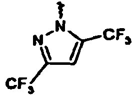 | N | Cl | 3,56 |
| I-1-235 | H | H | 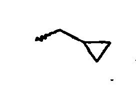 | I | CH₃ | CH₂ | 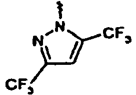 | N | Cl | 4,34 |
| I-1-236 | H | H | 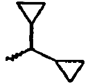 | I | CH₃ | CH₂ | 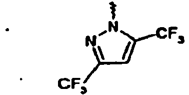 | N | Cl | 4,80 |
| I-1-237 | H | H | CH₃ | I | CH₃ | CH₂ | 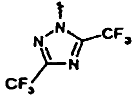 | N | Cl | 3,60 |
| I-1-238 | H | H | *i*-Pr | I | CH₃ | CH₂ | 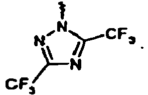 | N | Cl | 4,06 |
| I-1-239 | H | H | CH₃ | I | CH₃ | CH₂ | 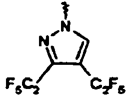 | N | Cl | 4,51 |
| I-1-240 | H | H | *i*-Pr | I | CH₃ | CH₂ | 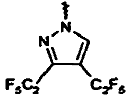 | N | Cl | 4,96 |
| I-1-241 | H | H | CH₂CN | I | CH₃ | CH₂ | 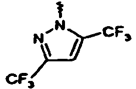 | N | Cl | 3,73 |
| I-1-242 | H | H | *i*-Pr | I | CH₃ | CH₂ | 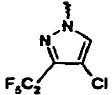 | N | Cl | 4,40 |
| I-1-243 | H | H | CH₃ | I | CH₃ | CH₂ | 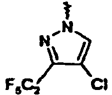 | N | Cl | 3,96 |
| I-1-244 | H | H | C₂H₅ | I | CH₃ | CH₂ | 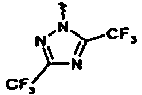 | N | Cl | 3,83 |
| I-1-245 | H | H | CH₂CN | I | CH₃ | CH₂ | 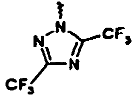 | N | Cl | 3,53 |
| I-1-246 | H | H | CH₂CN | I | CH₃ | CH₂ | 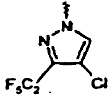 | N | Cl | 3,85 |
| I-1-247 | H | H | CH₃ | I | CH₃ | CH₂ | 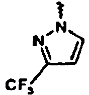 | N | Cl | 3,05 |
| I-1-248 | H | H | *i*-Pr | I | CH₃ | CH₂ | 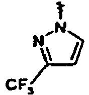 | N | Cl | 3,51 |
| I-1-249 | H | H | c-Pr | I | CH₃ | CH₂ | 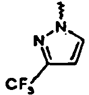 | N | Cl | 3,27 |
| I-1-250 | H | H | C₂H₅ | I | CH₃ | CH₂ | 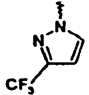 | N. | Cl | 3,28 |
| I-1-251 | H | H | CH₂CN | I | CH₃ | CH₂ | 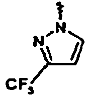 | N | Cl | 3,04 |
| I-1-252 | H | H | *c*-Pr | I | CH₃ | CH₂ | 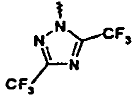 | N | Cl | 3,81 |
| I-1-253 | H | H | 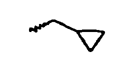 | I | CH₃ | CH₂ | 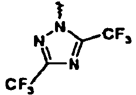 | N | Cl | 4,13 |
| I-1-254 | H | H | 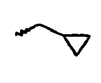 | I | CH₃ | CH₂ | 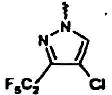 | N | Cl | 4,47 |
| I-1-255 | H | H | 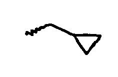 | I | CH₃ | CH₂ | 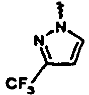 | N | Cl | 3,60 |
| I-1-256 | H | CH₃ | CH₃ | I | CH₃ | CH₂ | 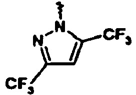 | N | Cl | 3,84 |
| I-1-257 | H | H | CH₃ | Cl | CH₃ | CH₂ | 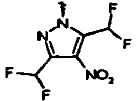 | N | Cl | 2,92 |
| I-1-258 | H | H | *i*-Pr | Cl | CH₃ | CH₂ | 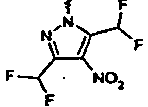 | N | Cl | 3,32 |
| I-1-259 | H | H | *i*-Pr | Cl | Cl | CH₂ | 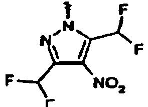 | N | Cl | 3,27 |
| I-1-260 | H | H | c-Pr | Cl | CH₃ | CH₂ | 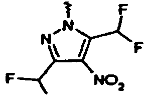 | N | Cl | 3,12 |
| I-1-261 | H | H | *c*-Pr | Cl | Cl | CH₂ | 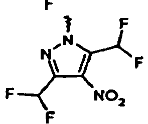 | N | Cl | 3,08 |
| I-1-262 | H | H | *i*-Pr | Cl | CH₃ | CH₂ | 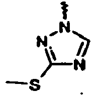 | N | Cl | 2,31 |
| I-1-263 | H | H | c-Pr | Cl | CH₃ | CH₂ | 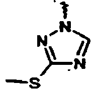 | N | Cl | 2,13 |
| I-1-264 | H | H | 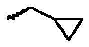 | Cl | CH₃ | CH₂ | 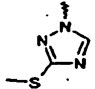 | N | Cl | 2,39 |
| I-1-265 | H | H | CH₃ | Cl | CH₃ | CH₂ | 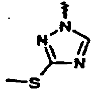 | N | Cl | 1,94 |
| I-1-266 | H | H | CH₃ | Cl | Cl | CH₂ | 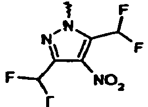 | N | Cl | 2,99 |
| I-I-267 | H | H | *i*-Pr | I | CH₃ | CH₂ | 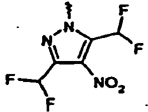 | N | Cl | 3,67 |
| I-1-268 | H | H | *c*-Pr | I | CH₃ | CH₂ | 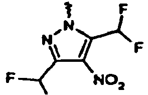 | N | Cl | 3,44 |
| I-1-269 | H | H | *i*-Pr | Cl | CH₃ | CH₂ | 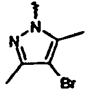 | N | Cl | 3,4 |
| I-1-270 | H | H | CH₃ | Cl | CH₃ | CH₂ | 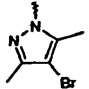 | N | Cl | 2,95 |
| I-1-271 | H | H | c-Pr | Cl | CH₃ | CH₂ | 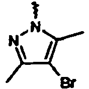 | N | Cl | 3,17 |
| I-1-272 | H | H | H | I | CH₃ | CH₂ | 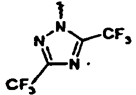 | N | Cl | 3,38 |
| I-1-273 | H | H | H | I | CH₃ | CH₂ | 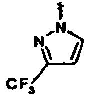 | N | Cl | 2,84 |
| I-1-274 | H | H | C₂H₅ | I | CH₃ | CH₂ | 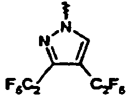 | N | Cl | 4,74 |
| I-1-275 | H | H | c-Pr | I | CH₃ | CH₂ | 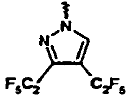 | N | Cl | 4,71 |
| I-1-276 | H | H | *c*-Pr | I | CH₃ | CH₂ | 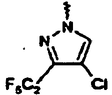 | N | Cl | 4,15 |
| I-1-277 | H | H | C₂H₅ | I | CH₃ | CH₂ | 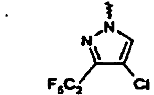 | N | Cl | 4,17 |
| I-1-278 | H | H | CH₃ | I | CH₃ | CH₂ | 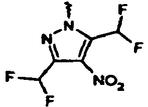 | N | Cl | 3,25 |
| I-1-279 | H | H | 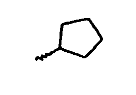 | I | CH₃ | CH₂ | 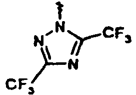 | N | Cl | 4,47 |
| I-1-280 | H | H | 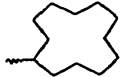 | I | CH₃ | CH₂ | 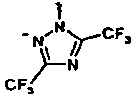 | N | Cl | 6,56 |
| I-1-281 | H | H | 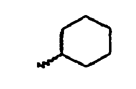 | I | CH₃ | CH₂ | 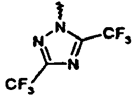 | N | Cl | 4,54 |
| I-1-282 | H | H | 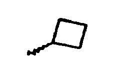 | I | CH₃ | CH₂ | 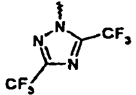 | N | Cl | 4,18 |
| I-1-283 | H | H | C₂H₅ | I | CH₃ | CH₂ | 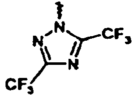 | N | Cl | 3,89 |
| I-1-284 | H | H | 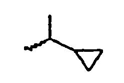 | I | CH₃ | CH₂ | 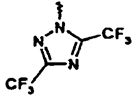 | N | Cl | 4,16 |
| I-1-285 | H | H | 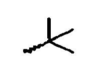 | I | CH₃ | ° CH₂ | 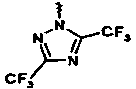 | N | Cl | 4,23 |
| I-1-286 | H | H | 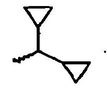 | I | CH₃ | CH₂ | 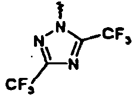 | N | Cl | 4,52 |
| I-1-287 | H | H | 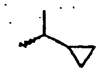 | I | CH₃ | CH₂ | 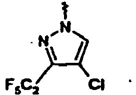 | N | Cl | 4,69 |
| I-1-288 | H | H | 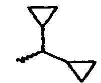 | I | CH₃ | CH₂ | 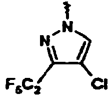 | N | Cl | 4,79 |
| I-1-289 | H | H | 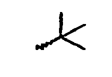 | I | CH₃ | CH₂ | 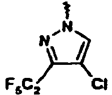 | N | Cl | 4,63 |
| I-1-290 | H | H | CH₃ | Cl | Cl | CH₂ | 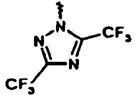 | N | Cl | 3,40 |
| I-1-291 | H | H | *i*-Pr | Cl | Cl | CH₂ | 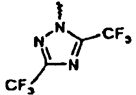 | N | Cl | 3,76 |
| I-1-292 | H | H | CH₂CN | Cl | Cl | CH₂ | 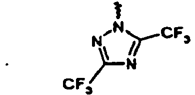 | N | Cl | 2,83 |
| I-1-293 | H | H | 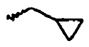 | Cl | Cl | CH₂ | 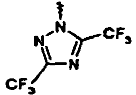 | N | Cl | 3.22 |
| I-1-294 | H | H | *i*-Pr | Cl | Cl | CH₂ | 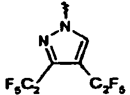 | N | Cl | 4,00 |
| I-1-295 | H | H | c-Pr | Cl | Cl | CH₂ | 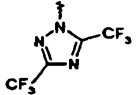 | N | Cl | 2,95 |
| I-1-296 | H | H | H | Cl | Cl | CH₂ | 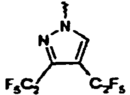 | N | Cl | 3,43 |
| I-1-297 | H | H | CH₃ | Cl | Cl | CH₂ | 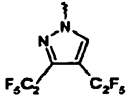 | N | Cl | 3,59 |
| I-1-298 | H | H | H | Cl | Cl | CH₂ | 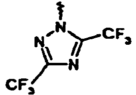 | N | Cl | 2,61 |
| I-1-299 | H | H | CH₂CN | Cl | Cl | CH₂ | 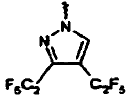 | N | Cl | 3,53 |
| I-1-300 | H | H | *c-Pr* | Cl | Cl | CH₂ | 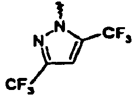 | N | Cl | 3,19 |
| I-1-301 | H | H | *i*-Pr | Cl | Cl | CH₂ | 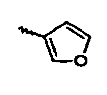 | N | Cl | 2,60 |
| I-1-302 | H | H | H | Cl | Cl | CH₂ | 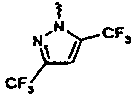 | N | Cl | 2,80 |
| I-1-303 | H | H | CH₂CN | Cl | Cl | CH₂ | 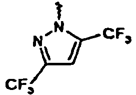 | N | Cl | 2,98 |
| I-1-304 | H | H | CH₃ | Cl | Cl | CH₂ | 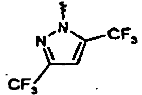 | N | Cl | 3,01 |
| I-1-305 | H | H | *i*-Pr | Cl | Cl | CH₂ | 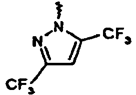 | N | Cl | 3,40 |
| I-1-306 | H | H | CH₃ | Cl | Cl | CH₂ | 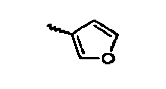 | N | Cl | 2,18 |
| I-1-307 | H | H | H | Cl | Cl | CH₂ | 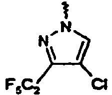 | N | Cl | 2,95 |
| I-1-308 | H | H | CH₃ | Cl | Cl | CH₂ | 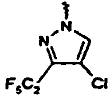 | N | Cl | 3,18 |
| I-1-309 | H | H | *i*-Pr | Cl | Cl | CH₂ | 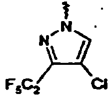 | N | Cl | 3,53 |
| I-1-310 | H | H | CH₂CN | Cl | Cl | CH₂ | 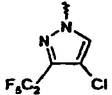 | N | Cl | 3,13 |
| I-1-311 | H | H | H | Cl | Cl | CH₂ | 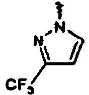 | N | Cl | 2,21 |
| I-1-312 | H | H | CH₃ | Cl | Cl | CH₂ | 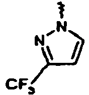 | N | Cl | 2,37 |
| I-1-313 | H | H | CH₂CN | Cl | Cl | CH₂ | 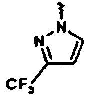 | N | Cl | 2,43 |
| I-1-314 | H | H | *i*-Pr | Cl | Cl | CH₂ | 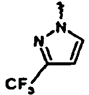 | N | Cl | 2,77 |
| I-1-315 | H | H | c-Pr | Cl | Cl | CH₂ | 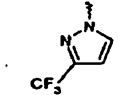 | N | Cl | 2,54 |
| I-1-316 | H | H | CH₃ | Cl | Cl | CH₂ | 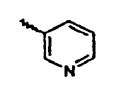 | N | Cl | 1,03 |
| I-1-317 | H | H | *i*-Pr | Cl | Cl | CH₂ | 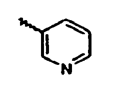 | N | Cl | 1,31 |
| I-1-318 | H | H | *i*-Pr | Cl | Cl | CH₂ | 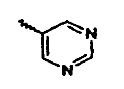 | N | Cl | 1,62 |
| I-1-319 | H | H | H | Cl | Cl | CH₂ | 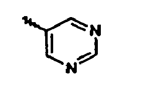 | N | Cl | 2,02 |
| I-1-320 | H | H | CH₂CN | Cl | Cl | CH₂ | 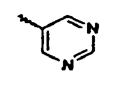 | N | Cl | 1,49 |
| I-1-321 | H | H | CH₃ | Cl | Cl | CH₂ | 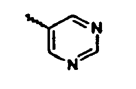 | N | Cl | 1,41 |
| I-1-322 | H | H | CH₃ | Cl | Cl | CH₂ | 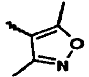 | N | Cl | 1,94 |
| I-1-323 | H | H | *i*-Pr | Cl | CH₃ | CH₂ | 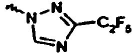 | N | Cl | 3,01 |
| I-1-324 | ⁻H | H | (*S*)-CH(CH₃) CH₂SCH₃ | Cl | Cl | CH₂ | 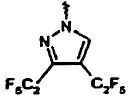 | N | Cl | * |
| I-1-325 | H | H | (*S*)-CH(CH₃) CH₂SOCH₃ | Cl | Cl | CH₂ | 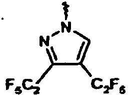 | N | Cl | 3,54 |
| I-1-326 | H | H | CH₃ | Cl | Cl | CH₂ | 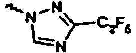 | N | Cl | 2,55 |
| I-1-327 | H | H | *i*-Pr | Cl | Cl | CH₂ | 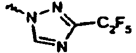 | N | Cl | 2,97 |
| I-1-328 | H | H | (*S*)-CH(CH₃) CH₂SO₂CH₃ | Cl | Cl | CH₂ | 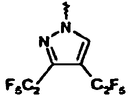 | N | Cl | 3,63 |
| I-1-329 | H | H | CH₃ | Cl | CH₃ | CH₂ | 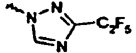 | N | Cl | 2,57 |
| I-1-330 | H | H | H | Cl | CH₃ | CH₂ | 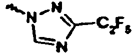 | N | Cl | 2,39 |
| I-1-331 | H | H | c-Pr | Cl | CH₃ | CH₂ | 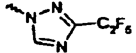 | N | Cl | 2,78 |
| I-1-332 | H | H | CH₂CN | Cl | CH₂ | CH₂ | 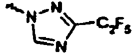 | N | Cl | 2,59 |
| I-1-333 | H | H | CH₃ | CN | CH₃ | CH₂ | 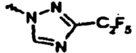 | N | Cl | 2,25 |
| I-1-334 | H | H | *i*-Pr | Cl | CH₃ | CH₂ | 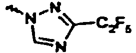 | N | Cl | 2,66 |
| I-1-335 | H | H | *c*-Pr | Cl | Cl | CH₂ | 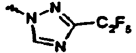 | N | Cl | 2,69 |
| I-1-336 | H | H | H | Cl | Cl | CH₂ | 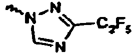 | N | Cl | 2,32 |
| I-1-337 | H | H | CH₂CN | Cl | Cl | CH₂ | 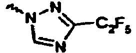 | N | Cl | 2,56 |
| I-1-338 | H | H | c-Pr | CN | CH₃ | CH₂ | 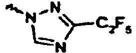 | N | Cl | 2,45 |
| I-1-339 | H | H | H | CN | CH₃ | CH₂ | 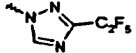 | N | Cl | 2,11 |
| I-1-340 | H | H | *i*-Pr | Cl | Cl | CH₂ | 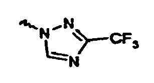 | N | Cl | 2,55 |
| I-1-341 | H | H | *i*-Pr | Cl | CH₃ | CH₂ | 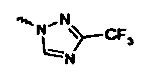 | N | Cl | 2,63 |
| I-1-342 | H | H | CH₃ | Cl | Cl | CH₂ | 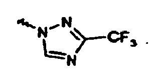 | N | Cl | 2,15 |
| I-1-343 | H | H | H | Cl | CH₃ | CH₂ | 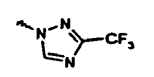 | N | Cl | 2,02 |
| I-1-344 | H | H | *c*-Pr | Cl | Cl | CH₂ | 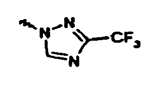 | N | Cl | 2,37 |
| I-1-345 | H | H | c-Pr | Cl | CH₃ | CH₂ | 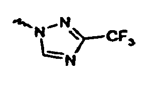 | N | Cl | 2,42 |
| I-1-346 | H | H | H | Cl | Cl | CH₂ | 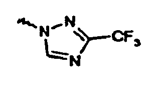 | N | Cl | 2,01 |
| I-1-347 | H | H | CH₃ | Cl | CH₃ | CH₂ | 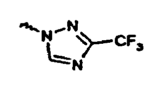 | N | Cl | 2,19 |
| I-1-348 | H | H | CH₂CN | Cl | CH₃ | CH₂ | 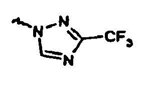 | N | Cl | 2,21 |
| I-1-349 | H | H | CH₂CN | Cl | Cl | CH₂ | 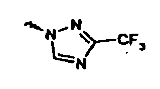 | N | Cl | 2,19 |
| I-1-350 | H | H | H | CN | CH₃ | CH₂ | 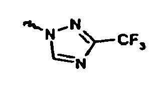 | N | Cl | 1,75 |
| I-1-351 | H | H | CH₂CN | CN | CH₃ | CH₂ | 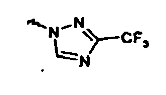 | N | Cl | 1,96 |
| I-1-352 | H | H | CH₃ | CN | CH₃ | CH₂ | 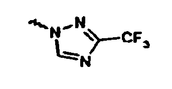 | N | Cl | 1,91 |
| I-1-353 | H | H | c-Pr | CN | CH₃ | CH₂ | 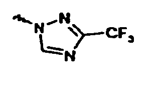 | N | Cl | 2,08 |
| I-1-354 | H | H | *i*-Pr | CN | CH₃ | CH₂ | 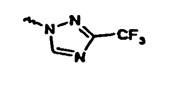 | N | Cl | 2,23 |
| I-1-355 | H | H | CH₃ | Cl | CH₃ | C=NOCH₃ | 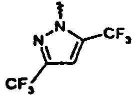 | N | Cl | 4,07 |

Analog zu den oben aufgeführten Beispielen sowie der allgemeinen Beschreibung werden folgende Verbindungen der Formel (I-2) erhalten.

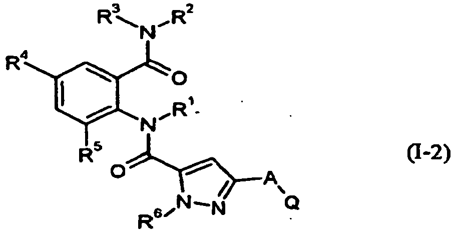

**Tabelle 2**

| **Nr.** | **R¹** | **R²** | **R³** | **R⁴** | **R⁵** | **R⁶** | **A** | **Q** | **logP** |
|---|---|---|---|---|---|---|---|---|---|
| I-2-1 | H | H | *i*-Pr | Cl | CH₃ | CH₃ | CH₂ | 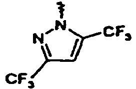 | 4,08 |
| I-2-2 | H | H | *i*-Pr | Cl | Cl | *i*-Pr | CH₂ | 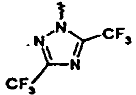 | 3,85 |
| I-2-3 | H | H | CH₃ | Cl | Cl | *i*-Pr | CH₂ | 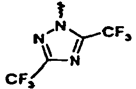 | 3,31 |
| I-2-4 | H | H | CH₃ | Cl | Cl | 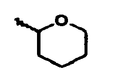 | CH₂ | 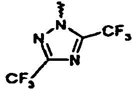 | * |
| I-2-5 | H | H | CH₃ | Cl | Cl | 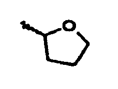 | CH₂ | 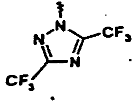 | 2,83 |
| I-2-6 | H | H | CH₃ | Cl | Cl | 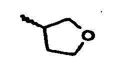 | CH₂ | 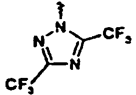 | 2,99 |
| I-2-7 | H | H | *i*-Pr | Cl | Cl | 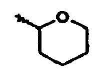 | CH₂ | 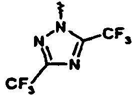 | * |
| I-2-8 | H | H | *i*-Pr | Cl | Cl | 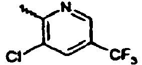 | CH₂ | 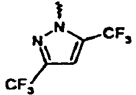 | 4,68 |
| I-2-9 | H | H | *i*-Pr | I | CH₃ | 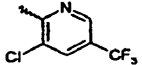 | CH₂ | 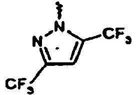 | 4,98 |
| I-2-10 | H | H | *c*-Pr | I | CH₃ | 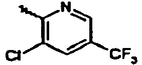 | CH₂ | 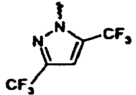 | 4,73 |
| I-2-11 | H | H | *i*-Pr | Cl | CH₃ | 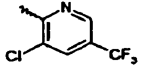 | CH₂ | 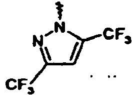 | 4,78 |
| I-2-12 | H | H | *c*-Pr | Cl | CH₃ | 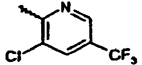 | CH₂ | 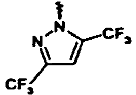 | 4,52 |
| I-2-13 | H | H | c-Pr | Cl | Cl | 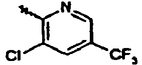 | CH₂ | 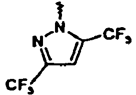 | 4,46 |
| I-2-14 | H | H | 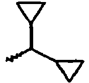 | Cl | CH₃ | 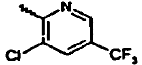 | CH₂ | 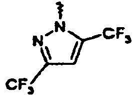 | 5,27 |
| I-2-15 | H | H | 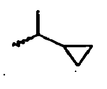 | Cl | CH₃ | 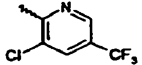 | CH₂ | 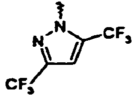 | 4,04 |
| I-2-16 | H | H | *t*-Bu | Cl | CH₃ | 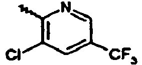 | CH₂ | 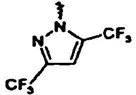 | 5,12 |
| I-2-17 | H | H | H | Cl | CH₃ | 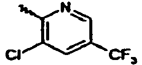 | CH₂ | 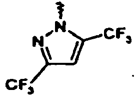 | 4,09 |
| I-2-18 | H | H | CH₂CN | Cl | CH₃ | 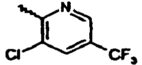 | CH₂ | 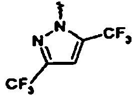 | 4,23 |
| I-2-19 | H | H | c-Pr | F | CH₃ | 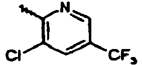 | CH₂ | 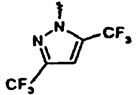 | 4,11 |
| I-2-20 | H | H | *i*-Pr | F | CH₃ | 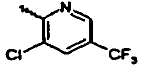 | CH₂ | 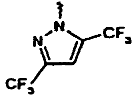 | 4,47 |
| I-2-21 | H | H | *i*-Pr | Br | CH₃ | 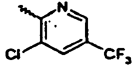 | CH₂ | 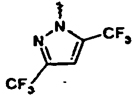 | 4,75 |
| I-2-22 | H | H | CH₃ | Cl | CH₃ | 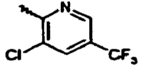 | CH₂ | 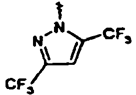 | 3,53 |
| I-2-23 | H | H | CH₃ | Cl | Cl | 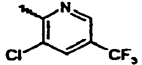 | CH₂ | 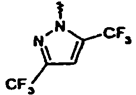 | 3,54 |
| I-2-24 | H | H | c-Pr | Br | CH₃ | 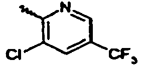 | CH₂ | 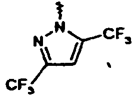 | 4,63 |

Analog zu den oben aufgeführten Beispielen sowie der allgemeinen Beschreibung werden folgende Verbindungen der Formel (I-3) erhalten.

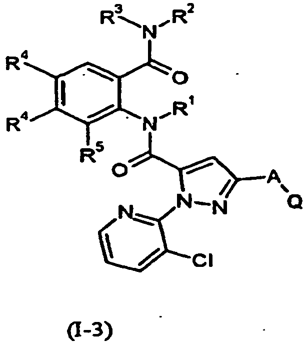

**Tabelle 3**

| **Nr.** | **R¹** | **R²** | **R³** | **R⁴-R⁴** | **R⁵** | **A** | **Q** | **logP** |
|---|---|---|---|---|---|---|---|---|
| I-3-1 | H | H | *i*-Pr | -CH=CH-CH=CH- | Cl | CH₂ | 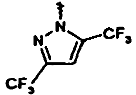 | 4.08 |

¹H-NMR-Daten ausgewählter Verbindungen:
1-1-21 (400 MHz, DMSO): 1.11 (d, 6 H), 2.14 (s, 3 H), 3.91 (m, 1 H), 5.55 (s, 2 H), 7.12 (s, 1 H), 7.28 (s, 1 H), 7.39 (s, 1 H), 7.55 (dd, 1 H), 7.76 (d, 1 H), 7.99 (s, 1 H), 8.08 (d, 1 H), 8.43 (d, 1 H), 8.61 (s, 1 H), 10.12 (s, 1 H).
I-1-22 (400 MHz, DMSO): 1.18 (d, 6 H), 2.14 (s, 3 H), 3.90 (m, 1 H), 5.87 (s, 2 H), 7.24 (s, 1 H), 7.42 (s, 1 H), 7.53 (s, 1 H), 7.55 (dd, 1 H), 7.80 (d, 1 H), 8.08 (d, 1 H), 8.44 (d, 1 H), 10.07 (s, 1 H).
I-1-26 (400 MHz, DMSO): 2.14 (s, 3 H), 2.67 (d, 3 H), 5.93 (s, 2 H), 7.21 (s, 1 H), 7.33 (s, 1 H), 7.40 (s, 1 H), 7.55 (dd, 1 H), 8.00 (d, 1 H), 8.08 (d, 1 H), 8.45 (d, 1 H), 10.12 (s, 1 H).
I-1-48 (400 MHz, DMSO): 2.13 (s, 3 H), 5.69 (s, 2 H), 7.18 (s, 1 H), 7.41 (s, 2 H), 7.55 (dd, 1 H), 8.10 (d, 1 H), 8.45 (d, 1 H), 8.80 (s, 1 H), 10.19 (s, 1 H).
I-1-65 (400 MHz, DMSO): 1.06 (d, 6 H), 2.17 (s, 3 H), 3.95 (m, 1 H), 5.58 (s, 2 H), 7.15 (s, 1 H), 7.31 (s, 1 H), 7.42 (s, 1 H), 7.58 (dd, 1 H), 7.78 (d, 1 H), 7.92 (s, 1 H), 8.09 (d, 1 H), 8.46 (d, 1 H), 8.47 (s, 1 H), 10.04 (s, 1 H).
I-1-75 (400 MHz, DMSO): 1.09 (d, 6 H), 2.00 (s, 3 H), 2.15 (s, 3 H), 2.44 (dd, 1 H), 2.53 (dd, 1 H), 3.99 (m, 1 H), 5.86 (s, 2 H), 7.23 (s, 1 H), 7.33 (s, 1 H), 7.42 (s, 1 H), 7.55 (dd, 1 H), 7.92 (d, 1 H), 8.09 (d, 1 H), 8.44 (d, 1 H), 10.04 (s, 1 H).
1-1-88 (400 MHz, DMSO): 2.14 (s, 3 H), 2.67 (d, 3 H), 5.38 (s, 2 H), 7.28 (s, 1 H), 7.33 (s, 1 H), 7.40 (s, 1 H), 7.55 (dd, 1 H), 7.96 (d, 2 H), 8.00 (d, 1 H), 8.08 (d, 1 H), 8.17 (d, 2 H), 8.46 (d, 1 H), 10.10 (s, 1 H).
I-1-104 (400 MHz, DMSO): 2.13 (s, 3 H), 2.64 (d, 3 H), 6.18 (s, 2 H), 7.28 (s, 1 H), 7.32 (s, 1 H), 7.40 (s, 1 H), 7.55 (dd, 1 H), 7.92 (d, 2 H), 8.00 (d, 1 H), 8.09 (d, 1 H), 8.30 (d, 2 H), 8.45 (d, 1 H), 10.10 (s, 1 H).
I-1-117 (400 MHz, DMSO): 1.02 (d, 6 H), 2.15 (s, 3 H), 3.34 (s, 3 H), 3:92 (m, 1 H), 5.10 (s, 2 H), 7.17 (s, 1 H), 7.29 (s, 1 H), 7.39 (s, 1 H), 7.55 (dd, 1 H), 7.78 (d, 1 H), 8.09 (d, 1 H), 8.44 (d, 1 H), 10.01 (s, 1 H).
I-1-126 (400 MHz, DMSO): 0.99-1.10 (m, 4 H), 1.03 (d, 6 H), 2.15 (s, 3 H), 3.00-3.08 (m, 1 H), 3.92 (m, 1 H), 5.05 (s, 2 H), 7.16 (s, 1 H), 7.29 (s, 1 H), 7.40 (s, 1 H), 7.55 (dd, 1 H), 7.78 (d, 1 H), 8.09 (d, 1 H), 8.44 (d, 1 H), 10.01 (s, 1 H).
I-1-139 (400 MHz, DMSO): 1.38 (s, 6 H), 2.14 (s, 3 H), 2.89 (s, 3 H), 3.68 (s, 2 H), 5.87 (s, 2 H), 7.21 (s, 1 H), 7.31 (s, 1 H), 7.40 (s, 1 H), 7.55 (dd, 1 H), 7.79 (s, 1 H), 8.09 (d, 1 H 8.44 (d, 1 H), 9.93 (s, 1 H).
I-1-148 (400 MHz, DMSO): 2.14 (s, 3 H), 2.66 (d, 3 H), 6.18 (s, 2 H), 7.29 (s, 1 H), 7.32 (s, 1 H), 7.40 (s, 1 H), 7.55 (dd, 1 H), 7.70 (d, 1 H), 8.09 (d, 1 H), 8.11 (d, 1 H), 8.45 (s, 1 H), 8.46 (d, 1 H), 9.07 (s, 1 H), 10.10 (s, 1 H).
I-1-161 (400 MHz, DMSO): 1.04 (d, 6 H), 1.31 (s, 9 H), 2.14 (s, 3 H), 3.90 (m, 1 H), 5.46 (s, 2 H), 7.11 (s, 1 H), 7.29 (s, 1 H), 7.39 (s, 1 H), 7.55 (dd, 1 H), 7.78 (d, 1 H), 8.09 (d, 1 H), 8.40 (s, 1 H), 8.44 (d, 1 H), 10.10 (s, 1 H).
I-1-172 (400 MHz, DMSO): 1.04 (d, 6 H), 2.15 (s, 3 H), 3.90 (m, 1 H), 5.58 (s, 2 H), 7.16 (s, 1 H), 7.55 (dd, 1 H), 7.70 (s, 1 H), 7.78 (s, 1 H), 7.93 (d, 1 H), 8.09 (d, 1 H), 8.38 (s, 1 H), 8.45 (d, 1 H), 10.32 (s, 1 H).
I-1-175 (400 MHz, DMSO): 2.21 (s, 3 H), 4.16 (d, 1 H), 5.57 (s, 2 H), 7.16 (s, 1 H), 7.55 (dd, 1 H), 7.70 (s, 1 H), 7.78 (s, 1 H), 7.86 (d, 1 H), 8.09 (d, 1 H), 8.37 (s, 1 H), 8.46 (d, 1 H), 10.32 (s, 1 H).
I-1-176 (400 MHz, DMSO): 2.19 (s, 3 H), 2.68 (d, 1 H), 5.88 (s, 2 H), 7.25 (s, 1 H), 7.55 (dd, 1 H), 7.80 (s, 1 H), 7.97 (s, 1 H), 8.09 (d, 1 H), 8.15 (d, 1H), 8.44 (d, 1 H), 10.40 (s, 1 H).
I-1-180 (400 MHz, DMSO): 2.21 (s, 3 H), 4.16 (d, 1 H), 5.87 (s, 2 H), 7.27 (s, 1 H), 7.55 (dd, 1 H), 7.77 (s, 1 H), 7.86 (s, 1 H), 8.09 (d, 1 H), 8.46 (d, 1 H), 8.96 (d, 1 H), 10.34 (s, 1 H).
I-1-182 (400 MHz, DMSO): 1.04 (d, 6 H), 2.19 (s, 3 H), 3.92 (m, 1 H), 5.57 (s, 2 H), 6.74 (s, 1 H), 7.15 (s, 1 H), 7.55 (dd, 1 H), 7.70 (s, 1 H), 7.78 (s, 1 H), 7.91 (d, 1 H), 8.06 (s, 1 H), 8.09 (d, 1 H), 8.45 (d, 1 H), 10.30 (s, 1 H).
I-1-190 (400 MHz, DMSO): 2.21 (s, 3 H), 4.15 (d, 1 H), 5.69 (s, 2 H), 7.18 (s, 1 H), 7.53 (s, 1 H), 7.55 (dd, 1 H), 7.76 (s, 1 H), 7.85 (s, 1 H), 8.07 (d,1 1 H), 8.45 (d, 1 H), 8.95 (s, 1 H), 10.30 (s, 1 H).
I-1-195 (400 MHz, DMSO): 1.11 (d, 6 H), 2.00 (s, 3 H), 2.15 (s, 3 H), 2.44 (dd, 1 H), 2.53 (dd, 1 H), 4.02 (m, 1 H), 5.57 (s, 2 H), 7.08 (d, 1 H), 7.15 (s, 1 H), 7.20 (d, 1 H), 7.55 (dd, 1 H), 7.84 (d, 1 H), 8.09 (d, 1 H), 8.37 (s, 1 H), 8.45 (d, 1 H), 10.30 (s, 1 H).
I-1-196 (400 MHz, DMSO): 2.15 (s, 3 H), 4.34 (d, 1 H), 5.57 (s, 2 H), 6.19 (d, 1 H), 6.29 (d, 1 H), 7.10 (s, 1 H), 7.13 (d, 1 H), 7.22 (d, 1 H), 7.43 (s, 1 H), 7.55 (dd, 1 H), 8.08 (d, 1 H), 8.38 (s, 1 H), 8.46 (d, 1 H), 8.55 (t, 1 H), 10.30 (s, 1 H).
I-1-201 (400 MHz, DMSO): 2.14 (s, 3 H), 4.33 (d, 1 H), 5.57 (s, 2 H), 6.19 (d, 1 H), 6.29 (d, 1 H), 7.10 (s, 1 H), 7.33 (s, 1 H), 7.44 (2 d, 2 H), 7.55 (dd, 1 H), 8.08 (d, 1 H), 8.37 (s, 1 H), 8.45 (d, 1 H), 8.55 (t, 1 H), 10.34 (s, 1 H).
I-1-208 (400 MHz, DMSO): 2.14 (s, 3 H), 4.33 (d, 1 H), 5.57 (s, 2 H), 6.19 (d, 1 H), 6.29 (d, 1 H), 7.13 (s, 1 H), 7.45 (s, 1 H), 7.55 (dd, 1 H), 7.75 (s, 1 H), 7.80 (s, 1 H), 8.08 (d, 1 H), 8.38 (s, 1 H), 8.69 (br s, 1 H), 8.55 (t, 1 H), 10.34 (s, 1 H).
I-1-241 (400 MHz, DMSO): 1.02 (d, 3 H), 2.11 (s, 3 H), 3.91 (m, 1 H), 5.57 (s, 2 H), 7.12 (s, 1 H), 7.55 (dd, 1 H), 7.57 (s, 1 H), 7.69 (s, 1 H), 7.76 (d, 1 H), 8.08 (d, 1 H), 8.37 (s, 1 H), 8.45 (d, 1 H), 10.02 (s, 1 H).
I-1-243 (400 MHz, DMSO): 2.10 (s, 3 H), 2.67 (d, 3 H), 3.07 (q, 2 H), 5.57 (s, 2 H), 7.13 (s, 1 H), 7.55 (dd, 1 H), 7.60 (s, 1 H), 7.70 (s, 1 H), 7.98 (d, 1 H), 8.10 (d, 1 H), 8.38 (s, 1 H), 8.45 (d, 1 H), 10.09 (s, 1 H).
I-1-244 (400 MHz, DMSO): 0.99 (t, 3 H), 2.11 (s, 3 H), 3.07 (q, 2 H), 5.87 (s, 2 H), 7.21 (s, 1 H), 7.55 (dd, 1 H), 7.59 (s, 1 H), 7.70 (s, 1 H), 7.98 (s, 1 H), 8.09 (d, 1 H), 8.45 (d, 1 H), 10.05 (s, 1 H).
I-1-248 (400 MHz, DMSO): 1.01 (d, 6 H), 2.10 (s, 3 H), 3.89 (m, 1 H), 5.56 (s, 2 H), 6.73 (s, 1 H), 7.11 (s, 1 H), 7.55 (dd, 1 H), 7.56 (s, 1 H), 7.76 (s, 1 H), 7.75 (d, 1 H), 8.04 (s, 1 H), 8.09 (d, 1 H), 8.45 (d, 1 H), 10.01 (s, 1 H).
I-1-274 (400 MHz, DMSO): 0.99 (t, 3 H), 2.10 (s, 3 H), 3.07 (q, 2 H), 5.68 (s, 2 H), 7.15 (s, 1 H), 7.55 (dd, 1 H), 7.60 (s, 1 H), 7.97 (s, 1 H), 8.08 (d, 1 H), 8.45 (d, 1 H), 10.09 (s, 1 H).
I-1-275 (400 MHz, DMSO): 0.40-0.47 (m, 2 H), 0.55-0.60 (m, 2 H), 2.10 (s, 3 H), 2.65-2.71 (m, 1 H), 5.69 (s, 2 H), 7.17 (s, 1 H), 7.55 (dd, 1 H), 7.56 (s, 1 H), 7.69 (s, 1 H), 8.00 (d, 1 H), 8.08 (d, 1 H), 8.45 (s, 1 H), 8.79 (d, 1 H), 10.05 (s, 1 H).
I-1-276 (400 MHz, DMSO): 0.41-0.45 (m, 2 H), 0.56-0.61 (m, 2 H), 2.10 (s, 3 H), 2.65-2.71 (m, 1 H), 5.57 (s, 2 H), 7.14 (s, 1 H), 7.55 (dd, 1 H), 7.55 (s, 1 H), 7.69 (s, 1 H), 8.01 (d, 1 H), 8.08 (d, 1 H), 8.38 (s, 1 H), 8.46 (d, 1 H), 10.02 (s, 1 H).
I-1-279 (400 MHz, DMSO): 1.34-1.60 (m, 6 H), 1.71-1.78 (m, 2 H), 2.10 (s, 3 H), 4.01-4.08 (m, 1 H), 5.87 (s, 2 H), 7.21 (s, 1 H), 7.55 (dd, 1 H), 7.57 (s, 1 H), 7.70 (s, 1 H), 7.85 (d, 1 H), 8.07 (d, 1 H), 8.43 (s, 1 H), 10.02 (s, 1 H).
I-1-280 (400 MHz, DMSO): 1.13-1.51 (m, 20 H), 1.71-1.78 (m, 2 H), 2.07 (s, 3 H), 3.93-3.96 (m, 1 H), 5.83 (s, 2 H), 7.13 (s, 1 H), 7.55 (dd, 1 H), 7.61 (s, 1 H), 7.68 (d, 1 H), 8.02 (s, 1 H), 8.07 (d, 1 H), 8.43 (s, 1 H), 10.02 (s, 1 H).
I-1-282 (400 MHz, DMSO): 1.36 (s, 2 H), 1.56-1.65 (m, 2 H), 1.84-1.94 (m, 2 H), 2.07 (s, 3 H), 4.18-4.24 (m, 1 H), 5.87 (s, 2 H), 7.18 (s, 1 H), 7.55 (dd, 1 H), 7.59 (s, 1 H), 7.70 (d, 1 H), 8.07 (s, 1 H), 8.19 (d, 1 H), 8.43 (s, 1 H), 10.02 (s, 1 H).
I-1-290 (400 MHz, CDCl₃): 2.90 (d, 3 H), 5.74 (s, 2 H), 6.10 (d, 1 H), 7.23 (s, 1 H), 7.23 (s, 1 H), 7.28 (s, 1 H), 7.40 (dd, 1 H), 7.87 (d, 1 H), 8.46 (d, 1 H), 9.74 (s, 1 H).
1-1-296 (400 MHz, CDCl₃): 5.54 (s, 2 H), 5.62 (s, 1 H), 6.11 (s, 1 H), 7.12 (s, 1 H), 7.39 (s, 1 H), 7.40 (dd, 1 H), 7.48 (s, 1 H), 7.90 (d, 1 H), 7.94 (s, 1 H), 8.48 (d, 1 H), 9.45 (s, 1 H).
I-1-310 (400 MHz, CDCl₃): 4.18 (d, 2 H), 5.48 (s, 2 H), 6.74 (t, 1 H), 7.08 (s, 1 H), 7.32 (s, 1 H), 7.43 (dd, 1 H), 7.48 (s, 1 H), 7.92 (d, 1 H), 7.94 (s, 1 H), 8.50 (d, 1 H), 8.93 (s, 1 H).
I-1-314 (400 MHz, CDCl₃): 1.10 (d, 6 H), 4.08 (m, 1 H), 5.50 (s, 2 H), 5.92 (d, 1 H), 6.57 (s, 1 H), 7.15 (s, 1 H), 7.28 (s, 1 H), 7.39 (s, 1 H), 7.40 (dd, 1 H), 7.57 (s, 1 H), 7.87 (d, 1 H), 8.48 (d, 1 H), 9.65 (s, 1 H).
I-1-324 (400 MHz, CDCl₃): 1.24 (d, 3 H), 2.10 (s, 3 H), 2.60 (m, 2 H), 4.22 (m, 1 H), 5.51 (d, 1 H), 5.58 (d, 1 H), 6.13 (d, 1 H), 7.17 (s, 1 H), 7.35 (s, 1 H), 7.39 (s, 1 H), 7.40 (dd, 1 H), 7.87 (d, 1 H), 7.90 (s, 1 H), 8.48 (d, 1 H), 9.60 (s, 1 H).
1-1-331 (400 MHz, CDCl₃): 0.55 (m, 2 H), 0.85 (m, 2 H), 2.16 (s, 3 H), 2.77 (m, 1 H), 5.57 (s, 2 H), 6.18 (d, 1 H), 7.12 (s, 1 H), 7.18 (s, 1 H), 7.24 (s, 1 H), 7.41 (dd, 1 H), 7.88 (d, 1 H), 8.35 (s, 1 H), 8.48 (d, 1 H), 10.15 (s, 1 H).
I-1-334 (400 MHz, CDCl₃): 1.24 (d, 6 H), 2.23 (s, 3 H), 4.18 (m, 1 H), 5.58 (s, 2 H), 5.99 (d, 1 H), 7.12 (s, 1 H), 7.28 (s, 1 H), 7.40 (dd, 1 H), 7.58 (d, 1 H), 7.88 (d, 1 H), 8.36 (s, 1 H), 8.48 (d, 1 H), 10.62 (s, 1 H).
I-1-340 (400 MHz, CDCl₃): 1.24 (d, 6 H), 4.08 (m, 1 H), 5.58 (s, 2 H), 5.92 (d, 1 H), 7.21 (s, 1 H), 7.30 (s, 1 H), 7.32 (s, 1 H), 7.40 (dd, 1 H), 7.77 (d, 1 H), 8.36 (s, 1 H), 8.48 (d, 1 H), 10.06 (s, 1 H).
I-1-345 (400 MHz, CDCl₃): 0.55 (m, 2 H), 0.85 (m, 2 H), 2.77 (m, 1 H), 5.58 (s, 2 H), 6.20 (d, 1 H), 7.14 (s, 1 H), 7.20 (s, 1 H), 7.22 (s, 1 H), 7.40 (dd, 1 H), 7.88 (d, 1 H), 8.32 (s, 1 H), 8.45 (d, 1 H), 10.12 (s, 1 H).
I-1-354 (400 MHz, CDCl₃): 1.21 (d, 6 H), 4.15 (m, 1 H), 5.58 (s, 2 H), 7.16 (s, 1 H), 7.18 (s, 1 H), 7.40 (dd, 1 H), 7.58 (s, 1 H), 7.75 (s, 1 H), 7.84 (d, 1 H), 8.38 (s, 1 H), 8.48 (d, 1 H), 10.95 (s, 1 H).
I-2-4 (400 MHz, CDCl₃): 1.74 (m, 3 H), 2.16 (m, 2 H), 2.40 (m, 1 H), 2.86 (d, 3 H), 3.70 (m, 1 H), 3.88 (m, 1 H), 5.75 (s, 2 H), 5.80 (m, 1 H), 6.45 (s, 1 H), 6.80 (s, 1 H), 7.16 (s, 1 H), 7.42 (s, 1 H), 7.56 (s, 1 H), 9.08 (s, 1 H).
I-2-7 (400 MHz, CDCl₃): 1.24 (m, 6 H), 1.74 (m, 3 H), 2.20 (m, 2 H), 2.40 (m, 1 H), 3.70 (m, 1 H), 3.88 (m, 1 H), 4.10 (m, 1 H), 5.80 (m, 3 H), 6.25 (s, 1 H), 6.80 (s, 1 H), 7.40 (s, 1 H), 7.52 (s, 1 H), 8.85 (s, 1 H).

Die Bestimmung der voranstehend angegebenen ¹H-NMR-Daten erfolgt mit einem Bruker Avance 400, ausgestattet mit einem BEST System (60 ul Volumenzelle), oder einem Bruker Avance 400, mit Tetramethylsilan als Referenz (0.0 ppm) und den Lösungsmitteln CDCl₃ bei 298 Kelvin oder d6-DMSO bei 304 Kelvin. Die Charakterisierung der Signalaufspaltung erfolgt mit s = Singulett, d = Dublett, t = Triplett, q = Quartett, m = Multiplett, dd = doppeltes Dublett.

Die Bestimmung der in den voranstehenden Tabellen und Herstellungsbeispielen angegebenen logP-Werte erfolgt gemäß EEC-Directive 79/831 Annex V.A8 durch HPLC (High Performance Liquid Chromatography) an einer Phasenumkehrsäule (C 18). Temperatur: 43°C.

Die Bestimmung mit der LC-MS im sauren Bereich erfolgt bei pH 2,7 mit 0,1 % wässriger Ameisensäure und Acetonitril (enthält 0,1% Ameisensäure) als Eluenten; linearer Gradient von 10% Acetonitril bis 95% Acetonitril

Die Eichung erfolgt mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinander folgenden Alkanonen).

Die lambda-max-Werte werden an Hand der UV-Spektren von 200 nm bis 400 nm in den Maxima der chromatographischen Signale ermittelt.

### Herstellung von Ausgangsstoffen der Formel (V)

### Beispiel 2

### 2-[5-(3,5-Bis-trifluoromethyl-pyrazol-1-ylmethyl)-2-(3-chlor-pyridin-2-yl)-2H-pyrazol-3-yl]-6-chlor-8-methyl-benzo[d][1,3]oxazin-4-on:

Unter Argon werden 0.12 ml (1.60 mmol) Methansulfonsäurechlorid in 3 ml Acetonitril auf 0°C abgekühlt und anschließend eine Lösung aus 540 mg (1.228 mmol) 5-(3,5-Bis-trifluormethyl-pyrazol-1-ylmethyl)-2-(3-chlor-pyridin-2-yl)-2*H-*pyrazol-3-carbonsäure in 0.17 ml (2.09 mmol) Pyridin und 6 ml Acetonitril zugetropft. Man läßt 15 min bei dieser Temperatur nachrühren und tropft anschießend eine Lösung aus 228 mg (1.228 mmol) 2-Amino-5-chlor-3,*N-*dimethyl-benzamid in 0.35 ml (4.30 mmol) Pyridin und 6 ml Acetonitril hinzu. Nach weiteren 15 min bei 0°C wird mit 0.12 ml (1.60 mmol) Methansulfonsäurechlorid versetzt und über Nacht langsam auf Raumtemperatur erwärmt. Man entfernt das Lösungsmittel i. Vak., versetzt mit 15 ml Wasser und saugt die entstandenen Kristalle ab.
Ausbeute: 500 mg (logP: 5.11)

### Herstellung von Ausgangsstoffen der Formel (IV)

### Beispiel 3

### 5-(3,5-Bis-trifluormethyl-pyrazol-1-ylmethyl)-2-(3-chlor-pyridin-2-yl)-2H-pyrazol-3-carbonsäure:

Zu einer Lösung aus 610 mg (1.34 mmol) 5-(3,5-Bis-trifluormethyl-pyrazol-1-ylmethyl)-2-(3-chlor-pyridin-2-yl)-2*H*-pyrazol-3-carbonsäuremethylester in 9 ml Ethanol wird eine Lösung aus 699 mg (1.78 mmol) Natriumhydroxid in 7 ml Wasser zugetropft. Man läßt. 2 Stunden bei Raumtemperatur rühren, engt am Rotationsverdampfer auf ca. 5 ml ein. Es wird mit 5 ml tert-Butylmethylether versetzt und die organische Phase anschließend mit Wasser gewaschen. Die vereinigten wäßrigen Phasen werden unter Eiskühlung mit konzentrierter Salzsäure auf einen pH-Wert von etwa 3 eingestellt und dreimal mit je 50 ml Ethylacetat extrahiert. Die vereinigten organischen Phasen werden über Magnesiumsulfat getrocknet und anschließend wird das Lösungsmittel im Rotationsverdampfer entfernt
Ausbeute: 560 mg (logP: 2.86)

### Herstellung von Ausgangsstoffen der Formel (IV)

### Beispiel 4

### 5-(3,5-Bis-trifluormethylpyrazol-1-ylmethyl)₋2-(3-chlorpyridin-2-yl)-2H-pyrazol-3-carbonsäuremethylester:

Eine Lösung aus 700 mg (2.03 mmol) 2-(3-Chlorpyridin-2-yl)-5-methansulfonyloxymethyl-2*H-*pyrazol-3-carbonsäuremethylester in 15 ml Acetonitril wird nacheinander mit 336 mg (2.43 mmol) Kaliumcarbonat und 413 mg (2.03 mmol) 3,5-Bis(trifluormethyl)pyrazol versetzt, und es wird anschließend 1 h bei 60°C gerührt. Nach dem Abkühlen auf Raumtemperatur engt man das Lösungsmittel am Rotationsverdampfer ein, versetzt mit Wasser und extrahiert dreimal mit je 50 ml Ethylacetat. Die vereinigten organischen Phasen werden über Magnesiumsulfat getrocknet, und anschließend wird das Lösungsmittel im Rotationsverdampfer entfernt.
Ausbeute: 970 mg (logP: 3.71)

### Herstellung von Ausgangsstoffen der Formel (VII)

### Beispiel 5

### 2-(3-Chlorpyridin-2-yl)-5-methansulfonyloxymethyl-2H-pyrazol-3-carbonsäure-methylester:

Unter Argon werden 2.10 g (7.85 mmol) 2-(3-Chlorpyridin-2-yl)-5-hydroxymethyl-2*H-*pyrazole-3-carbonsäuremethylester in 13 ml Dichlormethan vorgelegt, auf 0°C abgekühlt und nacheinander tropfenweise mit 1.64 ml (11.8 mmol) Triethylamin und 0.67 ml (8.63 mmol) Methansulfonsäurechlorid versetzt Man läßt 30 min bei dieser Temperatur nachrühren, verdünnt mit 50 ml Dichlormethan und wäscht sukzessive mit jeweils 50 ml ges. wäßriger Natriumhydrogensulfat-Lösung, 10-prozentiger wäßriger Salzsäure und ges. wäßriger Natriumchlorid-Lösung. Die organische Phase wird über Magnesiumsulfat getrocknet, und anschließend wird das Lösungsmittel im Rotationsverdampfer entfernt.
Ausbeute: 2.65 g (logP: 1.55)

### Herstellung von Ausgangsstoffen der Formel (X)

### Beispiel 6

### 2-(3-Chlorpyridin-2-yl-5-hydroxymethyl-2H-pyrazole-3-carbonsäuremethylester:

Unter Argon werden 12.3 g (41.7 mmol) 1-(3-Chlorpyridin-2-yl)-1*H*-pyrazol-3,5-dicarbonsäuredimethylester in 430 ml Tetrahydrofuran vorgelegt, auf -72°C abgekühlt und tropfenweise mit 100 ml (100 mmol einer 1 M Lösung in Hexan) Diisobutylaluminiumhydrid versetzt Man läßt über Nacht auf 0°C erwärmen und gibt vorsichtig 65 ml Wasser hinzu. Die Lösungsmittel werden im Rotationsverdampfer entfernt und der Rückstand am Soxlet mit Methanol erschöpfend extrahiert Nach Entfernen des Lösungsmittels wird der Rückstand an Kieselgel (Cyclohexan/Ethylacetat = 2 : 1 → 1 : 1) gereinigt.
Ausbeute: 9.26 g (logP: 1.26)

### Herstellung von Ausgangsstoffen der Formel (XI)

### Beispiel 7

### 1-(3-Chlorpyridin-2-yl)-1H-pyrazole-3.5-dicarbonsäuredimethylester:

Zu einer Lösung aus 15.6 g (64.8 mmol) 2-Pyrrolidino-4-oxo-2-pentendicarbonsäuredimethylester und 20.4 g (64.8 mmol) 3-Chlor-2-pyridin-2-ylhydrazin Toluolsulfonsäuresalz im 84 ml Methanol wird eine Spatelspize Toluolsulfonsäure gegeben und 5 h auf 50°C erhitzt. Anschließend wird mit 12.3 g (64.8 mmol) Toluolsulfonsäure Monohydrat versetzt und zuerst 1 h bei 50°C und 1 h unter Rückfluß gerührt Nach dem Abkühlen auf 0°C werden die ausgefallenen Kristalle abgesaugt und über Kieselgel (Cyclohexan/Ethylacetat = 1 : 1) filtriert.
Ausbeute: 8.56 g (logP: 1.97)

Beispiele zur biologischen Wirksamkeit der erfindungsgemässen Verbindungen

### Beispiel Nr, 1

### Heliothis virescens -Test

| | |
|---|---|
| Lösungsmittel: | 1 % N-Methylpyrolidon (NMP) |
| | 1 % Diacetonalkohol |

### Farbstoff: Brillantsulfoflavin zum Anfärben des Wassers

Zur Herstellung einer zweckmäßigen Wirkstoffzubereitung vermischt man den Wirkstoff mit den angegebenen Mengen Lösungsmittel und verdünnt das Konzentrat mit angefärbtem Wasser auf die gewünschte Konzentration.

Die Heliothis virescens Eier werden mit einer Wirkstoffzubereitung der gewünschten Konzentration behandelt.

Nach der gewünschten Zeit wird die Wirkung in % bestimmt. Dabei bedeutet 100 %, dass alle Eier/Larven abgetötet wurden; 0 % bedeutet, dass keine Eier/Larven abgetötet wurden.

Bei diesem Test zeigen z. B. die folgenden Verbindungen der Herstellungsbeispiele gute Wirksamkeit: siehe Tabelle

| **Heliothis virescens -Test** | | |
|---|---|---|
| Wirkstoff | Wirkstoffkonzentration in ppm | Abtötungsgrad nach 6-7^{d} in % |
| I-1-13 | 300 | 100 |
| I-1-14 | 300 | 100 |
| I-1-15 | 300 | 100 |

### Beispiel Nr. 2

### Myzus persicae - Test

| | |
|---|---|
| Lösungsmittel: | 1 % N-methylpyrolidon (NMP) |
| | 1 % Diacetonalkohol |

### Farbstoff: Brillantsulfoflavin zum Anfärben des Wassers

Zur Herstellung einer zweckmäßigen Wirkstoffzubereitung vermischt man den Wirkstoff mit den angegebenen Mengen Lösungsmittel und verdünnt das Konzentrat mit angefärbtem Wasser auf die gewünschte Konzentration.

Den Myzus persicae wird eine Wirkstoffzubereitung der gewünschten Konzentration zur Aufnahme zur Verfügung gestellt.

Nach der gewünschten Zeit wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele gute Wirksamkeit: siehe Tabelle

| **Myzus persicae -Test** | | |
|---|---|---|
| Wirkstoff | Wirkstoffkanzentration in ppm | Abtötungsgrad nach 6-7^{d} in % |
| I-1-13 | 30 | 100 |
| I-1-14 | 30 | 100 |
| I-1-15 | 30 | 100 |

### Beispiel Nr. 3

### Aedes Aegypti ― Test

| | |
|---|---|
| Lösungsmittel: | 1 % N-methylpyrolidon (NMP) |
| | 1 % Diacetonalkohol |

### Farbstoff: Brillantsulfoflavin zum Anfärben des Wassers

Zur. Herstellung einer zweckmäßigen Wirkstoffzubereitung vermischt man den Wirkstoff mit den angegebenen Mengen Lösungsmittel und verdünnt das Konzentrat mit angefärbtem Wasser auf die gewünschte Konzentration.

Die *Aedes aegypti* Larven werden mit einer Wirkstoffzubereitung der gewünschten Konzentration behandelt.

Nach der gewünschten Zeit wird die Wirkung in % bestimmt. Dabei bedeutet 100 %, dass alle *Aedes aegypti* abgetötet wurden; 0 % bedeutet, dass keine Aedes aegypti abgetötet wurden.

Bei diesem Test zeigen z. B. die folgenden Verbindungen der Herstellungsbeispiele gute Wirksamkeit: siehe Tabelle

| Aedes Aegypti -Test | | |
|---|---|---|
| Wirkstoff | Wirkstoffkonzentration in ppm | Abtötungsgrad nach 2-4^{d} in % |
| I-1-13 | 30 | 100 |
| I-1-14 | 30 | 100 |
| I-1-15 | 30 | 100 |

### Beispiel Nr. 4

### Diabrotica undecimpunctata - Test

| | |
|---|---|
| Lösungsmittel: | 1 % N-methylpyrolidon (NMP) |
| | 1 % Diacetonalkohol |

### Farbstoff: Brillantsulfoflavin zum Anfärben des Wassers

Zur Herstellung einer zweckmäßigen Wirkstoffzubereitung vermischt man den Wirkstoff mit den angegebenen Mengen Lösungsmittel und verdünnt das Konzentrat mit angefärbtem Wasser auf die gewünschte Konzentration.

Die Diabrotica undecimpunctata Eier werden mit einer Wirkstoffzubereitung der gewünschten Konzentration behandelt.

Nach der gewünschten Zeit wird die Wirkung in % bestimmt. Dabei bedeutet 100 %, dass alle Eier/Larven abgetötet wurden; 0 % bedeutet, dass keine Eier/Larven abgetötet wurden.

Bei diesem Test zeigen z. B. die folgenden Verbindungen der Herstellungsbeispiele gute Wirksamkeit: siehe Tabelle

| **Diabrotica undecimpunctata -Test** | | |
|---|---|---|
| Wirkstoff | Wirkstoffkonzentration in ppm | Abtötungsgrad nach 2-5^{d} in % |
| I-1-13 | 300 | 100 |
| I-1-14 | 300 | 100 |
| I-1-15 | 300 | 100 |

### Beispiel Nr. 5

### Phaedon-Test (Spritzbehandlung)

| | | |
|---|---|---|
| Lösungsmittel: | 78,0 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben *(Brassica pekinensis)* werden mit einer Wirkstoffzubereitung der gewünschten Konzentration gespritzt und nach dem Abtrocknen mit Larven des Meerrettichblattkäfers *(Phaedon cochleariae)* besetzt

Nach der gewünschten Zeit wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele eine Wirkung von ≥ 80 % bei einer Aufwandmenge von 100 g/ha:
Bsp. Nr. I*-*1-2, I-1-3, I-1-4, I-1-5, I-1-6, I-1-10, I-1-11, I-1-12, I-1-13, I-1-14, I-1-15, I-1-16, I-1-17, I-I-18, I-1-19, I-1-22, I-1-23, I-1-24, I-1-25, I-1-28, I-1-29, I-1-30, I-1-31, I-1-32, I-1-33, I-1-34, I-1-35, I-1-36, I-1-37, I-1-38, I-1-39, I-1-40, I-1-217, I-1-218, I-1-219, I-1-220, I-1-222, I-1-223, I-1-224, I-1-225, I-1-226, I-1-38, I-1-42, I-1-43, I-1-44, I-1-45, I-1-46, I-1-47, I-1-48, I-1-49, I-1-50, I-1-51, I-1-52, 1-1-53, I-1-54, I-1-55, I-1-56, I-1-57, I-1-58, I-1-59, I-1-60, I-1-61, I-1-62, I-1-63, I-1-64, I-1-65, I-1-66, I-1-68, I-1-69, I-1-70, I-1-71, I-1-72, I-1-73, I-1-74, I-1-75, I-1-76, I-1-77, I-1-78, I-1-79, I-1-80, I-1-81, I-1-82, I-1-83, I-1-84, I-1-85, I-1-86, I-1-87, I-1-92, I-1-93, I-1-97, I-1-99, I-1-100, I-I-101, I-1-113, I-1-120, I-1-121, I-1-125, I-1-128, I-1-129, I-1-130, I-1-131, I-1-134, I-1-135, I-1-I37, I-1-138, I-1-139, I-1-140, I-1-141, I-1-142, I-1-144, I-1-145, I-1-146, I-1-147, I-1-148, I-1-149, I-1-150, I-1-151, I-1-152, I-1-155, I-1-156, I-1-157, I-1-158, I-1-163, I-1-165, I-1-166, I-1-167, I-1-168, I-1-169, I-1-170, I-1-171, I-1-172, I-1-173, I-1-174, I-1-175, I-1-176, I-1-177, I-1-178, I-1-179, I-1-180, I-1-181, I-1-182, I-1-183, I-1-184, I-1-185, I-1-186, I-1-187, I-1-188, I-1-189, I-1-190, I-1-191, I-1-192, I-1-193, I-1-195, I-1-196, I-1-199, I-1-201, I-1-203, I-1-204, I-1-206, I-1-207, I-1-208, I-1-209, I-1-210, I,1-211, I-1-212, I-1-213, I-1-214, I-1-215, I-1-216, I-1-227, I-1-228, I-1-230, I-1-231, I-1-233, I-1-234, I-1-235, I-1-236, I-1-237, I-1-238, I-1-239, I-1-240, I-1-241, I-1-242, I-1-243, I-1-244, I-1-245, I-1-246, I-1-247, I-1-248, I-1-251, I-1-252, I-1-253, I-1-255, I-1-258, I-1-259, I-1-260, I-1-267, I-1-272, I-1-273, I-1-274, I-1-275, I-1-276, I-1-277, I-1-279, I-1-282, I-1-283, I-1-284, I-1-285, I-1-286, I-1-287, I-1-288, I-1-289, I-1-290, I-1-291, I-1-292, I-1-293, I-1-294, I-1-295, I-1-296, I-1-297, I-1-298, I-1-299, I-1-300, I-1-302, I-1-303, I-1-304, I-1-305, I-1-307, I-1-308, I-1-309, I-1-310, I-1-312, I-1-313, I-1-324, I-1-326, I-1-327, I-1-328, I-1-329, I-1-330, I-1-331, I-1-332, I-1-333, I-1-334, I-1-335, I-1-336, I-1-337, I-1-338, I-1-339, I-1-340, I-1-342, I-1-343, I-1-346, I-1-347, I-1-348, I-1-349, I-1-350, I-1-352, I-1-353, I-1-354, I-1-355, I-2-2

### Beispiel Nr. 6

### Spodoptera frugiperda-Test

| | | |
|---|---|---|
| Lösungsmittel: | 7 | Gewichtsteile Dimethylformamid |
| Emulgator: | 2 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter *(Brassica oleracea)* werden durch Tauchen in die Wirkstoffzubereitung der gewünschten Konzentration behandelt und mit Raupen des Heerwurms *(Spodoptera frugiperda)* besetzt, solange die Blätter noch feucht sind.

Nach der gewünschten Zeit wird die Abtötung in % bestimmt. Dabei bedeutet 100 %, dass alle Raupen abgetötet wurden; 0 % bedeutet, dass keine Raupen abgetötet wurden.

Bei diesem Test zeigt z. B. die folgenden Verbindungen der Herstellungsbeispiele gute Wirksamkeit: siehe Tabelle

| **Spodoptera frugiperda -Test** | | |
|---|---|---|
| Wirkstoff | Wirkstoffkonzentration in g/ha | Abtötungsgrad nach 7 in % |
| I-1-1 | 100 | 100 |
| I-1-2 | 100 | 100 |
| I-1-3 | 100 | 100 |
| I-1-4 | 100 | 100 |
| I-1-5 | 100 | 100 |
| I-1-6 | 100 | 100 |
| I-1-7 | 100 | 100 |
| I-1-8 | 100 | 100 |
| I-1-10 | 100 | 100 |
| I-1-11 | 100 | 100 |
| I-1-12 | 100 | 100 |
| I-1-13 | 100 | 100 |
| I-1-14 | 100 | 100 |
| I-1-15 | 100 | 100 |
| I-1-16 | 100 | 100 |
| I-1-17 | 100 | 100 |
| I-1-18 | 100 | 100 |
| I-1-19 | 100 | 100 |
| I-1-20 | 100 | 100 |
| I-1-21 | 100 | 100 |
| I-1-22 | 100 | 100 |
| I-1-23 | 100 | 100 |
| I-1-24 | 100 | 100 |
| I-1-25 | 100 | 100 |
| I-1-26 | 100 | 100 |
| I-1-27 | 20 | 83 |
| I-1-28 | 100 | 100 |
| I-1-29 | 100 | 100 |
| I-1-30 | 100 | 100 |
| I-1-31 | 100 | 100 |
| I-1-32 | 100 | 100 |
| I-1-33 | 100 | 100 |
| I-1-34 | 100 | 100 |
| I-1-35 | 100 | 100 |
| I-1-36 | 100 | 100 |
| I-1-37 | 100 | 100 |
| I-1-39 | 100 | 100 |
| I-1-40 | 100 | 100 |

### Beispiel Nr. 7

### Spodoptera exigua-Test

| | |
|---|---|
| Lösungsmittel: | 7 Gewichtsteile Dimethylformamid |
| Emulgator: | 2 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Bei erforderlicher Zugabe von Ammoniumsalzen, Penetrationsförderer oder Ammoniumsalzen und Penetrationsförderer werden diese in einer Konzentration von 1000 ppm nach dem Verdünnen jeweils der fertigen Präparatelösung zupipettiert. Die Beispiele I-1-3, I-1-5, I-1-6, I-1-8, I-1-22 werden ohne Zugabe von Ammoniumsalzen oder Penetrationsförderem geprüft.

Kohlpflanzen (*Brassica oleracea)* werden durch Spritzen der Wirkstoffzubereitung mit der gewünschten Konzentration behandelt und mit Raupen der Zuckerrübeneule *(Spodoptera exigua)* besetzt, solange die Blätter noch feucht sind.

Nach der gewünschten Zeit wird die Abtötung in % bestimmt. Dabei bedeutet 100 %, dass alle Raupen abgetötet wurden; 0 % bedeutet, dass keine Raupen abgetötet wurden.

Bei diesem Test zeigen z. B. die folgenden Verbindungen der Herstellungsbeispiele eine Wirkung von ≥ 80 % bei einer Aufwandmenge von 4 ppm:
Bsp. Nr. I-1-3, I-1-5, I-1-6, I-1-8, I-1-22, I*-*1-31, I-1-35, I-1-47, I-1-48, I-1-52, I-1-53, I-1-55, I-1-57, I-1-170, I-1-291, I-1-295, I-1-296, I-1-297, I-1-299, I-1-54

### Beispiel Nr. 8

### Plutella xylostella - Test

| | |
|---|---|
| Lösungsmittel: | 7 Gewichtsteile Dimethylformamid |
| Emulgator: | 2 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Bei erforderlicher Zugabe von Ammoniumsalzen, Penetrationsförderer oder Ammoniumsalzen und Penetrationsförderer werden diese jeweils in einer Konzentration von 1000 ppm nach dem Verdünnen jeweils der fertigen Präparatelösung zupipettiert. Die Beispiele I-1-3, I-1-5, I-1-6, I-1-8, I-1-22 werden ohne Zugabe von Ammoniumsalzen oder Penetrationsförderem geprüft.

Kohlblätter *(Brassica oleracea)* werden durch Spritzen der Wirkstoffzubereitung mit der gewünschten Konzentration behandelt und mit Raupen der Kohlschabe *(Plutella xylostella)* besetzt, solange die Blätter noch feucht sind.

Nach der gewünschten Zeit wird die Abtötung in % bestimmt. Dabei bedeutet 100 %, dass alle Raupen abgetötet wurden; 0 % bedeutet, dass keine Raupen abgetötet wurden.

Bei diesem Test zeigen z. B. die folgenden Verbindungen der Herstellungsbeispiele eine Wirkung von ≥ 80 % bei einer Aufwandmenge von 20 ppm:
Bsp. Nr. I-1-3, I-1-5, I-1-6, I-1-8, I-1-22, I-1-65, I-1-239, I-1-240, I-1-245, I-1-217, I-1-220, I-1-223, I-1-224, I-1-31, I-1-55, I-1-56, I-1-57, I-1-63, I-1-66, I-1-68, I-1-69, I-1-76, I-1-92, I-1-97, I-1-98, I-I-101, I-1-113, I-1-121, I-1-147, I-1-170, I-1-192, I-1-195, I-1-204, I-1-206, I-1-209, I-1-210, I-1-212, I-1-215, I-1-238, I-1-244, I-1-247, I-1-248, I-1-249, I-1-250, I-1-251, I-1-274, I-1-275, I-1-276, I-1-291, I-1-295, I-1-296, I-1-297, I-1-299, I-1-25, I-1-35, I-1-36, I-1-38, I-1-43, I-1-146, I-1-47, I-1-48, I-1-52, I-1-53, I-1-54, I-1-104, I-1-106, I-1-107, I-1-108, I-1-143, I-1-88, I-1-139

### Beispiel Nr. 9

### Spodoptera frugiperda-Test

| | |
|---|---|
| Lösungsmittel: | 7 Gewichtsteile Dimethylformamid |
| Emulgator: | 2 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter *(Brassica oleracea)* werden durch Tauchen in die Wirkstoffzubereitung der gewünschten Konzentration behandelt und mit Raupen des Heerwurms *(Spodoptera frugiperda)* besetzt, solange die Blätter noch feucht sind.

Nach der gewünschten Zeit wird die Abtötung in % bestimmt. Dabei bedeutet 100 %, dass alle Raupen abgetötet wurden; 0 % bedeutet, dass keine Raupen abgetötet wurden.

Bei diesem Test zeigen z. B. die folgenden Verbindungen der Herstellungsbeispiele gute Wirksamkeit: siehe Tabelle

| **Spodoptera frugiperda-Test** | | |
|---|---|---|
| Wirkstoff | Wirkstoffkonzentration in ppm | Abtötungsgrad nach 7^{d} in % |
| I-1-3 | 0,8 | 100 |
| I-1-5 | 0,8 | 100 |
| I-1-6 | 0,8 | 100 |
| I-1-8 | 0,8 | 100 |
| I-1-9 | 100 | 100 |
| I-1-22 | 4 | 100 |

### Beispiel Nr. 10

### Heliothis armigera - Test

| | |
|---|---|
| Lösungsmittel: | 7 Gewichtsteile Dimethylformamid |
| Emulgator: | 2 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Bei erforderlicher Zugabe von Ammoniumsalzen, Penetrationsförderer oder Ammoniumsalzen und Penetrationsförderer werden diese in einer Konzentration von 1000 ppm nach dem Verdünnen jeweils der fertigen Präparatelösung zupipettiert. Die Beispiele I-1-3, I-1-5, I-1-6, I-1-8 und I-1-22 werden ohne Zugabe von Ammoniumsalzen oder Penetrationsförderern geprüft.

Baumwollpflanzen *(Gossypium hirsutum),* werden durch Spritzen der Wirkstoffzubereitung mit der gewünschten Konzentration behandelt und mit Raupen des Baumwollkapselwurms *(Heliothis armigera)* besetzt, solange die Blätter noch feucht sind.

Nach der gewünschten Zeit wird die Abtötung in % bestimmt. Dabei bedeutet 100 %, dass alle Raupen abgetötet wurden; 0 % bedeutet, dass keine Raupen abgetötet wurden.

Bei diesem Test zeigen z. B. die folgenden Verbindungen der Herstellungsbeispiele eine Wirkung von ≥ 80 % bei einer Aufwandmenge von 20 ppm:
Bsp. Nr. I-1-3, I-1-5, I-1-6, I-1-8, I-1-22, I-1-63, I-1-65, I-1-66, I-1-68, I-1-69, I-1-192, I-1-240, I-1-245, I-1-217, I-1-220, I-1-223, I-1-224, I-1-31, I-1-35, I-1-36, I-1-38, I-1-43, I-1-46, I-1-47, I-1-48, I-1-52, I-1-53, I-1-54, I-1-55, I-1-56, I-1-57, I-1-76, I-1-88, I-1-131, I-1-139, I-1-92, I-1-97, I-1-98, I-1-100, I-1-101, I-1-113, I-1-121, I-1-147, I-1-170, I-1-195, I-1-204, I-1-206, I-1-212, I-1-215, I-1-238, I-1-244, I-1-247, I-1-248, I-1-249, I-1-250, I-1-251, I-1-274, I-1-275, I-1-276, I-1-291, I-1-295, I-1-296, I-1-297, I-1-299, I-1-104, I-1-106, I-1-107, I-1-108, I-1-143

### Beispiel Nr. 11

### Spodoptera exigua-Test; resistenter Stamm

| | |
|---|---|
| Lösungsmittel: | 7 Gewichtsteile Dimethylformamid |
| Emulgator: | 2 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Bei erforderlicher Zugabe von Ammoniumsalzen, Penetrationsförderer oder Ammoniumsalzen und Penetrationsförderer werden diese in einer Konzentration von 1000 ppm nach dem Verdünnen jeweils der fertigen Präparatelösung zupipettiert Die Beispiele I-1-5, I-1-6, I-1-22 werden ohne Zugabe von Ammoniumsalzen oder Penetrationsförderern geprüft.

Kohlpflanzen(*Brassica oleracea)* werden durch Spritzen der Wirkstoffzubereitung mit der gewünschten Konzentration behandelt und mit Raupen der Zuckerrübeneule *(Spodoptera exigua, resistenter Stamm)* besetzt, solange die Blätter noch feucht sind.

Nach der gewünschten Zeit wird die Abtötung in % bestimmt. Dabei bedeutet 100 %, dass alle Raupen abgetötet wurden; 0 % bedeutet, dass keine Raupen abgetötet wurden.

Bei diesem Test zeigen z. B. die folgenden Verbindungen der Herstellungsbeispiele eine Wirkung von ≥ 80 % bei einer Aufwandmenge von 4 ppm:
Bsp. Nr. I-1-5, I-1-6, I-1-22, I-1-31, I-1-35, I-1-38, I-1-46, I-1-47, I-1-48, I-1-52, I-1-53, I-1-54, I-1-56, I-1-57, I-1-170, I-1-295, I-1-296, I-1-297, I-1-299

### Beispiel Nr. 12

### Liriomyza trifolii

| | |
|---|---|
| Lösungsmittel: | 78 Gewichtsteile Aceton |
| | 1,5 Gewichtsteile Dimethylformamid |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Bohnenblattscheiben *(Phoseolus vulgaris).* die von Larven Minierfliege *(Liriomyza trifolii)* befallen sind, werden mit einer Wirkstoffzubereitung der gewünschten Konzentration gespritzt

Nach der gewünschten Zeit wird die Wirkung in % bestimmt. Dabei bedeutet 100 %, dass alle Minierfliegen abgetötet wurden; 0 % bedeutet, dass keine Minierfliege abgetötet wurde.

Bei diesem Test zeigen z. B. die folgenden Verbindungen der Herstellungsbeispiele gute Wirksamkeit: siehe Tabelle

| **Liriomyza trifolii -Test** | | |
|---|---|---|
| Wirkstoff | Wirkstoffkonzentration in ppm | Abtötungsgrad nach 7 in % |
| I-1-3 | 500 | 98 |
| I-1-4 | 500 | 95 |
| I-1-22 | 500 | 98 |

### Beispiel Nr. 13

### Lucilia cuprina-Test

| | |
|---|---|
| Lösungsmittel: | Dimethylsulfoxid |

Zur Herstellung einer zweckmäßigen Wirkstoffzubereitung vermischt man 1 Gewichtsteil Wirkstoff mit der angegebenen Menge Wasser und verdünnt das Konzentrat mit Wasser auf die gewünschten Konzentration.

Gefäße, die Pferdefleisch enthalten, das mit der Wirkstoffzubereitung der gewünschten Konzentration behandelt wurde, werden mit *Lucilia cuprina* Larven besetzt.

Nach der gewünschten Zeit wird die Abtötung in % bestimmt. Dabei bedeutet 100 %, dass alle Larven abgetötet wurden; 0 % bedeutet, dass keine Larven abgetötet wurden.

Bei diesem Test zeigen z. B. die folgenden Verbindungen der Herstellungsbeispiele eine Wirkung von ≥ 80 % bei einer Aufwandmenge von 100 ppm:
Bsp. Nr. I-1-1, I-1-2, I-1-3, I-1-4, I-1-5, I-1-6, I-1-7, I-1-8, I-1-9, I-1-12, I-1-14, I-1-15, I-1-16, I-1-17, I-1-18, I-1-19, I-1-21, I-1-22, I-1-29, I-1-30, I-1-31, I-1-217, I-1-220, I-1-223, I-1-224, I-1-42, I-1-43, I-1-46, I-1-47, I-1-48, I-1-52, I-1-53, I-1-54, I-1-55, I-1-56, I-1-57, I-1-64, I-1-65, I-1-66, I-1-67, I-1-68, I-1-74, I-1-88, I-1-92, I-1-97, I-1-104, I-1-107, I-1-108, I-1-109, I-1-111, I-1-114, I-1-122, I-1-123, I-1-192, I-1-238, I-1-239, I-1-240, I-1-244, I-1-247, I-1-274, I-1-275, I-1-291, I-1-295, I-1-296, I-1-297, I-1-299, I-1-323

### Beispiel Nr. 14

### Boophilus microplus ―Test (Injektion)

| | |
|---|---|
| Lösungsmittel: | Dimethylsulfoxid |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Lösungsmittel auf die gewünschte Konzentration.

Die Wirkstofflösung wird in das Abdomen *(Boophilus microplus)* injiziert, die Tiere werden in Schalen überführt und in einem klimatisierten Raum aufbewahrt.

Nach der gewünschten Zeit wird die Wirkung in % bestimmt. Dabei bedeutet 100%, dass keine Zecke fertile Eier gelegt hat.

Bei diesem Test zeigen z. B. die folgenden Verbindungen der Herstellungsbeispiele eine Wirkung von ≥ 80 % bei einer Aufwandmenge von 20 µg/Tier:
Bsp. Nr. I-1-1, I-1-2, I-1-3, I-1-4, I-1-5, I-1-6, I-1-7, I-1-8, I-1-9, I-1-12, I-1-14, I-1-15, I-1-16, I-1-17, I-1-18, I-1-19, I-1-21, I-1-29, I-1-30, I-1-31, I-1-217, I-1-222, I-1-223, I-1-224, I-1-42, I-1-43, I-1-46, I-1-47, I-1-48, I-1-52, I-1-53, I-1-54, I-1-55, I-1-56, I-1-57, I-1-64, I-1-65, I-1-66, I-1-67, I-1-68, I-1-74, I-1-88, I-1-92, I-1-97, I-1-104, I-1-107, I-1-108, I-1-109, I-1-111, I-1-114, I-1-122, I-1-123, I-1-192, I-1-238, I-1-239, I-1-240, I-1-244, I-1-247, I-1-274, I-1-275, I-1-291, I-1-295, I-1-296, I-1-297, I-1-299, I-1-323

### Beispiel Nr. 15

### Musca domestica-Test

| | |
|---|---|
| Lösungsmittel: | Dimethylsulfoxid |

Zur Herstellung einer zweckmäßigen Wirkstoffzubereitung vermischt man 1 Gewichtsteil Wirkstoff mit der angegebenen Menge Wasser und verdünnt das Konzentrat mit Wasser auf die gewünschten Konzentration.

Gefäße, die einen Schwamm enthalten, der mit der Wirkstoffzubereitung der gewünschten Konzentration behandelt wurde, werden mit *Musca domestica Adulten* besetzt.

Nach der gewünschten Zeit wird die Abtötung in % bestimmt. Dabei bedeutet 100 %, dass alle Fliegen abgetötet wurden; 0 % bedeutet, dass keine Fliegen abgetötet wurden. Bei den Beispielen I-1-16,1-1-19 und I-1-30 handelt es sich um einen Knock-down-effect.

Bei diesem Test zeigen z. B. die folgenden Verbindungen der Herstellungsbeispiele eine Wirkung von ≥80 % bei einer Aufwandmenge von 100 ppm:
Bsp. Nr. I-1-16 I-1-19, I-1-30, I-1-217, I-1-42, I-1-43, I-1-46, I-1-47, I-1-48, I-1-52, I-1-53, I-1-55, I-1-64, I-1-66, I-1-107, I-1-114, I-1-192, I-1-274, I-1-275, I-1-296, I-1-297, I-1-299

### Beispiel Nr. 16

### Spodoptera frugiperda-Test (Spritzbehandlung)

| | | |
|---|---|---|
| Lösungsmittel: | 78,0 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Maisblattscheiben *(Zea mays)* werden mit einer Wirkstoffzubereitung der gewünschten Konzentration gespritzt und nach dem Abtrocknen mit Raupen des Heerwurms *(Spodoptera frugiperda*) besetzt.

Nach der gewünschten Zeit wird die Wirkung in % bestimmt. Dabei bedeutet 100 %, dass alle Raupen abgetötet wurden; 0 % bedeutet, dass keine Raupe abgetötet wurde.

Bei diesem Test zeigen z. B. die folgenden Verbindungen der Herstellungsbeispiele eine Wirkung von ≥ 80 % bei einer Aufwandmenge von 100 g/ha:
Bsp. Nr. I-1-217, I-1-218, I-1-219, I-1-220, I-1-221, I-1-222, I-1-223, I-1-224, I-1-38, I-1-42, I-1-43, I-1-44, I-1-45, I-1-46, I-1-47, I-1-48, I-1-49, I-1-50, I-1-51, I-1-52, I-1-53, I-1-54, I-1-55, I-1-56, I-1-57, I-1-58, I-1-59, I-1-60, I-1-61, I-1-62, I-1-63, I-1-64, I-1-65, I-1-66, I-1-68, I-1-69, I-1-70, I-1-71, I-1-72, I-1-73, I-1-74, I-1-75, I-1-76, I-1-77, I-1-78, I-1-79, I-1-80, I-1-81, I-1-82, I-1-83, I-1-84, I-1-85, I-1-86. I-1-87, I-1- 92, I-1-93, I-1-97, I-1-99, I-1-100, I-1-101, I-1-102, I-1-103, I-1-113, I-1-116, I-1-117, I-1-118, I-1-119, I-1-I20, I-1-121, I-1-125, I-1-126, I-1-127, I-1-128, I-1-129, I-1-130, I-1-131, I-1-134, I-1-135, I-1-136, I-1-137, I-1-138, I-1-139, I-1-140, I-1-141, I-1-142, I-1-144, I-1-145, I-1-146, I-1-147, I-1-148, I-1-149, I-1-150, I-1-151, I-1-152, I-1-153. I-1-154, I-1-155, I-1-156, I-1-157, I-1-158, I-1-160, I-1-161, I-1-163, I-1-165, I-1-166, I-1-167, I-1-168, I-3-1, I-1-169, I-1-170, I-1-171, I-1-172, I-1-173, I-1-174, I-1-175, I-1-176, I-1-177, I-1-178, I-1-179, I-1-180, I-1-181, I-1-182, I-1-183, I-1-184, I-1-185, I-1-186, I-1-187, I-1-188, I-1-189, I-1-190, I-1-191, I-1-192, I-1-193, I-1-195, I-1-196, I-1-199, I-1-200, I-1-201, I-1-202, I-1-203, I-1-204, I-1-205, I-1-206, I-1-207, I-1-208, I-1-209, I-1-210, I-1-211, I-1-212, I-1-213, I-1-214, I-1-215, I-1-216, I-1-225, I-1-226, I-1-227, I-1-228, I-1-231, I-1-233, I-1-234, I-1-235, I-1-237, I-1-238, I-1-239, I-1-240, I-1-241, I-1-242, I-1-243, I-1-244, I-1-245, I-1-246, I-1-247, I-1-248, I-1-249, I-1-250, I-1-251, I-1-252, I-1-253, I-1-255, I-1-257, I-1-258, I-1-259, I-1-260, I-1-261, I-1-262, I-1-263, I-1-264, I-1-266, I-1-267, I-1-268, I-1-269, I-1-270, I-1-271, I-1-272, I-1-273, I-1-274, I-1-275, I-1-276, I-1-277, I-1-279, I-1-282, I-1-283, I-1-284, I-1-285, I-1-286, I-1-287, I-1-289, I-2-8, I-2-12, I-2-17, I-2-19, I-1-290, I-1-291, I-1-292, I-1-293, I-1-294, I-1-295, I-1-296, I-1-297, I-1-298, I-1-299, I-1-300, I-1-301, I-1-302, I-1-303, 1-1-304, I-1-305, I-1-307, I-1-308, I-1-309, I-1-310, I-1-311, I-1-312, I-1-313, I-1-314, I-1-315, I-1-318, I-1-324, I-1-326, I-1-327, I-1-328, I-1-329, I-1-330, I-1-331, I-1-332, I-1-333, I-1-334, I-1-335, I-1-336, I-1-337, I-1-338, I-1-339, I-1-341, I-1-343, I-1-344, I-1-346, I-1-347, I-1-348, I-1-349, I-1-350, I-1-351, I-1-352, I-1-353, I-1-354, I-1-355, I-2-2 , I-2-3

### Beispiel Nr. 17

### Myzus-Test (Spritzbehandlung)

| | | |
|---|---|---|
| Lösungsmittel: | 78,0 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben (*Brassica pekinensis),* die von allen Studien der Grünen Pfirsichblattlaus (*Myzus persicae*) befallen sind, werden mit einer Wirkstoffzubereitung der gewünschten Konzentration gespritzt

Nach der gewünschten Zeit wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele eine Wirkung von ≥ 80 % bei einer Aufwandmenge von 100 g/ha:
Bsp. Nr. I-1-64, I-1-68, I-1-74, I-1-75, I-1-,76, I-1-77, I-1-78, I-1-80, I-1-81, I-1-83, I-1-84, I-1-92, I-1-97, I-1-99, I-1-100, I-1-101, I-1-113, I-1-116, I-1-117, I-1-118, I-1-120, I-1-121, I-1-126, I-1-127, I-1-128, I-1-130, I-1-131, I-1-134, I-1-135, I-1-36, I-1-137, I-1-138, I-1-139, I-1-142, I-1-144, I-1-145, I-1-146, I-1-150, I-1-152, I-1-163, I-1-174, I-1-176, I-1-177, I-1-178, I-1-179, I-1-180, I-1-184, I-1-185, I-1-186, I-1-198, I-1-203, I-1-207, I-1-209, I-1-211, I-1-212, I-1-216, I-1-272, I-2-10, I-1-308, I-1-326, I-1-327, I-1-329, I-1-330, I-1-331, I-1-332, I-1-333, I-1-336, I-1-338, I-1-339, I-1-354

### Beispiel Nr. 18

### Aphis gossypii -Test

| | | |
|---|---|---|
| Lösungsmittels: | 7 | Gewichtsteile Dimethylformamid |
| Emulgator: | 2 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Bei erforderlicher Zugabe von Ammoniumsalzen, Penetrationsförderer oder Ammoniumsalzen und Penetrationsförderer werden diese in einer Konzentration von 1000 ppm nach dem Verdünnen jeweils der fertigen Präparatelösung zupipettiert

Baumwollblätter *(Gossypium hirsutum*), die stark von der Baumwollblattlaus *(Aphis gossypii)* befallen sind, werden mit einer Wirkstoffzubereitung mit der gewünschten Konzentration gespritzt.

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele eine Wirkung von ≥ 80 % bei einer Aufwandmenge von 100 ppm:
Bsp. Nr. I-1-68, I-1-75, I-1-76, I-1-88, I-1-98, I-1-99, I-1-104, I-1-108, I-1-113, I-1-131, I-1-139, I-1-143, I-1-176, I-1-251

## Patentansprüche

1. Verbindungen der Formel (I) 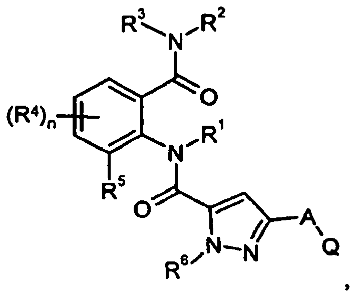 in welcher
R¹ für Wasserstoff, Methyl, Cyclopropyl, Cyanomethyl, Methoxymethyl, Methylthiomethyl, Methylsulfinylmethyl oder Methylsulfonylmethyl steht,
R² für Wasserstoff oder Methyl steht,
R³ für Wasserstoff oder für jeweils gegebenenfalls einfach oder mehrfach, gleich oder verschieden substituiertes C₁-C₆-Alkyl, C₁-C₆-Alkoxy steht, wobei die Substituenten unabhängig voneinander ausgewählt sein können aus Halogen, Cyano, Nitro, Hydroxy, C₁-C₆-Alkyl, C₃-C₆-Cycloalkyl, C₁-C₄-Alkoxy, C₁-C₄-Haloalkoxy, C₁-C₄-Alkylthio, C₁-C₄-Alkyl-sulfinyl, C₁-C₄-Alkylsulfonyl, C₁-C₄-Alkylsulfimino, C₁-C₄-Alkylsulfimino-C₁-C₄-alkyl, C₁-C₄-Alkylsulfimino-C₂-C₅-alkylcarbonyl, C₁-C₄-Alkylsulfoximino, C₁-C₄-Alkylsulfoximino-C₁-C₄-alkyl, C₁-C₄-Alkylsulfoximino-C₂-C₅-alkylcarbonyl, C₂-C₆-Alkoxycarbonyl, C₂-C₆-Alkylcarbonyl oder C₃-C₆-Trialkylsilyl,
R³ weiterhin für gegebenenfalls einfach oder mehrfach, gleich oder verschieden substituiertes C₃-C₆-Cycloalkyl steht, wobei die Substituenten unabhängig voneinander ausgewählt sein können aus Halogen, Cyano, Nitro, Hydroxy, C₁-C₆-Alkyl, C₃-C₆-Cycloalkyl, C₁-C₄-Alkoxy, C₁-C₄-Haloalkoxy, C₁-C₄-Alkylthio, C₁-C₄-Alkylsulfinyl, C₁-C₄-Alkylsulfonyl, C₁-C₄-Alkylsulfimino, C₁-C₄-Alkylsulfimino-C₁-C₄-alkyl, C₁-C₄-Alkylsulfimino-C₂-C₅-alkylcarbonyl, C₁-C₄-Alkylsulfoximino, C₁-C₄-Alkylsulfoximino-C₁-C₄-alkyl, C₁-C₄-Alkylsulfoximino-C₂-C₅-alkylcarbonyl, C₂-C₆-Alkoxycarbonyl, C₂-C₆-Alkylcarbonyl oder C₃-C₆-Tri-alkylsilyl,
R⁴ für Wasserstoff, C₁-C₄-Alkyl, C₁-C₂-Haloalkyl, Halogen, Cyano oder C₁-C₂-Haloalkoxy steht,
zwei benachbarte Reste R⁴ ausserdem für -(CH₂)₄-, -(CH=CH-)₂-, -O(CH₂)₂O-, -O(CF₂)₂O-,-(CH=CH-CH=N)- oder -(CH=CH-N=CH stehen,
n für 0 bis 3 steht,
R⁵ für C₁-C₄-Alkyl, C₃-C₆-Cycloalkyl, C₁-C₄-Haloalkyl, C₁-C₆-Halocycloalkyl, C₂-C₆-Alkenyl, C₂-C₄-Haloalkenyl, C₂-C₄-Alkinyl, C₂-C₄-Haloalkinyl, C₁-C₄-Alkoxy, C₁-C₄-Haloalkoxy, Fluor, Chlor, Brom, Iod, Cyano, Nitro oder C₃-C₆-Trialkylsilyl steht,
_{R}⁶ für Methyl oder 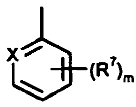 steht,
R⁷ unabhängig voneinander für Wasserstoff, Halogen oder C₁-C₄-Haloalkyl steht,
m für 1 oder 2 steht,
X für N, CH, CF, CCl oder CBr steht,
A für CH₂, CH(CH₃), C(CH₃)₂ oder CH₂CH₂ steht,
A weiterhin für -CH(CN)- steht,
Q für einen gegebenenfalls einfach oder mehrfach substituierten 5- oder 6-gliedrigen aromatischen heterocyclischen Ring der Reihe Q-36 bis Q-40 oder ein aromatisches 9-gliedriges annelliertes heterobicyclisches Ringsystem Q-54 bis Q-56 steht, wobei die Substituenten unabhängig voneinander ausgewählt sein können aus C₁-C₃-Alkyl, C₁-C₃-Haloalkyl, C₁-C₂-Alkoxy , Halogen, Cyano, Hydroxy, Nitro oder C₁-C₂-Haloalkoxy,
Q weiterhin für einen gegebenenfalls einfach oder mehrfach substituierten 5- oder 6-gliedrigen aromatischen heterocyclischen Ring der Reihe Q-36 bis Q-40 und Q-58 bis Q-59, einen aromatisches 9-gliedriges annelliertes heterobicyclisches Ringsystem Q-54 bis Q-56 sowie für ein 5-gliedrigen heterocyclischen Ring Q-60 bis Q-61 steht, wobei die Substituenten unabhängig voneinander ausgewählt sein können aus C₁-C₃-Alkyl, C₁-C₃-Haloalkyl, C₁-C₂-Alkoxy, Halogen, Cyano, Hydroxy, Nitro oder C₁-C₂-Haloalkoxy,
oder wobei die Substituenten unabhängig voneinander ausgewählt sein können aus Phenyl oder einem 5- oder 6-gliedrigen heteroaromatischen Ring, wobei Phenyl oder der Ring gegebenenfalls einfach oder mehrfach, gleich oder verschieden mit C₁-C₆-Alkyl, C₂-C₆-Alkenyl, C₂-C₆-Alkinyl, C₃-C₆-Cycloalkyl, C₁-C₆-Haloalkyl, C₂-C₆-Haloalkenyl, C₂-C₆-Haloalkinyl, C₃-C₆-Halocycloalkyl, Halogen, CN, NO₂, C₁-C₄-Alkoxy, C₁-C₄-Haloalkoxy substituiert sein können, 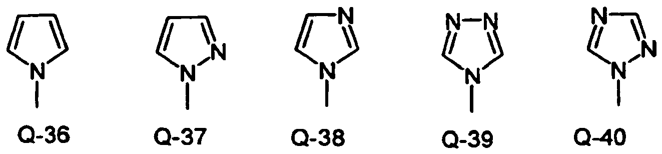 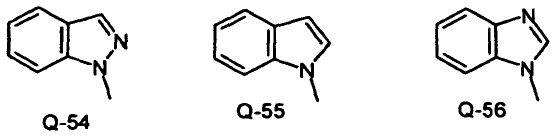 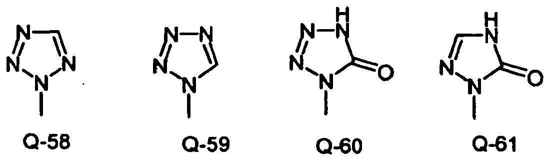
sowie ihre N-Oxide und Salze.

2. Verbindungen der Formel (1) gemäß Anspruch 1, in welcher
A für CH₂, CH(CH₃), C(CH₃)₂ oder CH₂CH₂ steht,
R³ für Wasserstoff oder für jeweils gegebenenfalls einfach oder mehrfach, gleich oder verschieden substituiertes C₁-C₆-Alkyl, C₁-C₆-Alkoxy steht, wobei die Substituenten unabhängig voneinander ausgewählt sein können aus Halogen, Cyano, Nitro, Hydroxy, C₁-C₆-Alkyl, C₃-C₆-Cycloalkyl, C₁-C₄-Alkoxy, C₁-C₄-Haloalkoxy, C₁-C₄-Alkylthio, C₁-C₄-Alkylsulfinyl, C₁-C₄-Alkylsulfonyl, C₁-C₄-Alkylsulfimino, C₁-C₄-Alkylsulfimino-C₁-C₄-alkyl, C₁-C₄-Alkylsulfimino-C₂-C₅-alkylcarbonyl, C₁-C₄-Alkylsulfoximino, C₁-C₄-Alkylsulfoximino-C₁-C₄-alkyl, C₁-C₄-Alkylsulfoximino-C₂-C₅-alkylcarbonyl, C₂-C₆-Alkoxycarbonyl, C₂-C₆-Alkylcarbonyl oder C₃-C₆-Trialkylsilyl,
Q für einen gegebenenfalls einfach oder mehrfach substituierten 5- oder 6-gliedrigen aromatischen heterocyclischen Ring der Reihe Q-36 bis Q-40 oder ein aromatisches 9-gliedriges annelliertes heterobicyclisches Ringsystem Q-54 bis Q-56 steht, wobei die Substituenten unabhängig voneinander ausgewählt sein können aus C₁-C₃-Alkyl, C₁-C₃-Haloalkyl, C₁-C₂-Alkoxy , Halogen, Cyano, Hydroxy, Nitro oder C₁-C₂-Haloalkoxy.

3. Verbindungen der Formel (I-1) gemäß Anspruch 1 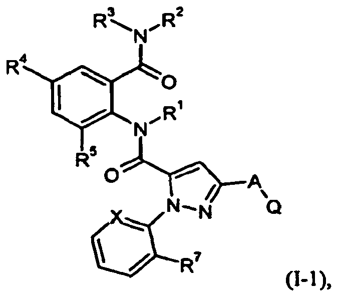 in welcher
R¹ für Wasserstoff steht,
R² für Wasserstoff steht,
R³ for C₁-C₄-Alkyl (Methyl, Ethyl, n-Propyl, iso-Propyl, n-Butyl, iso-Butyl, sec-Butyl oder tert-Butyl) oder Cyano-C₁-C₃-Alkyl (Cyanomethyl, 1-Cyanoethyl, 2-Cyanoethyl, 1-Cyano-n-Propyl, 2-Cyano-n-Propyl, 3-Cyano-n-Propyl, 1-Cyano-iso-Propyl, 2-Cyano-iso-Propyl) steht,
R⁴ für Wasserstoff, Methyl, Trifluormethyl, Cyano, Fluor, Chlor, Brom, Iod oder Trifluormethoxy steht,
R⁵ für Methyl, Fluor, Chlor, Brom oder Iod steht,
R⁷ für Fluor, Chlor oder Brom steht
X für N, CCl oder CH steht,
A für CH₂ oder CH(CH₃) steht,
Q für einen gegebenenfalls einfach oder mehrfach substituierten aromatischen heterocyclischen Ring der Reihe Q-37, Q-38, Q-39, Q-40, Q-58 und Q-59, sowie für ein 5-gliedrigen heterocyclischen Ring Q-60 steht, wobei die Substituenten unabhängig voneinander ausgewählt sein können aus C₁-C₃-Alkyl, C₁-C₃-Haloalkyl, C₁-C₂-Alkoxy, Halogen, Cyano, Hydroxy, Nitro oder C₁-C₂-Haloalkoxy,
oder wobei die Substituenten unabhängig voneinander ausgewählt sein können aus Phenyl oder einem 5- oder 6-gliedrigen heteroaromatischen Ring, wobei Phenyl oder der Ring gegebenenfalls einfach oder mehrfach, gleich oder verschieden mit C₁-C₆-Alkyl, C₂-C₆-Alkenyl, C₂-C₆-Alkinyl, C₃-C₆-Cycloalkyl, C₁-C₆-Haloalkyl, C₂-C₆-Haloalkenyl, C₂-C₆-Haloalkinyl, C₃-C₆-Halocycloalkyl, Halogen, CN, NO₂, C₁-C₄-Alkoxy, C₁-C₄-Haloalkoxy substituiert sein können,
sowie ihre N-Oxide und Salze.

4. Verfahren zur Herstellung von Verbindungen der allgemeinen Formel (I) gemäß Anspruch 1, **dadurch gekennzeichnet, dass**
(A) Aniline der Formel (II) 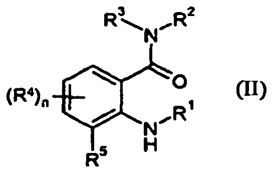 in welcher R¹, R², R³, R⁴, R⁵ und n die oben angegebenen Bedeutungen haben,
mit Carbonsäurechloriden der Formel (III) 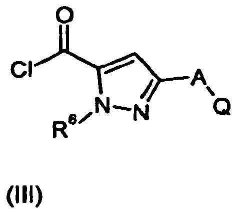 in welcher R⁶, A und Q die oben angegebenen Bedeutungen haben, in Gegenwart eines Säurebindemittels umsetzt,
(B) Aniline der Formel (II) 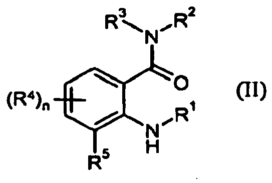 in welcher R¹, R², R³, R⁴, R⁵ und n die oben angegebenen Bedeutungen haben,
mit einer Carbonsäure der Formel (IV) 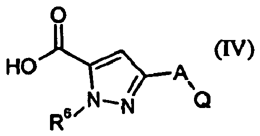 in welcher R⁶, A und Q die oben angegebenen Bedeutungen hat,
in Gegenwart eines Kondensationsmittels umsetzt oder indem man
(C) zur Synthese von Verbindungen der Formel (I), in welcher R¹ für Wasserstoff steht, Benzoxazinone der Formel (V) 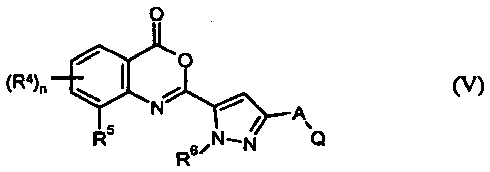 in welcher R⁴, R⁵, R⁶, A, Q und n die oben angegebenen Bedeutungen haben,
mit einem Amin der Formel (XV) 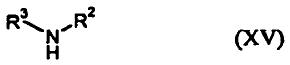 in welcher R² und R³ die oben angegebenen Bedeutungen haben,
in Gegenwart eines Verdünnungsmittels umsetzt.

5. Verwendung von Verbindungen der Formel (I) oder (1-1) gemäß den Ansprüchen 1 bis 3 zur Bekämpfung tierischer Schädlinge ausgenommen zur therapeutischen Behandlung des tierischen oder menschlichen Körpers.

6. Verfahren zur Bekämpfung tierischer Schädlinge, **dadurch gekennzeichnet, dass** man Verbindungen der Formel (I) oder (I-1) gemäß den Ansprüchen 1 bis 3 auf tierische Schädlinge und/oder phytopathogene Pilze und/oder deren Lebensraum und/oder Saatgut einwirken lässt ausgenommen zur therapeutischen Behandlung des tierischen oder menschlichen Körpers.

7. Verfahren zur Herstellung agrochemischer Zusammensetzungen, **dadurch gekennzeichnet, dass** man Verbindungen der Formel (I) oder (I-1) gemäß den Ansprüchen 1 bis 3 mit Streckmitteln und/oder oberflächenaktiven Stoffen vermischt.

8. Verwendung von Verbindungen der Formel (I) oder (I-1) gemäß den Ansprüchen 1 bis 3 zur Behandlung von Saatgut.

9. Verwendung von Verbindungen der Formel (I) oder (I-1) gemäß den Ansprüchen 1 bis 3 zur Behandlung von transgenen Pflanzen.

10. Verwendung von Verbindungen der Formel (I) oder (I-1) gemäß den Ansprüchen 1 bis 3 zur Behandlung von Saatgut transgener Pflanzen.

11. Saatgut, welches mit einer Verbindung der Formel (I) oder (I-1) gemäß den Ansprüchen 1 bis 3 umhüllt ist.

## Claims

1. Compounds of the formula (I) 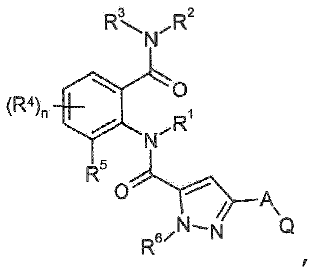 in which
R¹ represents hydrogen, methyl, cyclopropyl, cyanomethyl, methoxymethyl, methylthiomethyl, methylsulphinylmethyl or methylsulphonylmethyl,
R² represents hydrogen or methyl,
R³ represents hydrogen or represents C₁-C₆-alkyl, C₁-C₆-alkoxy, each of which is optionally substituted one or more times by identical or different substituents selectable independently of one another from halogen, cyano, nitro, hydroxyl, C₁-C₆-alkyl, C₃-C₆-cycloalkyl, C₁-C₄-alkoxy, C₁-C₄-haloalkoxy, C₁-C₄-alkylthio, C₁-C₄-alkylsulphinyl, C₁-C₄-alkylsulphonyl, C₁-C₄-alkylsulphimino, C₁-C₄-alkylsulphimino-C₁-C₄-alkyl, C₁-C₄-alkylsulphimino-C₂-C₅-alkylcarbonyl, C₁-C₄-alkylsulphoximino, C₁-C₄-alkylsulphoximino-C₁-C₄-alkyl, C₁-C₄-alkylsulphoximino-C₂-C₅-alkylcarbonyl, C₂-C₆-alkoxycarbonyl, C₂-C₆-alkylcarbonyl or C₃-C₆-trialkylsilyl,
R³ further represents C₃-C₆-cycloalkyl which is unsubstituted or substituted one or more times by identical or different substituents selectable independently of one another from halogen, cyano, nitro, hydroxyl, C₁-C₆-alkyl, C₃-C₆-cycloalkyl, C₁-C₄-alkoxy, C₁-C₄-halo-alkoxy, C₁-C₄-alkylthio, C₁-C₄-alkylsulphinyl, C₁-C₄-alkylsulphonyl, C₁-C₄-alkylsulphimino, C₁-C₄-alkylsulphimino-C₁-C₄-alkyl, C₁-C₄-alkylsulphimino-C₂-C₅-alkylcarbonyl, C₁-C₄-alkylsulphoximino, C₁-C₄-alkylsulphoximino-C₁-C₄-alkyl, C₁-C₄-alkylsulphoximino-C₂-C₅-alkylcarbonyl, C₂-C₆-alkoxycarbonyl, C₂-C₆-alkylcarbonyl or C₃-C₆-trialkylsilyl,
R⁴ represents hydrogen, C₁-C₄-alkyl, C₁-C₂-haloalkyl, halogen, cyano or C₁-C₂-haloalkoxy, moreover, two adjacent radicals R⁴ represent -(CH₂)₄-, -(CH=CH-)₂-, -O(CH₂)₂O-, -O(CF₂)₂O-, -(CH=CH-CH=N)- or -(CH=CH-N=CH)-,
n represents 0 to 3,
R⁵ represents C₁-C₄-alkyl, C₃-C₆-cycloalkyl, C₁-C₄-haloalkyl, C₁-C₆-halocycloalkyl, C₂-C₆-alkenyl, C₂-C₄-haloalkenyl, C₂-C₄-alkynyl, C₂-C₄-haloalkynyl, C₁-C₄-alkoxy, C₁-C₄-haloalkoxy, fluorine, chlorine, bromine, iodine, cyano, nitro or C₃-C₆-trialkylsilyl,
R⁶ represents methyl or 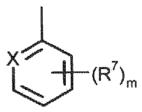
R⁷ represents independently at each occurrence hydrogen, halogen or C₁-C₄-haloalkyl,
m represents 1 or 2,
X represents N, CH, CF, CCl or CBr,
A represents CH₂, CH(CH₃), C(CH₃)₂ or CH₂CH₂,
A further represents -CH(CN)-,
Q represents an optionally mono- or polysubstituted 5- or 6-membered aromatic heterocyclic ring of series Q-36 to Q-40 or an aromatic 9-membered fused heterobicyclic ring system Q-54 to Q-56, the substituents being selectable independently of one another from C₁-C₃-alkyl, C₁-C₃-haloalkyl, C₁-C₂-alkoxy, halogen, cyano, hydroxyl, nitro or C₁-C₂-haloalkoxy,
Q further represents an optionally mono- or polysubstituted 5- or 6-membered aromatic heterocyclic ring of series Q-36 to Q-40 and Q-58 to Q-59, an aromatic 9-membered fused heterobicyclic ring system Q-54 to Q-56 and also represents a 5-membered heterocyclic ring Q-60 to Q-61, the substituents being selectable independently of one another from C₁-C₃-alkyl, C₁-C₃-haloalkyl, C₁-C₂-alkoxy, halogen, cyano, hydroxyl, nitro or C₁-C₂-haloalkoxy,
or the substituents being selectable independently of one another from phenyl or a 5- or 6-membered heteroaromatic ring, it being possible for phenyl or the ring to be unsubstituted or substituted one or more times by identical or different C₁-C₆-alkyl, C₂-C₆-alkenyl, C₂-C₆-alkynyl, C₃-C₆-cycloalkyl, C₁-C₆-haloalkyl, C₂-C₆-haloalkenyl, C₂-C₆-haloalkynyl, C₃-C₆-halocycloalkyl, halogen, CN, NO₂, C₁-C₄-alkoxy, C₁-C₄-haloalkoxy substituents, 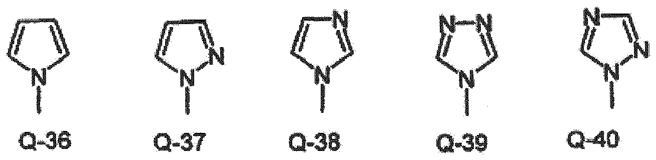 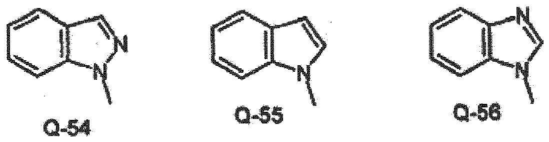 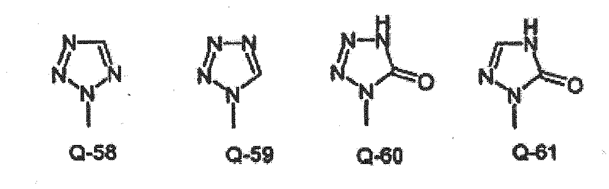 and also their N-oxides and salts.

2. Compounds of the formula (I) according to Claim 1, in which
A represents CH₂, CH(CH₃), C(CH₃) ₂ or CH₂CH₂,
R³ represents hydrogen or represents C₁-C₆-alkyl, C₁-C₆-alkoxy, each of which is optionally substituted one or more times by identical or different substituents selectable independently of one another from halogen, cyano, nitro, hydroxyl, C₁-C₆-alkyl, C₃-C₆-cycloalkyl, C₁-C₄-alkoxy, C₁-C₄-haloalkoxy, C₁-C₄-alkylthio, C₁-C₄-alkylsulphinyl, C₁-C₄-alkylsulphonyl, C₁-C₄-alkylsulphimino, C₁-C₄-alkylsulphimino-C₁-C₄-alkyl, C₁-C₄-alkylsulphimino-C₂-C₅-alkylcarbonyl, C₁-C₄-alkylsulphoximino, C₁-C₄-alkylsulphoximino-C₁-C₄-alkyl, C₁-C₄-alkylsulphoximino-C₂-C₅-alkylcarbonyl, C₂-C₆-alkoxycarbonyl, C₂-C₆-alkylcarbonyl or C₃-C₆-trialkylsilyl,
Q represents an optionally mono- or polysubstituted 5- or 6-membered aromatic heterocyclic ring of series Q-36 to Q-40 or an aromatic 9-membered fused heterobicyclic ring system Q-54 to Q-56, the substituents being selectable independently of one another from C₁-C₃-alkyl, C₁-C₃-haloalkyl, C₁-C₂-alkoxy, halogen, cyano, hydroxyl, nitro or C₁-C₂-haloalkoxy.

3. Compounds of the formula (I-1) according to Claim 1 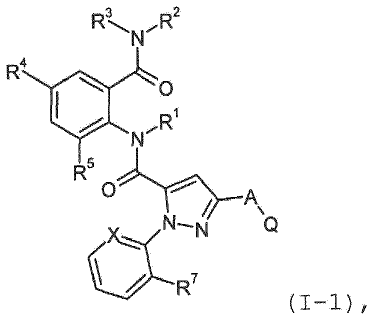 in which
R¹ represents hydrogen,
R² represents hydrogen,
R³ represents C₁-C₄-alkyl (methyl, ethyl, n-propyl, isopropyl, n-butyl, isobutyl, sec-butyl or tert-butyl) or cyano-C₁-C₃-alkyl (cyanomethyl, 1-cyanoethyl, 2-cyanoethyl, 1-cyano-n-propyl, 2-cyano-n-propyl, 3-cyano-n-propyl, 1-cyanoisopropyl, 2-cyano-isopropyl),
R⁴ represents hydrogen, methyl, trifluoromethyl, cyano, fluorine, chlorine, bromine, iodine or trifluoromethoxy,
R⁵ represents methyl, fluorine, chlorine, bromine or iodine,
R⁷ represents fluorine, chlorine or bromine,
X represents N, CCl or CH,
A represents CH₂ or CH(CH₃),
Q represents an optionally mono- or polysubstituted aromatic heterocyclic ring of series Q-37, Q-38, Q-39, Q-40, Q-58 and Q-59, and also represents a 5-membered heterocyclic ring Q-60, the substituents being selectable independently of one another from C₁-C₃-alkyl, C₁-C₃-haloalkyl, C₁-C₂-alkoxy, halogen, cyano, hydroxyl, nitro or C₁-C₂-haloalkoxy,
or the substituents being selectable independently of one another from phenyl or a 5- or 6-membered heteroaromatic ring, it being possible for phenyl or the ring to be unsubstituted or substituted one or more times by identical or different C₁-C₆-alkyl, C₂-C₆-alkenyl, C₂-C₆-alkynyl, C₃-C₆-cycloalkyl, C₁-C₆-haloalkyl, C₂-C₆-haloalkenyl, C₂-C₆-haloalkynyl, C₃-C₆-halocycloalkyl, halogen, CN, NO₂, C₁-C₄-alkoxy or C₁-C₄-haloalkoxy substituents,
and also their N oxides and salts.

4. Process for preparing compounds of the general formula (I) according to Claim 1, **characterized in that**
(A) anilines of the formula (II) 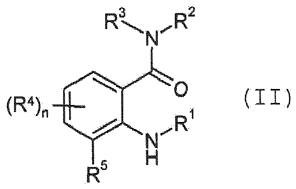 in which R¹, R², R³, R⁴, R⁵ and n have the definitions stated above
are reacted with carbonyl chlorides of the formula (III) 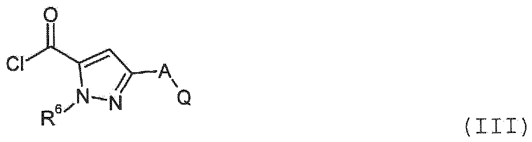 in which R⁶, A and Q have the definitions stated above, in the presence of an acid-binding agent,
(B) anilines of the formula (II) 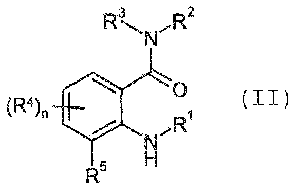 in which R¹, R², R³, R⁴, R⁵ and n have the definitions stated above
are reacted with a carboxylic acid of the formula (IV) 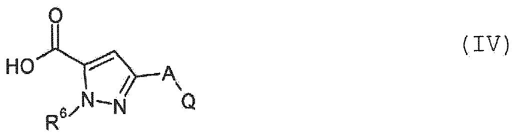 in which R⁶, A and Q have the definitions stated above
in the presence of a condensing agent, or
(C) for the synthesis of compounds of the formula (I) in which R¹ represents hydrogen, benzoxazinones of the formula (V) 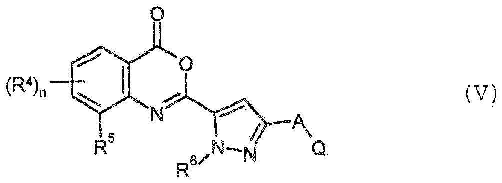 in which R⁴, R⁵, R⁶, A, Q and n have the definitions stated above
are reacted with an amine of the formula (XV) 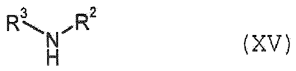 in which R² and R³ have the definitions stated above,
in the presence of a diluent.

5. Use of compounds of the formula (I) or (I-1) according to Claims 1 to 3 to control animal pests with the exception of the therapeutic treatment of the animal or human body.

6. Method of controlling animal pests, **characterized in that** compounds of the formula (I) or (I-1) according to Claims 1 to 3 are caused to act on animal pests and/or phytopathogenic fungi and/or their habitat and/or seed with the exception of the therapeutic treatment of the animal or human body.

7. Process for producing agrochemical compositions, **characterized in that** compounds of the formula (I) or (I-1) according to Claims 1 to 3 are mixed with extenders and/or surface-active substances.

8. Use of compounds of the formula (I) or (1-1) according to Claims 1 to 3 to treat seed.

9. Use of compounds of the formula (I) or (I-1) according to Claims 1 to 3 to treat transgenic plants.

10. Use of compounds of the formula (I) or (I-1) according to Claims 1 to 3 to treat seed of transgenic plants.

11. Seed coated with a compound of the formula (I) or (1-1) according to Claims 1 to 3.

## Revendications

1. Composés de formule (I) 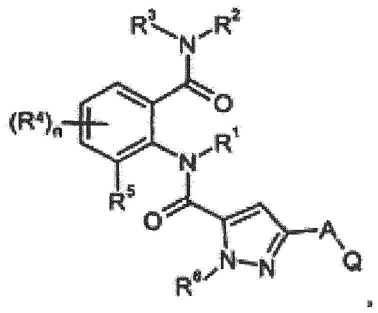 dans laquelle
R¹ représente un atome d'hydrogène, le groupe méthyle, cyclopropyle, cyanométhyle, méthoxy-méthyle, méthylthiométhyle, méthylsulfinylméthyle ou méthylsulfonylméthyle,
R² représente un atome d'hydrogène ou le groupe méthyle,
R³ représente un atome d'hydrogène ou un groupe alkyle en C₁-C₆, alcoxy en C₁-C₆, chacun portant éventuellement un ou plusieurs substituants, identiques ou différents, les substituants étant choisis indépendamment les uns des autres parmi halogéno, cyano, nitro, hydroxy, alkyle en C₁-C₆, cycloalkyle en C₃-C₆, alcoxy en C₁-C₄, halogénoalcoxy(C₁-C₄), alkyl(C₁-C₄)thio, alkyl(C₁-C₄)sulfinyle, alkyl(C₁-C₄)sulfonyle, alkyl(C₁-C₄) sulfimino, alkyl (C₁-C₄) sulfimino-alkyle (C₁-C₄), alkyl (C₁-C₄) sulfimino-alkyl (C₂-C₅)-carbonyle, alkyl(C₁-C₄)sulfoximino, alkyl(C₁-C₄)-sulfoximino-alkyle (C₁-C₄), alkyl (C₁-C₄) sulfoximino-alkyl (C₂-C₅) carbonyle, alcoxy (C₂-C₆) carbonyle, alkyl(C₂-C₆)carbonyle ou trialkyl(C₃-C₆)silyle,
R³ en outre représente un groupe cycloalkyle en C₃-C₆ portant éventuellement un ou plusieurs substituants, identiques ou différents, les substituants étant choisis indépendamment les uns des autres parmi halogéno, cyano, nitro, hydroxy, alkyle en C₁-C₆, cycloalkyle en C₃-C₆, alcoxy en C₁-C₄, halogénoalcoxy(C₁-C₄), alkyl (C₁-C₄) thio, alkyl (C₁-C₄) sulfinyle, alkyl (C₁-C₄) sulfonyle, alkyl (C₁-C₄) sulfimino, alkyl (C₁-C₄) sulfimino-alkyle(C₁-C₄), alkyl (C₁-C₄) sulfimino-alkyl (C₂-C₅)-carbonyle, alkyl (C₁-C₄) sulfoximino, alkyl (C₁-C₄)-sulfoximino-alkyle (C₁-C₄), alkyl (C₁-C₄) sulfoximino-alkyl (C₂-C₅) carbonyle, alcoxy (C₂-C₆) carbonyle, alkyl(C₂-C₆)carbonyle ou trialkyl (C₃-C₆) silyle,
R⁴ représente un atome d'hydrogène, un groupe alkyle en C₁-C₄, halogénoalkyle(C₁-C₂₎, un atome d'halogène, un groupe cyano ou halogénoalcoxy (C₁-C₂),
deux radicaux R⁴ adjacents représentent en outre - (CH₂)₄-, - (CH=CH-)₂-, -O(CH₂)₂O-, -O(CF2)₂O-, -(CH=CH-CH=N)- ou - (CH=CH-N=CH) -
n représente 0 à 3,
R⁵ représente un groupe alkyle en C₁-C₄, cycloalkyle en C₃-C₆, halogénoalkyle(C₁-C₄), halogéno-cycloalkyle(C₁-C₆), alcényle en C₂-C₆, halogénoalcényle(C₂-C₄), alcynyle en C₂-C₄, halogénoalcynyle(C₂-C₄), alcoxy en C₁-C₄, halogénoalcoxy(C₁-C₄), un atome de fluor, de chlore, de brome, d'iode, un groupe cyano, nitro ou trialkyl(C₃-C₆)silyle,
R⁶ représente le groupe méthyle ou 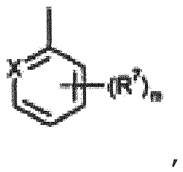
R⁷ représente chaque fois indépendamment un atome d'hydrogène ou d'halogène ou un groupe halogénoalkyle (C₁-C₄),
m représente 1 ou 2,
X représente N, CH, CF, CCl, ou CBr,
A représente CH₂, CH(CH₃), C(CH₃)₂ ou CH₂CH₂,
A représente en outre -CH(CN)-,
Q représente un cycle aromatique hétérocyclique à 5 ou 6 chaînons, de la série Q-36 à Q-40, éventuellement une ou plusieurs fois substitué, ou un système cyclique hétérobicyclique condensé aromatique à 9 chaînons Q-54 à Q-56, les substituants pouvant être choisis indépendamment les uns des autres parmi alkyle en C₁-C₃, halogénoalkyle(C₁-C3₎, alcoxy en C₁-C₂, halogéno, cyano, hydroxy, nitro ou halogénoalcoxy(C₁-C₂),
Q en outre représente un cycle aromatique hétérocyclique à 5 ou 6 chaînons, de la série Q-36 à Q-40 et Q-58 à Q-59, éventuellement une ou plusieurs fois substitué, un système cyclique hétérobicyclique aromatique condensé à 9 chaînons Q-54 à Q-56 ou représente un cycle hétérocyclique à 5 chaînons Q-60 à Q-61, les substituants pouvant être choisis indépendamment les uns des autres parmi alkyle en C₁-C₃, halogénoalkyle(C₁-C₃), alcoxy en C₁-C₂, halogéno, cyano, hydroxy, nitro ou halogénoalcoxy(C₁-C₂),
ou les substituants pouvant être choisis indépendamment les uns des autres parmi phényle ou un cycle hétéroaromatique à 5 ou 6 chaînons, le groupe phényle ou le cycle pouvant éventuellement porter un ou plusieurs substituants, identiques ou différents, alkyle en C₁-C₆, alcényle en C₂-C₆, alcynyle en C₂-C₆, cycloalkyle en C₃-C₆, halogénoalkyle(C₁-C₆), halogénoalcényle(C₂-C₆), halogénoalcynyle(C₂-C₆), halogéno-cycloalkyle(C₃-C₆), halogéno, CN, NO₂, alcoxy en C₁-C₄, halogénoalcoxy(C₁-C₄), 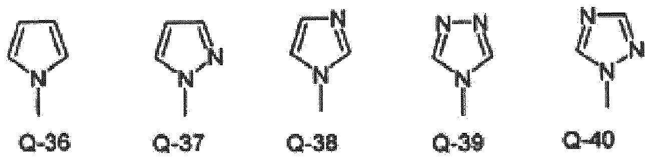 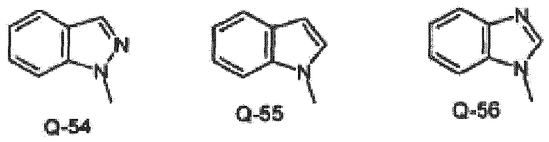 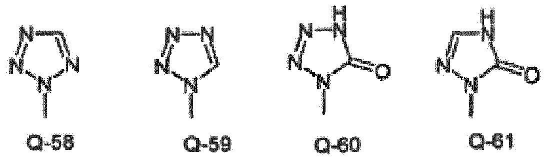
ainsi que leurs N-oxydes et sels.

2. Composés de formule (I) selon la revendication 1, dans lesquels
A représente CH₂, CH(CH₃), C (CH₃) ₂ ou CH₂CH₂,
R³ représente un atome d'hydrogène ou un groupe alkyle en C₁-C₆, alcoxy en C₁-C₆, chacun portant éventuellement un ou plusieurs substituants, identiques ou différents, les substituants étant choisis indépendamment les uns des autres parmi halogéno, cyano, nitro, hydroxy, alkyle en C₁-C₆, cycloalkyle en C₃-C₆, alcoxy en C₁-C₄, halogénoalcoxy(C₁-C₄), alkyl(C₁-C₄)thio, alkyl (C₁-C₄) sulfinyle, alkyl(C₁-C₄)sulfonyle, alkyl (C₁-C₄) sulfimino, alkyl (C₁-C₄) sulfimino-alkyle(C₁-C₄), alkyl(C₁-C₄) sulfimino-alkyl (C₂-C₅)-carbonyle, alkyl (C₁-C₄) sulfoximino, alkyl (C₁-C₄)-sulfoximino-alkyle (C₁-C₄) , alkyl (C₁-C₄) sulfoximino-alkyl (C₂-C₅) carbonyle, alcoxy (C₂-C₆) carbonyle, alkyl (C₂-C₆) carbonyle ou trialkyl (C₃-C₆) silyle,
Q représente un cycle aromatique hétérocyclique à 5 ou 6 chaînons, de la série Q-36 à Q-40, éventuellement une ou plusieurs fois substitué, ou un système cyclique hétérobicyclique condensé aromatique à 9 chaînons Q-54 à Q-56, les substituants pouvant être choisis indépendamment les uns des autres parmi alkyle en C₁-C₃, halogénoalkyle(C₁-C₃), alcoxy en C₁-C₂, halogéno, cyano, hydroxy, nitro ou halogénoalcoxy(C₁-C₂).

3. Composés de formule (I-1) selon la revendication 1 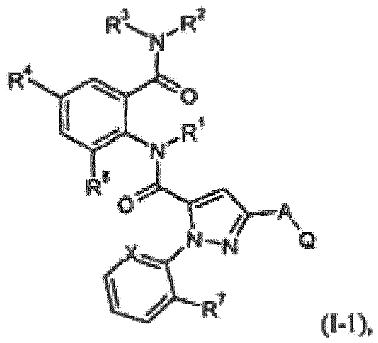 dans laquelle
R¹ représente un atome d'hydrogène,
R² représente un atome d'hydrogène,
R³ représente un groupe alkyle en C₁-C₄ (méthyle, éthyle, n-propyle, isopropyle, n-butyle, isobutyle, sec-butyle ou tert-butyle) ou cyano-alkyle(C₁-C₃) (cyanométhyle, 1-cyanoéthyle, 2-cyanoéthyle, 1-cyano-n-propyle, 2-cyano-n-propyle, 3-cyano-n-propyle, 1-cyano-isopropyle, 2-cyano-isopropyle),
R⁴ représente un atome d'hydrogène, le groupe méthyle, trifluorométhyle, cyano, un atome de fluor, de chlore, de brome, d'iode ou le groupe trifluorométhoxy,
R⁵ représente le groupe méthyle, un atome de fluor, de chlore, de brome ou d'iode,
R⁷ représente un atome de fluor, chlore ou brome,
X représente N, CCl ou CH,
A représente CH₂ ou CH(CH₃),
Q représente un cycle aromatique hétérocyclique de la série Q-37, Q-38, Q-39, Q-40, Q-58 et Q-59 éventuellement une ou plusieurs fois substitué, ainsi qu'un cycle hétérocyclique à 5 chaînons Q-60, les substituants pouvant être choisis indépendamment les uns des autres parmi alkyle en C₁-C₃, halogénoalkyle(C₁-C₃), alcoxy en C₁-C₂, halogéno, cyano, hydroxy, nitro ou halogénoalcoxy(C₁-C₂),
ou les substituants pouvant être choisis indépendamment les uns des autres parmi phényle ou un cycle hétéroaromatique à 5 ou 6 chaînons, le groupe phényle ou le cycle pouvant éventuellement porter un ou plusieurs substituants, identiques ou différents, alkyle en C₁-C₆, alcényle en C₂-C₆, alcynyle en C₂-C₆, cycloalkyle en C₃-C₆, halogénoalkyle(C₁-C₆), halogênoalcényle(C₂-C₆), halogénoalcynyle(C₂-C₆), halogéno-cycloalkyle(C₃-C₆), halogéno, CN, NO₂, alcoxy en C₁-C₄, halogénoalcoxy(C₁-C₄),
ainsi que leurs N-oxydes et sels.

4. Procédé pour la préparation des composés de formule générale (I) selon la revendication 1, **caractérisé en ce qu'**on fait réagir
(A) des anilines de formule (II) 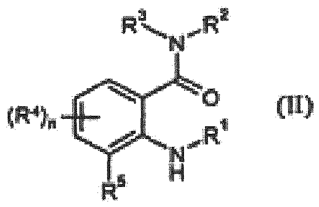 dans laquelle R¹, R², R³, R⁴, R⁵ et n ont les significations indiquées plus haut,
avec des chlorures d'acyle de formule (III) 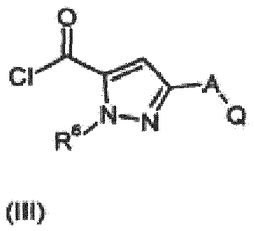 dans laquelle R⁶, A et Q ont les significations indiquées plus haut, en présence d'un capteur d'acide,
(B) des anilines de formule (II) 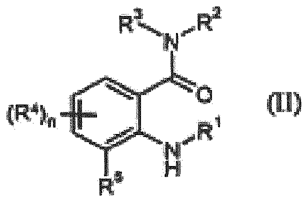 dans laquelle R¹, R², R³, R⁴, R⁵ et n ont les significations indiquées plus haut,
avec un acide carboxylique de formule (IV) 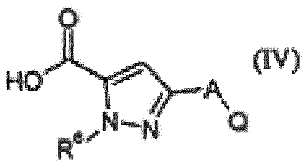 dans laquelle R⁶, A et Q ont les significations indiquées plus haut,
en présence d'un agent de condensation ou
(C) pour la synthèse des composés de formule (I) dans lesquels R¹ représente un atome d'hydrogène, on fait réagir des benzoxazinones de formule (V) 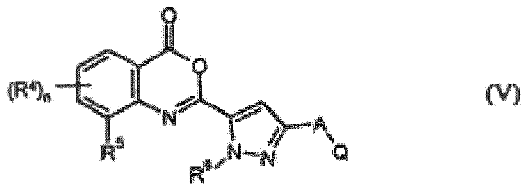 dans laquelle R⁴, R⁵, R⁶, A, Q et n ont les significations indiquées plus haut,
avec une amine de formule (XV) 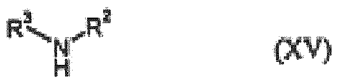 dans laquelle R² et R³ ont les significations indiquées plus haut,
en présence d'un diluant.

5. Utilisation des composés de formule (I) ou (I-1) selon les revendications 1 à 3, pour la lutte contre des ravageurs animaux, à l'exclusion du traitement thérapeutique du corps humain ou animal.

6. Procédé pour la lutte contre des ravageurs animaux, **caractérisé en ce qu'**on fait agir des composés de formule (I) ou (1-1) selon les revendications 1 à 3 sur des ravageurs animaux et/ou des champignons phytopathogènes et/ou leur habitat et/ou des semences, à l'exclusion du traitement thérapeutique du corps humain ou animal.

7. Procédé pour la fabrication de compositions agrochimiques, **caractérisé en ce qu'**on mélange des composés de formule (I) ou (1-1) selon les revendications 1 à 3 avec des excipients et/ou des substances tensioactives.

8. Utilisation des composés de formule (I) ou (I-1) selon les revendications 1 à 3 pour le traitement de semences.

9. Utilisation des composés de formule (I) ou (1-1) selon les revendications 1 à 3 pour le traitement de plantes transgéniques.

10. Utilisation des composés de formule (I) ou (I-1) selon les revendications 1 à 3 pour le traitement de semences de plantes transgéniques.

11. Semence, qui est enrobée avec un composé de formule (I) ou (1-1) selon les revendications 1 à 3.
